# EUROPEAN PATENT APPLICATION

(11) **EP 4 317 144 A1**
(43) Date of publication of application: **07.02.2024**
(21) Application number: 22774267.3
(22) Date of filing: 23.03.2022
(51) Int. Cl.: C07D 401/14, C07D 403/14, C07D 417/14, C07D 413/14, C07D 241/20, C07D 221/20, C07D 487/04, C07D 495/04, C07D 498/04, C07D 471/10, A61K 31/497, A61P 35/00, A61P 35/02

(54) **HETEROCYCLE SUBSTITUTED KETONE DERIVATIVE, AND COMPOSITION AND MEDICINAL USE THEREOF**

(30) Priority: 23.03.2021 CN 202110306983; 02.11.2021 CN 202111287417
(71) Applicant: Shanghai Haiyan Pharmaceutical Technology Co., Ltd., Shanghai 201203 (CN); Yangtze River Pharmaceutical Group Co., Ltd., Taizhou, Jiangsu 225321 (CN)
(72) Inventor: ZHU, Weixing, Shanghai 201203 (CN); LIU, Qiang, Shanghai 201203 (CN); HAO, Xuhui, Shanghai 201203 (CN); CHEN, Yonggang, Shanghai 201203 (CN); LI, Shaobo, Shanghai 201203 (CN); HE, Shanghua, Shanghai 201203 (CN); HUANG, Xiaotian, Shanghai 201203 (CN); ZHAO, Zhiming, Shanghai 201203 (CN)
(74) Representative: Plate, Jürgen
(86) International application number: PCT/CN2022/082485
(87) International publication number: WO 2022/199611

(57) **Abstract**

Provided is a heterocycle substituted ketone derivative, the structure of which is as shown in formula (I). Further provided are a pharmaceutically acceptable salt of the derivative, a stereoisomer thereof, a pharmaceutical composition and the medicinal use thereof.

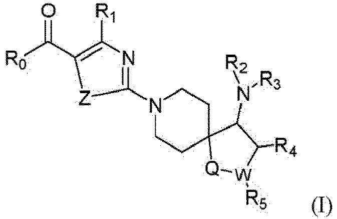

## Description

### TECHNICAL FIELD

The present invention relates to the field of pharmaceutical technology, in particular to a heterocycle-substituted ketone derivative, a pharmaceutically acceptable salt thereof, a stereoisomer thereof, a pharmaceutical composition thereof, and pharmaceutical use thereof.

### BACKGROUND

Protein tyrosine phosphatase (PTP) catalyzes the dephosphorylation of phosphotyrosine and is a key control element in signaling in mammals. Deviations in biological function of PTP may cause disorder in body regulation, leading to a variety of diseases such as cancer, diabetes and autoimmune diseases. Currently, Src homology-2 domain-containing protein tyrosine phosphatase (SHP2) is the only confirmed proto-oncoprotein in the PTP family. Src homology-2 phosphatase (SHP2) that contributes to a variety of cellular functions including proliferation, differentiation, cell cycle maintenance and migration is a non-receptor protein tyrosine phosphatase encoded by PTPN11 gene. SHP2 is involved in signaling through Ras-mitogen-activated protein kinases (JAK-STAT or phosphatidylinositol 3-kinase-AKT pathway).

SHP2 is considered as an ideal target for cancer intervention due to 3 aspects as follows. Firstly, SHP2 is a common node of multiple signaling pathways for activating RAS. Activation of RAS is very important for both growth and survival of cancer cells, and almost all protein tyrosine kinases (PTKs) initiate the RAS signaling pathway by activating SHP2. Therefore, a suitable SHP2 inhibitor that can "catch all" different PTK mutations has a potential to be a broad-spectrum anticancer drug. Secondly, due to the overlap of PTK and SHP2 signaling pathways, SHP2 inhibitor can be used in combination with a kinase inhibitor for dual inhibition of the interconnected signaling pathways. Such combination therapy is more effective than monotherapy, is less prone to drug resistance and can reverse the acquired resistance to PTK inhibitors. Thirdly, SHP2 is also involved in signaling pathway of programmed cell death checkpoint PD-1/PD-L1 and is a direct downstream complex component of PD-1/PD-L1. Inhibiting the binding of SHP2 to PD-1 may restore T cell activation. Therefore, these reasons open up a new route for this target in immunotherapy. Mutations in the PTPN11 gene and subsequently in SHP2 are identified in a variety of human diseases including, for example, Noonan syndrome, Leopard syndrome, juvenile myelomonocytic leukemia, neuroblastoma, melanoma, acute myeloid leukemia, as well as breast cancer, lung cancer and colon cancer. Therefore, SHP2 represents a highly attractive target for developing new therapies for treatment of various diseases. The compound of the present invention meets the need for a small molecule that inhibit SHP2 activity.

### SUMMARY

The object of the present invention is to provide a heterocycle-substituted ketone derivative with high activity and high selectivity. A first aspect of the present invention provides a compound represented by formula (I), or a pharmaceutically acceptable salt thereof, or a stereoisomer thereof: 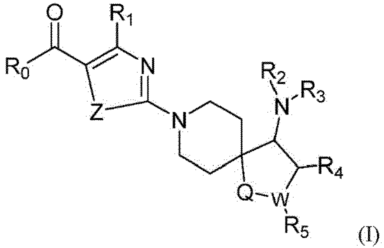 wherein in formula (I),
R₀ is of the structure represented by formula (a): 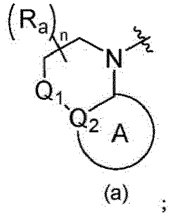
wherein Q₁ is a bond or is CR_{q1}R_{q2}, O or NR_{q3}; Q₂ represents a ring atom that is C or N; and when Q₁ contains N, Q₂ is not N;
R_{q1} and R_{q2} are each independently hydrogen, C₁₋₈ alkyl (preferably C₁₋₆ alkyl, more preferably C₁₋₃ alkyl), C₃₋₈ cycloalkyl (preferably C₃₋₆ cycloalkyl), C₁₋₈ alkoxy (preferably C₁₋₆ alkoxy, more preferably C₁₋₃ alkoxy), cyano, hydroxyl, carboxyl, halogen (preferably fluorine or chlorine), -C(O)NRₐ₀R_{b0}, -C(O)C₁₋₈ alkyl (preferably -C(O)C₁₋₆ alkyl, more preferably -C(O)C₁₋₃ alkyl) or -C(O)OC₁₋₈ alkyl (preferably -C(O)OC₁₋₆ alkyl, more preferably -C(O)OC₁₋₃ alkyl); or R_{q1}, R_{q2} and the linked carbon atom together form a 3- to 7-membered saturated or partially unsaturated heteromonocycle or a 3- to 7-membered saturated or partially unsaturated monocycle; wherein C₁₋₈ alkyl, C₁₋₈ alkoxy, C₃₋₈ cycloalkyl, 3- to 7-membered saturated or partially unsaturated heteromonocycle and 3- to 7-membered saturated or partially unsaturated monocycle are unsubstituted or substituted by 1, 2 or 3 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5-to 6-membered heteroaryl among the substituents are each optionally substituted by 1, 2 or 3 substituents each independently selected from a substituent group S;
R_{q3} is hydrogen, C₁₋₈ alkyl (preferably C₁₋₆ alkyl, more preferably C₁₋₃ alkyl), C₃₋₈ cycloalkyl (preferably C₃₋₆ cycloalkyl), -C(O)NRₐ₀R_{b0}, -C(O)C₁₋₈ alkyl (preferably -C(O)C₁₋₆ alkyl, more preferably -C(O)C₁₋₃ alkyl) or -SO₂C₁₋₈ alkyl (preferably -SO₂C₁₋₆ alkyl, more preferably -SO₂C₁₋₃ alkyl); wherein C₁₋₈ alkyl and C₃₋₈ cycloalkyl are unsubstituted or substituted by 1, 2 or 3 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from the substituent group S;
(Rₐ)ₙ represents n Rₐ that substitute hydrogens on ring atoms of nitrogen-containing 6-membered heterocycle, and n is 0, 1 or 2; each Rₐ is identical or different, and each Rₐ is independently deuterium, cyano, hydroxyl, carboxyl, halogen (preferably fluorine or chlorine), C₁₋₈ alkyl (preferably C₁₋₆ alkyl, more preferably C₁₋₃ alkyl), C₁₋₈ alkoxy (preferably C₁₋₆ alkoxy, more preferably C₁₋₃ alkoxy), -C(O)C₁₋₈ alkyl (preferably -C(O)C₁₋₆ alkyl, more preferably -C(O)C₁₋₃ alkyl), -C(O)OC₁₋₈ alkyl (preferably -C(O)OC₁₋₆ alkyl, more preferably -C(O)OC₁₋₃ alkyl), -OC(O)C₁₋₈ alkyl (preferably -OC(O)C₁₋₆ alkyl, more preferably -OC(O)C₁₋₃ alkyl) or -C(O)NRₐ₀R_{b0}; or any two Rₐ linked to the same ring atom or to adjacent ring atoms connect and form a 3- to 7-membered saturated or partially unsaturated heteromonocycle or a 3- to 7-membered saturated or partially unsaturated monocycle; wherein C₁₋₈ alkyl, C₁₋₈ alkoxy, 3- to 7-membered saturated or partially unsaturated heteromonocycle and 3- to 7-membered saturated or partially unsaturated monocycle are unsubstituted or substituted by 1, 2 or 3 substituents each independently selected from the group consisting of: deuterium, halogen, cyano, hydroxy, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from the substituent group S;
ring A is benzene ring or 5- to 6-membered heteroaryl ring; benzene ring and 5- to 6-membered heteroaryl ring are unsubstituted or substituted by 1, 2, 3 or 4 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)C₁₋₃ alkyl, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from the substituent group S;
or R₀ is of the structure represented by formula (b): 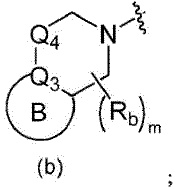
wherein Q₃ represents a ring atom that is C or N; Q₄ is a bond or is CR_{q4}R_{q5};
R_{q4} and R_{q5} are each independently hydrogen, deuterium, C₁₋₈ alkyl (preferably C₁₋₆ alkyl, more preferably C₁₋₃ alkyl), C₃₋₈ cycloalkyl (preferably C₃₋₆ cycloalkyl), C₁₋₈ alkoxy (preferably C₁₋₆ alkoxy, more preferably C₁₋₃ alkoxy), cyano, hydroxyl, carboxyl, halogen (preferably fluorine or chlorine), -C(O)NRₐ₀R_{b0}, -C(O)C₁₋₈ alkyl (preferably -C(O)C₁₋₆ alkyl, more preferably -C(O)C₁₋₃ alkyl) or -C(O)OC₁₋₈ alkyl (preferably -C(O)OC₁₋₆ alkyl, more preferably -C(O)OC₁₋₃ alkyl); or R_{q4}, R_{q5} and the linked carbon atom together form a 3- to 7-membered saturated or partially unsaturated heteromonocycle or a 3- to 7-membered saturated or partially unsaturated monocycle; wherein C₁₋₈ alkyl, C₁₋₈ alkoxy, C₃₋₈ cycloalkyl, 3- to 7-membered saturated or partially unsaturated heteromonocycle and 3- to 7-membered saturated or partially unsaturated monocycle are unsubstituted or substituted by 1, 2 or 3 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5-to 6-membered heteroaryl among the substituents are each optionally substituted by 1, 2 or 3 substituents each independently selected from the substituent group S;
(R_{b})ₘ represents m R_{b} that substitute hydrogens on ring atoms of nitrogen-containing heterocycle, and m is 0, 1 or 2; each R_{b} is identical or different, and each R_{b} is independently deuterium, cyano, hydroxyl, carboxyl, halogen (preferably fluorine or chlorine), C₁₋₈ alkyl (preferably C₁₋₆ alkyl, more preferably C₁₋₃ alkyl), C₁₋₈ alkoxy (preferably C₁₋₆ alkoxy, more preferably C₁₋₃ alkoxy ), -C(O)C₁₋₈ alkyl (preferably -C(O)C₁₋₆ alkyl, more preferably -C(O)C₁₋₃ alkyl), -C(O)OC₁₋₈ alkyl (preferably -C(O)OC₁₋₆ alkyl, more preferably -C(O)OC₁₋₃ alkyl), -OC(O)C₁₋₈ alkyl (preferably -OC(O)C₁₋₆ alkyl, more preferably -OC(O)C₁₋₃ alkyl) or -C(O)NRₐ₀R_{b0};
or any two R_{b} linked to the same ring atom or to different ring atoms connect and form a 3- to 7-membered saturated or partially unsaturated heteromonocycle;
or one of R_{q4} and R_{q5} together with R_{b} on the adjacent carbon atom connect and form a 3- to 7-membered saturated or partially unsaturated heteromonocycle or a 3- to 7-membered saturated or partially unsaturated monocycle;
wherein C₁₋₈ alkyl, C₁₋₈ alkoxy, 3- to 7-membered saturated or partially unsaturated heteromonocycle and 3- to 7-membered saturated or partially unsaturated monocycle are unsubstituted or substituted by 1, 2 or 3 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5-to 6-membered heteroaryl among the substituents are each optionally substituted by 1, 2 or 3 substituents each independently selected from the substituent group S;
ring B is benzene ring or 5- to 6-membered heteroaryl ring; benzene ring and 5- to 6-membered heteroaryl ring are unsubstituted or substituted by 1, 2, 3 or 4 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)C₁₋₃ alkyl, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from the substituent group S;
R₁ is hydrogen, C₁₋₈ alkyl (preferably C₁₋₆ alkyl, more preferably C₁₋₃ alkyl), NRₐ₀R_{b0}, C₃₋₈ cycloalkyl (preferably C₃₋₆ cycloalkyl), C₁₋₈ alkoxy (preferably C₁₋₆ alkoxy, more preferably C₁₋₃ alkoxy), cyano, hydroxyl, carboxyl, halogen (preferably fluorine or chlorine), -C(O)NRₐ₀R_{b0}, -C(O)C₁₋₈ alkyl (preferably -C(O)C₁₋₆ alkyl, more preferably -C(O)C₁₋₃ alkyl) or -C(O)OC₁₋₈ alkyl (preferably -C(O)OC₁₋₆ alkyl, more preferably -C(O)OC₁₋₃ alkyl); wherein C₁₋₈ alkyl, C₁₋₈ alkoxy and C₃₋₈ cycloalkyl are unsubstituted or substituted by 1, 2 or 3 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from the substituent group S;
R₂ and R₃ are each independently hydrogen, C₁₋₈ alkyl (preferably C₁₋₆ alkyl, more preferably C₁₋₃ alkyl), C₃₋₈ cycloalkyl (preferably C₃₋₆ cycloalkyl), -C(O)NRₐ₀R_{b0} or -C(O)C₁₋₈ alkyl (preferably -C(O)C₁₋₆ alkyl, more preferably -C(O)C₁₋₃ alkyl); wherein C₁₋₈ alkyl and C₃₋₈ cycloalkyl are unsubstituted or substituted by 1, 2 or 3 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from the substituent group S;
Z is -N=CR_{Z1}-, -N=N-, -C(O)-NR_{Z2}- or -NR_{Z3}-CHR_{Z4}-; or Z is of the structure represented by formula (c): 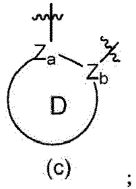
wherein Zₐ and Z_{b} represent ring atoms, and are each independently C or N;
ring D is 5- to 6-membered heteroaryl ring; 5- to 6-membered heteroaryl ring is unsubstituted or substituted by 1 2, or 3 substituents each independently selected from the group consisting of: deuterium, halogen, cyano, hydroxyl, carboxyl, C₁₋₃ alkyl, hydroxyl-substituted C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)C₁₋₃ alkyl, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from the substituent group S;
R_{Z1} is hydrogen, C₁₋₈ alkyl (preferably C₁₋₆ alkyl, more preferably C₁₋₃ alkyl), C₃₋₈ cycloalkyl (preferably C₃₋₆ cycloalkyl), C₁₋₈ alkoxy (preferably C₁₋₆ alkoxy, more preferably C₁₋₃ alkoxy), cyano, hydroxyl, carboxyl, halogen (preferably fluorine or chlorine), -C(O)NRₐ₀R_{b0}, -C(O)C₁₋₈ alkyl (preferably -C(O)C₁₋₆ alkyl, more preferably -C(O)C₁₋₃ alkyl) or -C(O)OC₁₋₈ alkyl (preferably -C(O)OC₁₋₆ alkyl, more preferably -C(O)OC₁₋₃ alkyl); wherein C₁₋₈ alkyl, C₁₋₈ alkoxy and C₃₋₈ cycloalkyl are unsubstituted or substituted by 1, 2 or 3 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from the substituent group S;
R_{Z2} is hydrogen, C₁₋₈ alkyl (preferably C₁₋₆ alkyl, more preferably C₁₋₃ alkyl), C₃₋₈ cycloalkyl (preferably C₃₋₆ cycloalkyl), -C(O)NRₐ₀R_{b0}, -C(O)C₁₋₈ alkyl (preferably -C(O)C₁₋₆ alkyl, more preferably -C(O)C₁₋₃ alkyl) or -SO₂C₁₋₈ alkyl (preferably -SO₂C₁₋₆ alkyl, more preferably -SO₂C₁₋₃ alkyl); wherein C₁₋₈ alkyl and C₃₋₈ cycloalkyl are unsubstituted or substituted by 1, 2 or 3 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from the substituent group S;
R_{Z3} and R_{Z4} connect and form a 3- to 7-membered saturated or partially unsaturated heteromonocycle; 3- to 7-membered saturated or partially unsaturated heteromonocycle is unsubstituted or substituted by 1, 2 or 3 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from the substituent group S;
Q, W, R₄ and R₅ are selected from the group consisting of:
   (i) Q is CR_{q6}R_{q7}, O or NR_{q8}; W is C or N; and Q and W are not heteroatoms at the same time; R₄, R₅ and the linked ring atoms together form benzene ring or 5- to 6-membered heteroaryl ring, and benzene ring or 5- to 6-membered heteroaryl ring is fused with a 5-membered ring; benzene ring and 5- to 6-membered heteroaryl ring are unsubstituted or substituted by 1, 2, 3 or 4 substituents each independently selected from the group consisting of: deuterium, halogen, cyano, hydroxyl, carboxyl, C₁₋₃ alkyl, hydroxyl-substituted C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)C₁₋₃ alkyl, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5-to 6-membered heteroaryl among the substituents are each optionally substituted by 1, 2 or 3 substituents each independently selected from the substituent group S;
   and (ii) Q is CR_{q6}R_{q7}; W is O; R₅ is absent; R₄ is hydrogen, C₁₋₈ alkyl (preferably C₁₋₆ alkyl, more preferably C₁₋₃ alkyl), C₃₋₈ cycloalkyl (preferably C₃₋₆ cycloalkyl), C₁₋₈ alkoxy (preferably C₁₋₆ alkoxy, more preferably C₁₋₃ alkoxy), cyano, hydroxy, carboxyl, or halogen (preferably fluorine or chlorine);
   wherein R_{q6} and R_{q7} are each independently hydrogen, C₁₋₈ alkyl (preferably C₁₋₆ alkyl, more preferably C₁₋₃ alkyl), C₃₋₈ cycloalkyl (preferably C₃₋₆ cycloalkyl), C₁₋₈ alkoxy (preferably C₁₋₆ alkoxy, more preferably C₁₋₃ alkoxy), cyano, hydroxyl, carboxyl, halogen (preferably fluorine or chlorine), -C(O)NRₐ₀R_{b0}, -C(O)C₁₋₈ alkyl (preferably -C(O)C₁₋₆ alkyl, more preferably -C(O)C₁₋₃ alkyl) or -C(O)OC₁₋₈ alkyl (preferably -C(O)OC₁₋₆ alkyl, more preferably -C(O)OC₁₋₃ alkyl); or R_{q6}, R_{q7} and the linked carbon atom together form a 3- to 7-membered saturated or partially unsaturated heteromonocycle or a 3- to 7-membered saturated or partially unsaturated monocycle; wherein C₁₋₈ alkyl, C₁₋₈ alkoxy, C₃₋₈ cycloalkyl, 3- to 7-membered saturated or partially unsaturated heteromonocycle and 3- to 7-membered saturated or partially unsaturated monocycle are unsubstituted or substituted by 1, 2 or 3 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5-to 6-membered heteroaryl among the substituents are each optionally substituted by 1, 2 or 3 substituents each independently selected from the substituent group S;
   R_{q8} is hydrogen, C₁₋₈ alkyl (preferably C₁₋₆ alkyl, more preferably C₁₋₃ alkyl), C₃₋₈ cycloalkyl (preferably C₃₋₆ cycloalkyl), -C(O)NRₐ₀R_{b0}, -C(O)C₁₋₈ alkyl (preferably -C(O)C₁₋₆ alkyl, more preferably -C(O)C₁₋₃ alkyl) or -SO₂C₁₋₈ alkyl (preferably -SO₂C₁₋₆ alkyl, more preferably -SO₂C₁₋₃ alkyl); wherein C₁₋₈ alkyl and C₃₋₈ cycloalkyl are unsubstituted or substituted by 1, 2 or 3 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from the substituent group S;
   the substituent group S is selected from the group consisting of: halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl;
   Rₐ₀ and R_{b0} are each independently hydrogen, C₁₋₃ alkyl or acetyl; or Rₐ₀, R_{b0} and the linked nitrogen atom together form a 4- to 6-membered saturated heteromonocycle; 4- to 6-membered saturated heteromonocycle is optionally substituted by 1, 2 or 3 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, -SO₂C₁₋₈ alkyl, -S(O)C₁₋₃ alkyl, -C(O)NH₂, -C(O)NH(C₁₋₃ alkyl), -C(O)N(C₁₋₃ alkyl)₂, -C(O)OC₁₋₃ alkyl, -OC(O)C₁₋₃ alkyl, C₃₋₆ cycloalkyl, C₃₋₆ cycloalkoxy and 3- to 6-membered heterocycloalkyl;
   Rₐ₁ and R_{b1} are each independently hydrogen, C₁₋₃ alkyl or acetyl; or Rₐ₁, R_{b1} and the linked nitrogen atom together form 4- to 6-membered saturated heteromonocycle; 4- to 6-membered saturated heteromonocycle is optionally substituted by 1, 2 or 3 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, -SO₂C₁₋₈ alkyl, -S(O)C₁₋₃ alkyl, -C(O)NH₂, -C(O)NH(C₁₋₃ alkyl), -C(O)N(C₁₋₃ alkyl)₂, -C(O)OC₁₋₃ alkyl, -OC(O)C₁₋₃ alkyl, C₃₋₆ cycloalkyl, C₃₋₆ cycloalkoxy and 3- to 6-membered heterocycloalkyl.

In some embodiments, R₁ is hydrogen or NH₂.

In some embodiments, R₁ is NH₂.

In some embodiments, R₂ and R₃ are each independently hydrogen.

In some embodiments, Q is CH₂; W is C; R₄, R₅ and the linked ring atoms together form benzene ring or 5- to 6-membered heteroaryl ring, and benzene ring and 5- to 6-membered heteroaryl ring are unsubstituted or substituted with 1, 2, 3 or 4 substituents each independently selected from the group consisting of: deuterium, halogen (preferably fluorine or chlorine), cyano, hydroxyl, carboxyl, C₁₋₃ alkyl (preferably methyl), hydroxy-substituted C₁₋₃ alkyl (preferably hydroxymethyl), C₁₋₃ alkoxy (preferably methoxy), C₂₋₄ alkenyl, C₂₋₄ alkynyl, halo-C₁₋₃ alkyl (preferably trifluoromethyl), halo-C₁₋₃ alkoxy, NRₐ₁R_{b1}, -SO₂C₁₋₃ alkyl, -S(O)C₁₋₃ alkyl, -C(O)NH₂, -C(O)C₁₋₃ alkyl, -C(O)OC₁₋₃ alkyl, -OC(O)C₁₋₃ alkyl, C₃₋₆ cycloalkyl (preferably cyclopropyl), C₃₋₆ cycloalkyloxy and 3- to 6-membered heterocycloalkyl.

In some embodiments, the compound represented by formula (I) is of the structure represented by formula (IA): 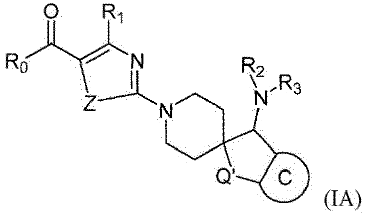
wherein in formula (IA), Q' is CR_{q6}R_{q7}, O or NR_{q8};
ring C is benzene ring or 5- to 6-membered heteroaryl ring; benzene ring and 5- to 6-membered heteroaryl ring are unsubstituted or substituted by 1, 2, 3 or 4 substituents each independently selected from the group consisting of: deuterium, halogen, cyano, hydroxyl, carboxyl, C₁₋₃ alkyl, hydroxyl-substituted C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1},-SO₂C₁₋₃ alkyl, -S(O)C₁₋₃ alkyl, -C(O)NRₐ₁R_{b1}, -C(O)C₁₋₃ alkyl, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from the substituent group S; and the rest groups are as defined above.

In some embodiments, Q' is CH₂.

In some embodiments, the compound represented by formula (IA) is of the structure represented by formula (IA-a) or formula (IA-b):

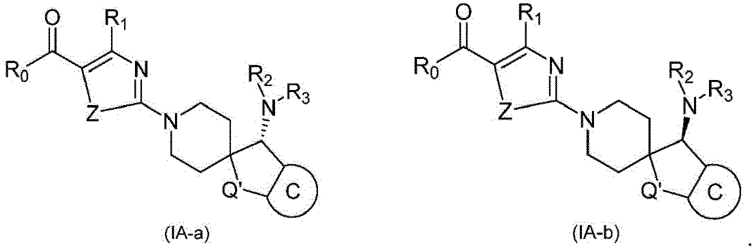

In some embodiments, the compound represented by formula (IA) is of the structure represented by formula (IA-a):

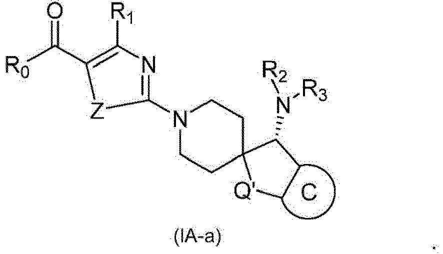

In some embodiments, the compound represented by formula (I) is of the structure represented by formula (IB): 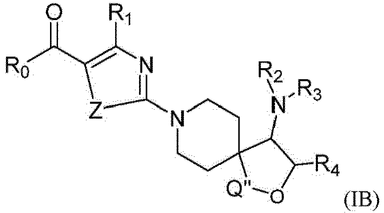 wherein in formula (IB), Q" is CR_{q6}R_{q7}; R₄ is hydrogen, C₁₋₈ alkyl (preferably C₁₋₆ alkyl, more preferably C₁₋₃ alkyl), C₃₋₈ cycloalkyl (preferably C₃₋₆ cycloalkyl), C₁₋₈ alkoxy (preferably C₁₋₆ alkoxy, more preferably C₁₋₃ alkoxy), cyano, hydroxy, carboxyl or halogen (preferably fluorine or chlorine); and the rest groups are as defined above.

In some embodiments, the compound represented by formula (IB) is of the structure represented by formula (IB-a) or formula (IB-b):

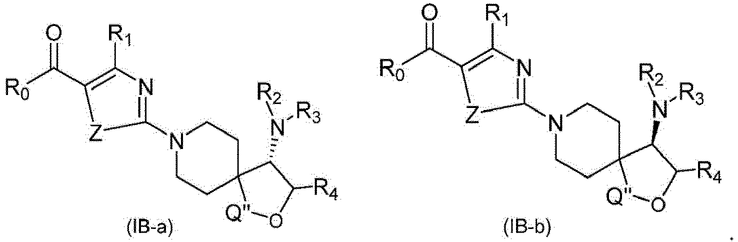

In some embodiments, Z is -N=CR_{Z1}-; R_{Z1} is hydrogen, C₁₋₃ alkyl (preferably methyl), C₃₋₆ cycloalkyl (preferably cyclopropyl), C₁₋₃ alkoxy (preferably methoxy), cyano, hydroxyl, carboxyl, halogen (preferably fluorine or chlorine), -C(O)NH₂, -C(O)C₁₋₃ alkyl (preferably -C(O)CH₃) or -C(O)OC₁₋₃ alkyl (preferably -C(O)OCH₃); wherein C₁₋₃ alkyl, C₁₋₃ alkoxy and C₃₋₆ cycloalkyl are unsubstituted or substituted with 1, 2 or 3 substituents each independently selected from the group consisting of: deuterium, halogen, cyano, hydroxyl, carboxyl, C₁₋₃ alkyl, C₁₋₃ alkoxy, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)OC₁₋₃ alkyl, -OC(O)C₁₋₃ alkyl, C₃₋₆ cycloalkyl, C₃₋₆ cycloalkyloxy and 3- to 6-membered heterocycloalkyl.

In some embodiments, Z is -N=CH-.

In some embodiments, the substituent group S is selected from the group consisting of: halogen (preferably fluorine or chlorine), cyano, hydroxyl, carboxyl, amino, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NH(C₁₋₃ alkyl), N(C₁₋₃ alkyl)₂, -SO₂C₁₋₃ alkyl, -C(O)NH₂, -C(O)NH₂, -C(O)NH(C₁₋₃ alkyl), -C(O)N(C₁₋₃ alkyl)₂, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl.

In some embodiments, the substituent group S is selected from the group consisting of: fluorine, chlorine, bromine, cyano, hydroxy, carboxyl, amino, methyl, ethyl, n-propyl, isopropyl, methoxy, ethoxy, n-propoxy, isopropxy, monochloromethyl, dichloromethyl, trichloromethyl, monochloroethyl, 1,2-dichloroethyl, trichloroethyl, monobromoethyl, monofluoromethyl, difluoromethyl, trifluoromethyl, monofluoroethyl, difluoroethyl, trifluoroethyl, trifluoromethoxy, trifluoroethoxy, monofluoromethoxy, monofluoroethoxy, difluoromethoxy, difluoroethoxy, NH(C₁₋₃ alkyl), N(C₁₋₃ alkyl)₂, -SO₂C₁₋₈ alkyl, -C(O)NH₂, -C(O)NH(C₁₋₃ alkyl), -C(O)N(C₁₋₃ alkyl)₂, cyclopropyl, cyclobutyl, cyclopentyl and cyclohexyl.

In some embodiments, 5- to 6-membered heteroaryl ring formed by R₄, R₅ and the linked ring atoms is selected from the group consisting of: thiophene ring, furan ring, thiazole ring, isothiazole ring, imidazole ring, oxazole ring, pyrrole ring, pyrazole ring, triazole ring, 1,2,3-triazole ring, 1,2,4-triazole ring, 1,2,5-triazole ring, 1,3,4-triazole ring, tetrazole ring, isoxazole ring, oxadiazole ring, 1,2,3-oxadiazole ring, 1,2,4-oxadiazole ring, 1,2,5-oxadiazole ring, 1,3,4-oxadiazole ring, thiadiazole ring, pyridine ring, pyridazine ring, pyrimidine ring, pyrazine ring, triazine ring and tetrazine ring.

In some embodiments, 5- to 6-membered heteroaryl ring formed by connecting R_{Z3} and R_{Z4} is selected from the group consisting of: thiophene ring, furan ring, thiazole ring, isothiazole ring, imidazole ring, oxazole ring, pyrrole ring, pyrazole ring, triazole ring, 1,2,3-triazole ring, 1,2,4-triazole ring, 1,2,5-triazole ring, 1,3,4-triazole ring, tetrazole ring, isoxazole ring, oxadiazole ring, 1,2,3-oxadiazole ring, 1,2,4-oxadiazole ring, 1,2,5-oxadiazole ring, 1,3,4-oxadiazole ring, thiadiazole ring, pyridine ring, pyridazine ring, pyrimidine ring, pyrazine ring, triazine ring and tetrazine ring.

In some embodiments, 4- to 6-membered saturated heteromonocycle formed by Rₐ₁, R_{b1} and the linked nitrogen atom is selected from the group consisting of: azetidine, oxetane, tetrahydrofuran ring, tetrahydrothiophene ring, tetrahydropyrrole ring, piperidine ring, piperazine ring, morpholine ring, thiomorpholine ring, thiomorpholin-1,1-dioxide and tetrahydropyran ring.

In some embodiments, 4- to 6-membered saturated heteromonocycle formed by Rₐ₀, R_{b0} and the linked nitrogen atom is selected from the group consisting of: azetidine, oxetane, tetrahydrofuran ring, tetrahydrothiophene ring, tetrahydropyrrole ring, piperidine ring, piperazine ring, morpholine ring, thiomorpholine ring, thiomorpholin-1,1-dioxide and tetrahydropyran ring.

In some embodiments, ring A is benzene ring or 5- to 6-membered heteroaryl ring; benzene ring and 5- to 6-membered heteroaryl ring are unsubstituted or substituted by 1, 2, 3 or 4 substituents each independently selected from the group consisting of: deuterium, halogen, cyano, hydroxyl, carboxyl, amino, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NH(C₁₋₃ alkyl), N(C₁₋₃ alkyl)₂, -SO₂C₁₋₃ alkyl, -C(O)NH₂, -C(O)NH(C₁₋₃ alkyl), -C(O)N(C₁₋₃ alkyl)₂, -C(O)C₁₋₃ alkyl, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from the group consisting of: halogen, cyano, hydroxy, carboxyl, amino, C₁₋₃ alkyl, C₁₋₃ alkoxy, halo-C₁₋₃ alkyl, halo-C₁₋₃ alkoxy, NH(C₁₋₃ alkyl), N(C₁₋₃ alkyl)₂, -SO₂C₁₋₃ alkyl, -C(O)NH₂, -C(O)NH(C₁₋₃ alkyl), -C(O)N(C₁₋₃ alkyl)₂, -C(O)OC₁₋₃ alkyl, -OC(O)C₁₋₃ alkyl, C₃₋₆ cycloalkyl, C₃₋₆ cycloalkyloxy and 3- to 6-membered heterocycloalkyl.

In some embodiments, R₀ is of the structure represented by formula (b):

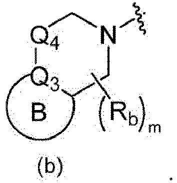

In some embodiments, m in formula (b) is 0, 1 or 2; each R_{b} is identical or different, and each R_{b} is independently deuterium, halogen (preferably fluorine or chlorine) or C₁₋₃ alkyl (preferably methyl); In some embodiments, ring B is benzene ring or 5- to 6-membered heteroaryl ring; benzene ring and 5- to 6-membered heteroaryl ring are unsubstituted or substituted by 1, 2, 3 or 4 substituents each independently selected from the group consisting of: deuterium, halogen, cyano, hydroxyl, carboxyl, amino, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NH(C₁₋₃ alkyl), N(C₁₋₃ alkyl)₂, -SO₂C₁₋₃ alkyl, -C(O)NH₂, -C(O)NH(C₁₋₃ alkyl), -C(O)N(C₁₋₃ alkyl)₂, -C(O)C₁₋₃ alkyl, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from the group consisting of: halogen, cyano, hydroxy, carboxyl, amino, C₁₋₃ alkyl, C₁₋₃ alkoxy, halo-C₁₋₃ alkyl, halo-C₁₋₃ alkoxy, NH(C₁₋₃ alkyl), N(C₁₋₃ alkyl)₂, -SO₂C₁₋₃ alkyl, -C(O)NH₂, -C(O)NH(C₁₋₃ alkyl), -C(O)N(C₁₋₃ alkyl)₂, -C(O)OC₁₋₃ alkyl, -OC(O)C₁₋₃ alkyl, C₃₋₆ cycloalkyl, C₃₋₆ cycloalkyloxy and 3- to 6-membered heterocycloalkyl.

In some embodiments, ring C is benzene ring or 5- to 6-membered heteroaryl ring; benzene ring and 5- to 6-membered heteroaryl ring are unsubstituted or substituted by 1, 2, 3 or 4 substituents each independently selected from the group consisting of: deuterium, halogen, cyano, hydroxyl, carboxyl, amino, C₁₋₃ alkyl, hydroxyl-substituted C₁₋₃ alkyl, C₁₋₃ alkoxy, C₂₋₄ alkenyl, C₂₋₄ alkynyl, halo-C₁₋₃ alkyl, halo-C₁₋₃ alkoxy, NH(C₁₋₃ alkyl), N(C₁₋₃ alkyl)₂, -SO₂C₁₋₃ alkyl, -C(O)NH₂, -C(O)NH(C₁₋₃ alkyl), -C(O)N(C₁₋₃ alkyl)₂, -C(O)C₁₋₃ alkyl, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from the group consisting of: halogen, cyano, hydroxy, carboxyl, amino, C₁₋₃ alkyl, C₁₋₃ alkoxy, halo-C₁₋₃ alkyl, halo-C₁₋₃ alkoxy, NH(C₁₋₃ alkyl), N(C₁₋₃ alkyl)₂, -SO₂C₁₋₃ alkyl, -C(O)NH₂, -C(O)NH(C₁₋₃ alkyl), -C(O)N(C₁₋₃ alkyl)₂, -C(O)OC₁₋₃ alkyl, -OC(O)C₁₋₃ alkyl, C₃₋₆ cycloalkyl, C₃₋₆ cycloalkyloxy and 3- to 6-membered heterocycloalkyl.

In some embodiments, ring C is benzene ring; benzene ring is unsubstituted or substituted by 1, 2, 3 or 4 substituents each independently selected from the group consisting of: deuterium, halogen (preferably fluorine or chlorine), cyano, hydroxyl, carboxyl, amino, C₁₋₃ alkyl (preferably methyl), hydroxy-substituted C₁₋₃ alkyl (preferably hydroxymethyl), C₁₋₃ alkoxy (preferably methoxy), halo-C₁₋₃ alkyl (preferably trifluoromethyl), halo-C₁₋₃ alkoxy, NH(C₁₋₃ alkyl), N(C₁₋₃ alkyl)₂, -SO₂C₁₋₃ alkyl, -C(O)NH₂, -C(O)NH(C₁₋₃ alkyl), -C(O)N(C₁₋₃ alkyl)₂, -C(O)C₁₋₃ alkyl, -C(O)OC₁₋₃ alkyl, -OC(O)C₁₋₃ alkyl, C₃₋₆ cycloalkyl (preferably cyclopropyl), C₃₋₆ cycloalkyloxy and 3- to 6-membered heterocycloalkyl.

In some embodiments, when ring A, ring B and ring C are 5- to 6-membered heteroaryl rings, ring A, ring B and ring C are each independently selected from the group consisting of: thiophene ring, furan ring, thiazole ring, isothiazole ring, imidazole ring, oxazole ring, pyrrole ring, pyrazole ring, triazole ring, 1,2,3-triazole ring, 1,2,4-triazole ring, 1,2,5-triazole ring, 1,3,4-triazole ring, tetrazole ring, isoxazole ring, oxadiazole ring, 1,2,3-oxadiazole ring, 1,2,4-oxadiazole ring, 1,2,5-oxadiazole ring, 1,3,4- oxadiazole ring, thiadiazole ring, pyridine ring, pyridazine ring, pyrimidine ring, pyrazine ring, triazine ring and tetrazine ring.

In some embodiments, when ring A is 5- to 6-membered heteroaryl ring, ring A is selected from the group consisting of pyridine ring, pyrimidine ring, imidazole ring, thiazole ring and pyrazole ring.

In some embodiments, when ring A is 5- to 6-membered heteroaryl ring, ring A is a ring selected from the group consisting of the following structures: 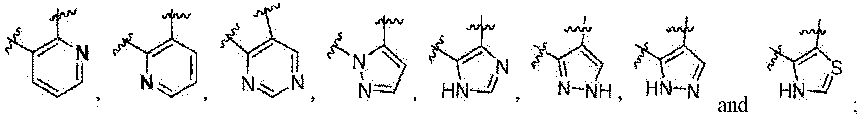 wherein " 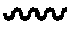 " represents that the two linked ring atoms share a pair of adjacent atoms with the other ring to which they are fused, and the above rings are each optionally substituted by 1, 2, 3 or 4 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)C₁₋₃ alkyl, -C(O)OC₁₋₃ alkyl, - OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from the substituent group S, wherein Rₐ₁, R_{b1} and the substituent group S are as defined above.

In some embodiments, the structure represented by formula (a) is selected from the group consisting of: 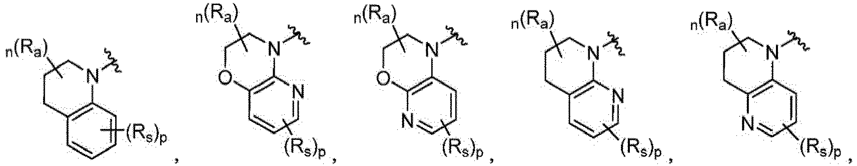 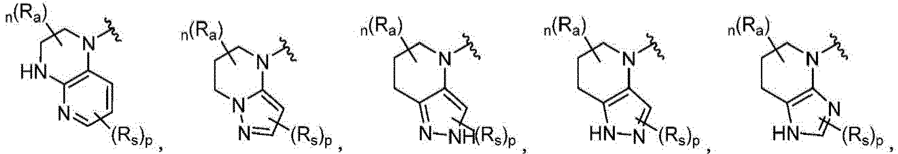 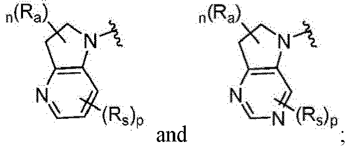 wherein (Rₐ)ₙ is as defined above; (Rs)ₚ represent p Rₛ that substitute hydrogens on the benzene or heteroaryl ring, and p is 0, 1, 2, 3 or 4; each Rₛ is identical or different, and each Rₛ is independently deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)C₁₋₃ alkyl, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl or 5- to 6-membered heteroaryl; wherein 3- to 6-membered heterocycloalkyl, phenyl and 5- to 6-membered heteroaryl are each optionally substituted by 1, 2 or 3 substituents each independently selected from the substituent group S; wherein Rₐ₁, R_{b1} and the substituent group S are as defined above.

In some embodiments, Rₛ is identical or different, and is each independently deuterium, fluorine, chlorine, bromine, cyano, hydroxyl, carboxyl, amino, C₁₋₃ alkyl, C₁₋₃ alkoxy, halo-C₁₋₃ alkyl, halo-C₁₋₃ alkoxy, NH(C₁₋₃ alkyl), N(C₁₋₃ alkyl)₂, -SO₂C₁₋₃ alkyl, -C(O)NH₂, -C(O)NH(C₁₋₃ alkyl), -C(O)N(C₁₋₃ alkyl)₂, -C(O)C₁₋₃ alkyl, -C(O)OC₁₋₃ alkyl, -OC(O)C₁₋₃ alkyl, cyclopropyl, cyclobutyl, cyclopentyl, cyclohexyl, cyclopropyloxy, cyclobutyloxy, cyclopentyloxy, cyclohexyloxy, 3 - to 6- heterocycloalkyl, phenyl or 5- to 6- heteroaryl; wherein 3- to 6- heterocycloalkyl, phenyl and 5 to 6 heteroaryl are each optionally substituted with 1, 2 or 3 substituents each independently selected from the substituent group S; wherein the substituent group S is as defined above.

In some embodiments, when ring B is 5- to 6-membered heteroaryl ring, ring B is selected from the group consisting of imidazole ring, pyrazole ring, 1,2,4-triazole ring, thiazole ring, oxazole ring and pyridine ring.

In some embodiments, ring B is thiazole ring; and thiazole ring is unsubstituted or substituted by NH₂.

In some embodiments, when ring B is 5- to 6-membered heteroaryl ring, ring B is selected from the group consisting of: 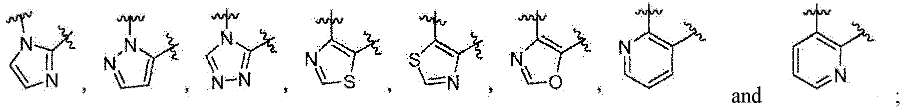 wherein " 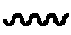 " represents that the two linked ring atoms share a pair of adjacent atoms with the other ring to which they are fused, and the above rings are each optionally substituted by 1, 2, 3 or 4 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)C₁₋₃ alkyl, -C(O)OC₁₋₃ alkyl, - OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from the substituent group S; wherein Rₐ₁, R_{b1} and the substituent group S are as defined above.

In some embodiments, ring B is 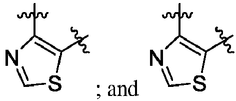 is unsubstituted or substituted by NH₂.

In some embodiments, the structure represented by formula (b) is selected from the group consisting of: 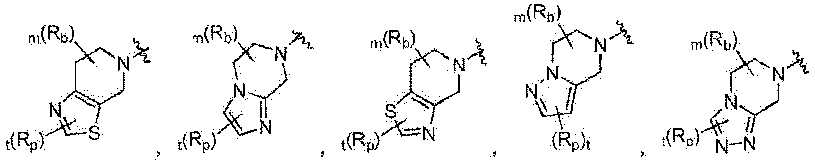 and 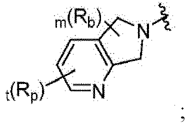 wherein (R_{b})ₙ is as as defined above; (Rₚ)ₜ represent t Rₚ that substitute hydrogens on heteroaryl ring, and t is 0, 1, 2, 3 or 4; each Rₚ is identical or different, and each Rₚ is independently deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)C₁₋₃ alkyl, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl or 5- to 6-membered heteroaryl; wherein 3-to 6-membered heterocycloalkyl, phenyl and 5- to 6-membered heteroaryl are each optionally substituted by 1, 2 or 3 substituents each independently selected from the substituent group S; wherein Rₐ₁, R_{b1} and the substituent group S are as defined above.

In some embodiments, Q₃ is C; Q₄ is CR_{q4}R_{q5}, and R_{q4} and R_{q5} are as defined above.

In some embodiments, Q₃ is C; Q₄ is CR_{q4}R_{q5}; and R_{q4} and R_{q5} are each independently hydrogen, deuterium, C₁₋₃ alkyl (preferably methyl) or halogen (preferably fluorine).

In some embodiments, Q₃ is C; Q₄ is CF₂.

In some embodiments, Q₃ is C; Q₄ is CD₂.

In some embodiments, the structure represented by formula (b) is of the structure represented by formula (b1):

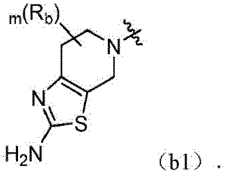

In some embodiments, Rₚ is identical or different, and is each independently deuterium, fluorine, chlorine, bromine, cyano, hydroxyl, carboxyl, amino, C₁₋₃ alkyl, C₁₋₃ alkoxy, halo-C₁₋₃ alkyl, halo-C₁₋₃ alkoxy, NH(C₁₋₃ alkyl), N(C₁₋₃ alkyl)₂, -SO₂C₁₋₃ alkyl, -C(O)NH₂, -C(O)NH(C₁₋₃ alkyl), -C(O)N(C₁₋₃ alkyl)₂, -C(O)C₁₋₃ alkyl, -C(O)OC₁₋₃ alkyl, -OC(O)C₁₋₃ alkyl, cyclopropyl, cyclobutyl, cyclopentyl, cyclohexyl, cyclopropyloxy, cyclobutyloxy, cyclopentyloxy, cyclohexyloxy, 3 - to 6- heterocycloalkyl, phenyl or 5- to 6- heteroaryl; wherein 3- to 6- heterocycloalkyl, phenyl and 5 to 6 heteroaryl are each optionally substituted with 1, 2 or 3 substituents each independently selected from the substituent group S; wherein the substituent group S is as defined above.

In some embodiments, when ring C is 5- to 6-membered heteroaryl ring, ring C is selected from the group consisting of pyridine ring and thiazole ring.

In some embodiments, when ring A is 5- to 6-membered heteroaryl ring, ring C is a ring selected from the group consisting of the following structures: 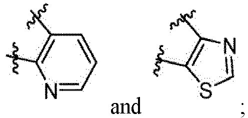 wherein " 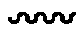 " represents the shared neighboring atom pair when the two linked ring atoms are fused to other rings; the above rings are each optionally substituted by 1, 2, 3 or 4 substituents each independently selected from the group consisting of: deuterium, halogen, cyano, hydroxyl, carboxyl, C₁₋₃ alkyl, hydroxyl-substituted C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)C₁₋₃ alkyl, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from the substituent group S; wherein Rₐ₁, R_{b1} and the substituent group S are as defined above.

In some embodiments, the structure represented by formula (c) is selected from the group consisting of: 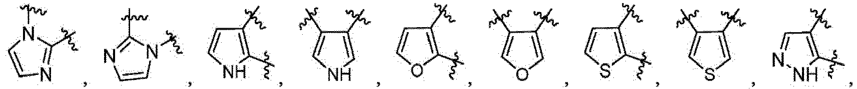 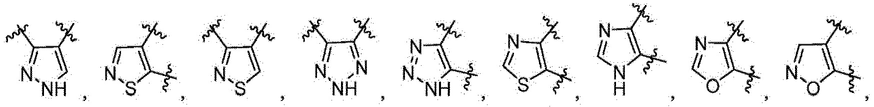 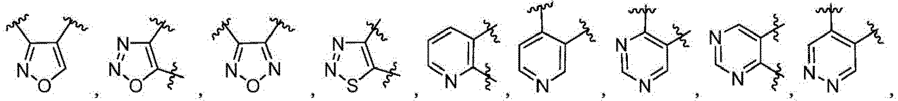 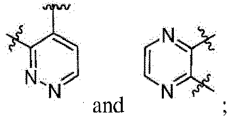 and the above rings are each optionally substituted by 1, 2, 3 or 4 substituents each independently selected from the group consisting of: deuterium, halogen, cyano, hydroxyl, carboxyl, C₁₋₃ alkyl, hydroxyl-substituted C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)C₁₋₃ alkyl, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl are each optionally substituted by 1, 2 or 3 substituents each independently selected from the substituent group S; wherein Rₐ₁, R_{b1} and the substituent group S are as defined above.

In some embodiments, 5- to 6-membered heteroaryl of each group in each structural formula and each group among the substituents as described above is each independently selected from the group consisting of: thienyl, furanyl, thiazolyl, isothiazolyl, imidazolyl, oxazolyl, pyrrolyl, pyrazolyl, triazolyl, 1,2,3-triazolyl, 1,2,4-triazolyl, 1,2,5-triazolyl, 1,3,4-triazolyl, tetrazolyl, isoxazolyl, oxadiazolyl, 1,2,3-oxadiazolyl, 1,2,4-oxadiazolyl, 1,2,5-oxadiazolyl, 1,3,4-oxadiazolyl, thiadiazolyl, pyridinyl, pyridazinyl, pyrimidyl, pyrazinyl, triazinyl and tetrazinyl.

In some embodiments, 5- to 6-membered heteroaryl is selected from the group consisting of: 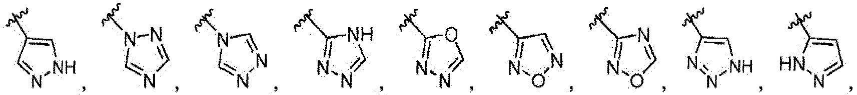 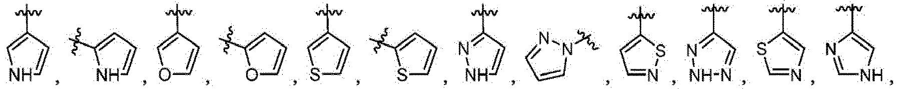 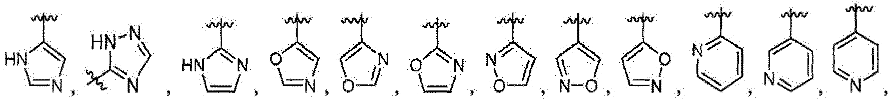 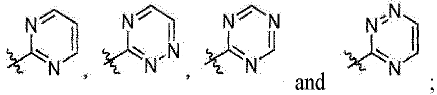 and the above 5- to 6-membered heteroaryl is unsubstituted or substituted by 1, 2 or 3 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)C₁₋₃ alkyl, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl are each optionally substituted by 1, 2 or 3 substituents each independently selected from the substituent group S; wherein the substituent group S, Rₐ₁ and R_{b1} are as defined above.

In some embodiments, 3- to 6-membered heterocycloalkyl of each group in each structural formula and each group among the substituents as described above are 4- to 6-membered heterocycloalkyl, and 3- to 6-membered heterocycloalkyl is each independently selected from the group consisting of: azetidinyl, oxetanyl, tetrahydrofuranyl, tetrahydrothienyl, tetrahydropyrrolyl, oxazolanyl, dioxolanyl, piperidyl, piperazinyl, morpholinyl, dioxanyl, thiomorpholinyl, thiomorpholin-1,1-dioxide, tetrahydropyranyl, pyrrolidon-2-carbonyl, dihydrofuryl-2(3H)-carbonyl, morpholin-3-carbonyl, piperazin-2-carbonyl and piperidin-2-carbonyl.

In some embodiments, in the above structural formulae, 3- to 7-membered saturated or partially unsaturated heteromonocycles formed in each group are each independently selected from the group consisting of: azetidine ring, oxetane ring, tetrahydrofuran ring, tetrahydrothiophene ring, tetrahydropyrrole ring, piperidine ring, pyrroline ring, oxazolidine ring, piperazine ring, dioxolane ring, dioxane ring, morpholine ring, thiomorpholine ring, thiomorpholin-1,1-dioxide, tetrahydropyran ring, azetidin-2-one ring, oxetan-2-one ring, pyrrolidin-2-one ring, pyrrolidin-2,5-dione ring, piperidin-2-one ring, dihydrofuran-2(3H)-one ring, dihydrofuran-2,5-dione ring, tetrahydro-2H-pyran-2-one ring, piperazin-2-one ring, morpholin-3-one ring, 1,2-dihydroazacyclobutadiene ring, 1,2-dihydrooxacyclobutadiene ring, 2,5-dihydro-1H-pyrrole ring, 2,5-dihydrofuran ring, 2,3-dihydrofuran ring, 2,3-dihydro-1H-pyrrole ring, 3,4-dihydro-2H-pyran ring, 1,2,3,4-tetrahydropyridine ring, 3,6-dihydro-2H-pyran ring, 1,2,3,6-tetrahydropyridine ring, 4,5-dihydro-1H-imidazole ring, 1,4,5,6-tetrahydropyrimidine ring, 3,4,7,8-tetrahydro-2H-1,4,6-oxadiazolazine ring, 1,6-dihydropyrimidine ring, 4,5,6,7-tetrahydro-1H-1,3-diazepine ring and 2,5,6,7-tetrahydro-1,3,5-oxadiazepine ring.

In some embodiments, in each of the above structural formulae, 3- to 7-membered saturated or partially unsaturated monocycles formed in each group are each independently selected from the group consisting of: cyclopropyl ring, cyclobutyl ring, cyclopentyl ring, cyclopentenyl ring, cyclohexyl ring, cyclohexenyl ring, cyclohexadienyl ring, cycloheptyl ring, cycloheptatrienyl ring, cyclopentanone ring, and cyclopentan-1,3-dione ring.

In some embodiments, the compound of formula (1) is a compound selected from those in Examples.

In some embodiments, the compound of formula (I) is at lease one compound selected from those in Table A or a stereoisomer thereof.

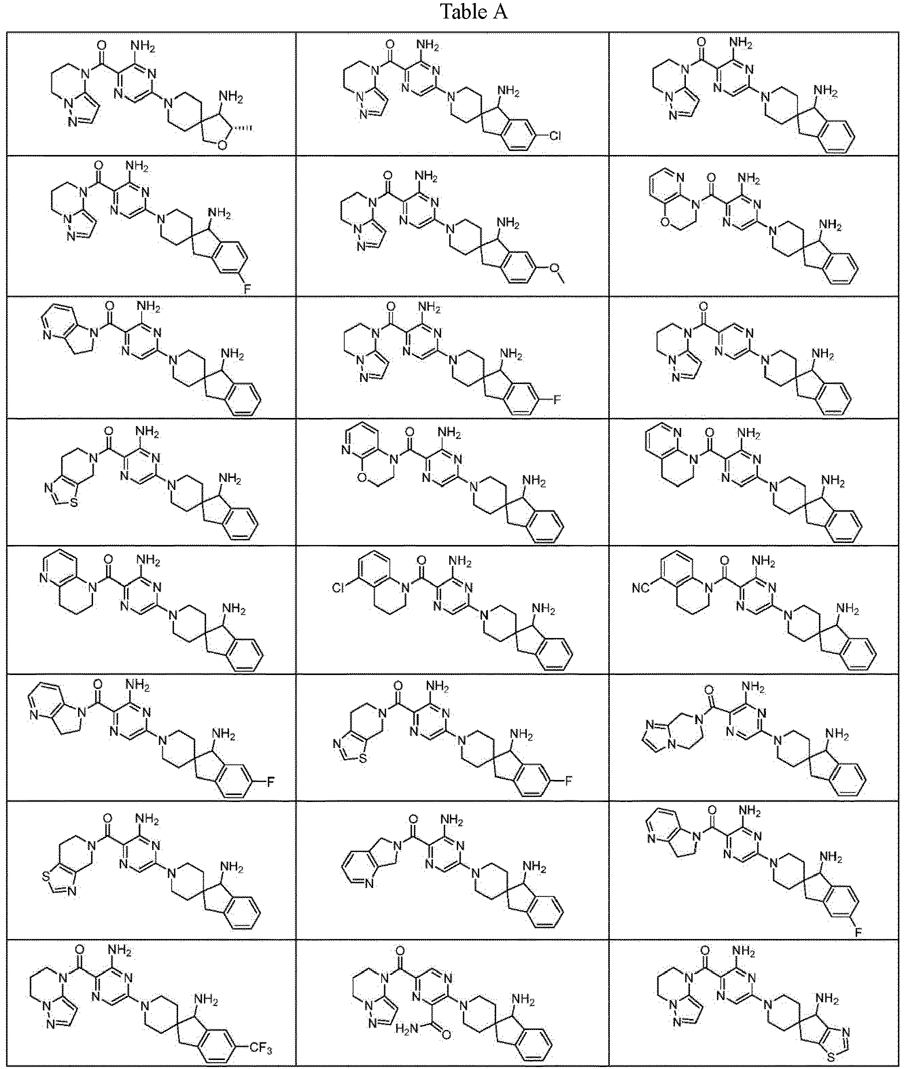

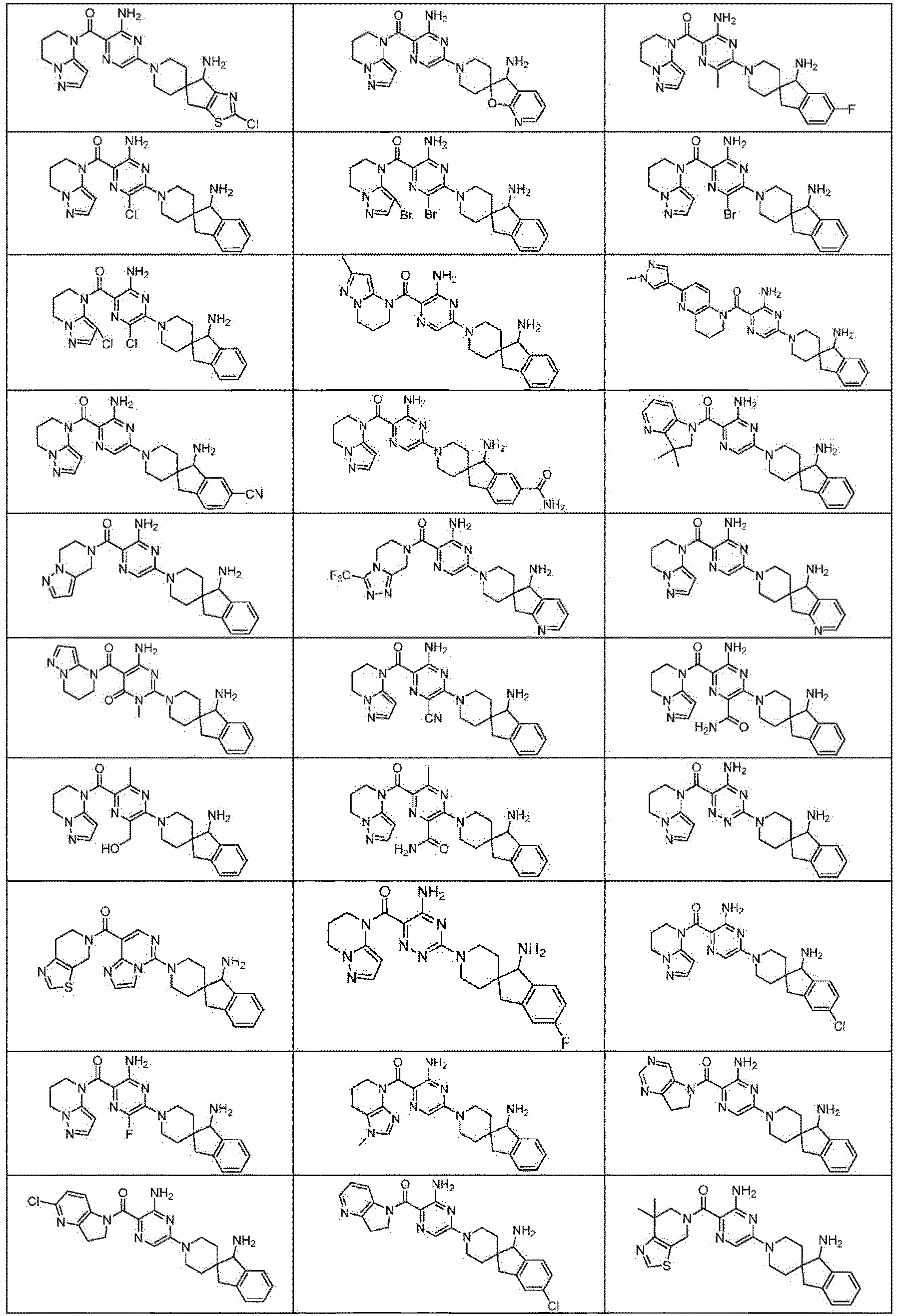

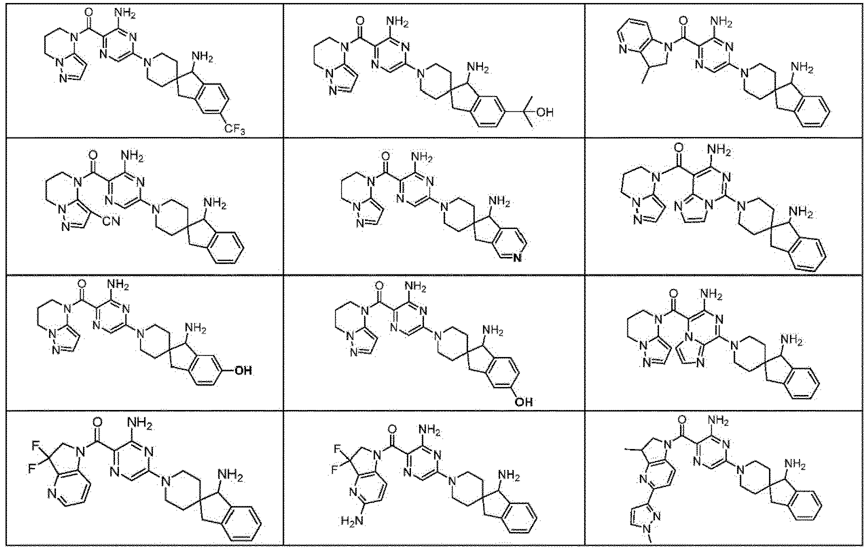

In some embodiments, the compound of formula (I) is at lease one compound selected from those in Table B.

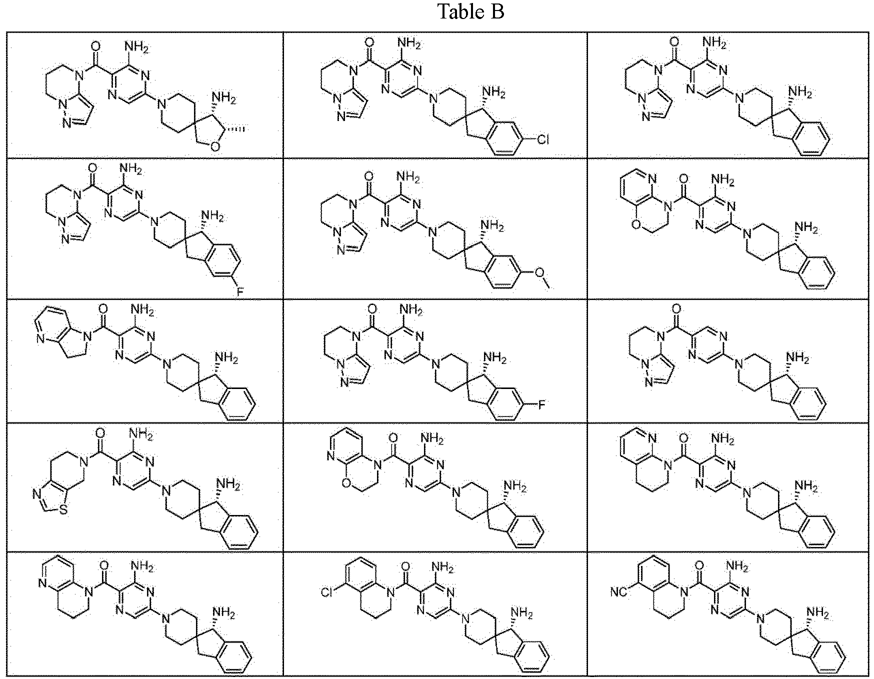

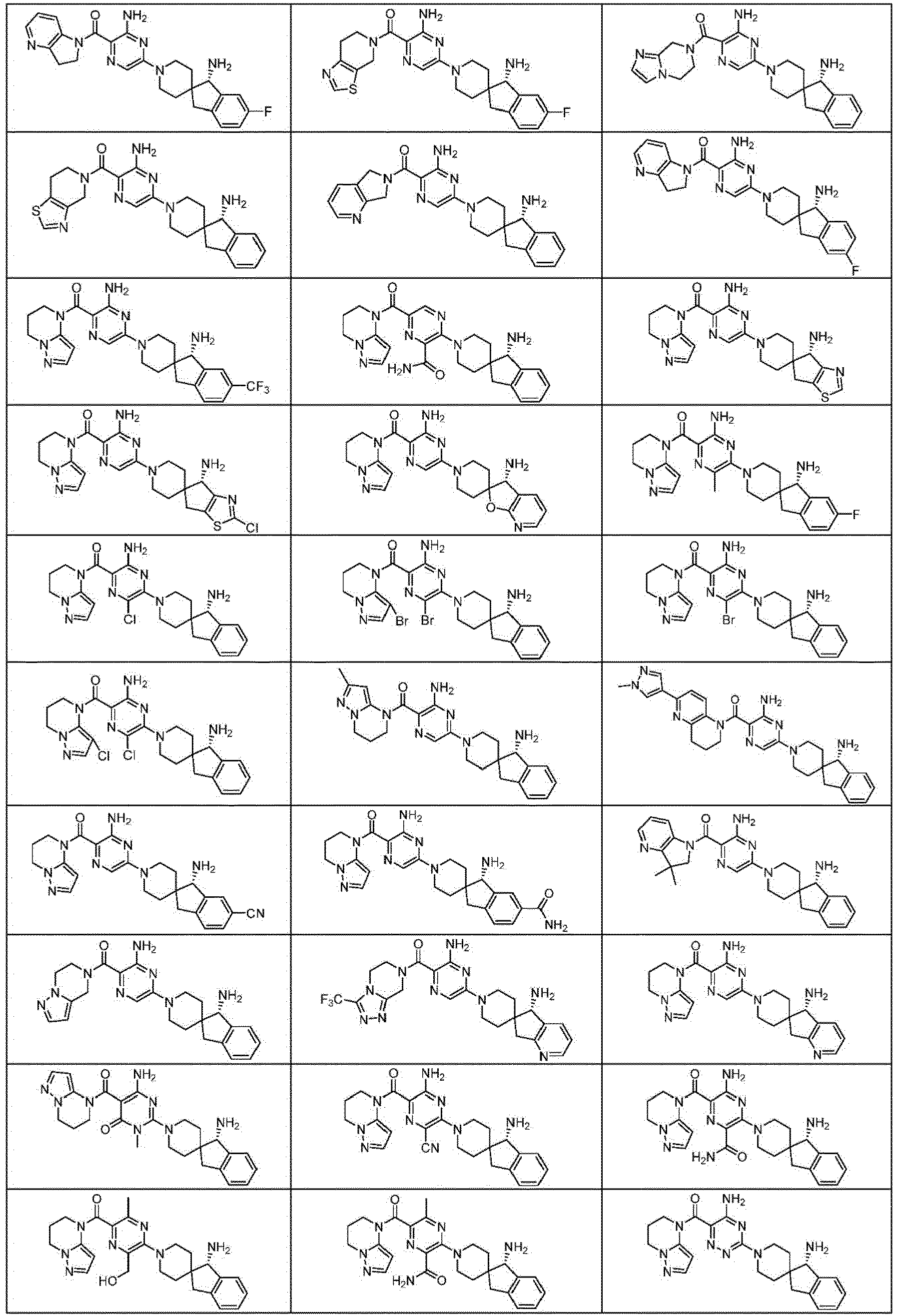

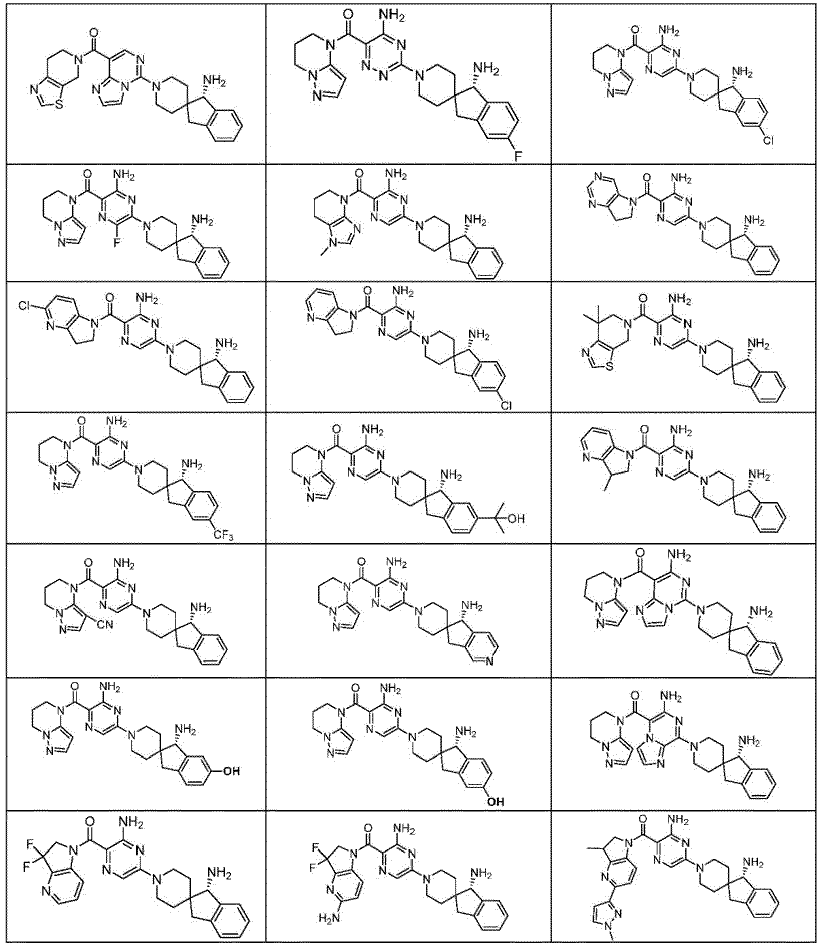

In some embodiments, the compound of formula (I) is at lease one compound selected from those in Table C, and wavy line 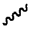 in Table C represents 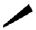 or 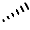 .

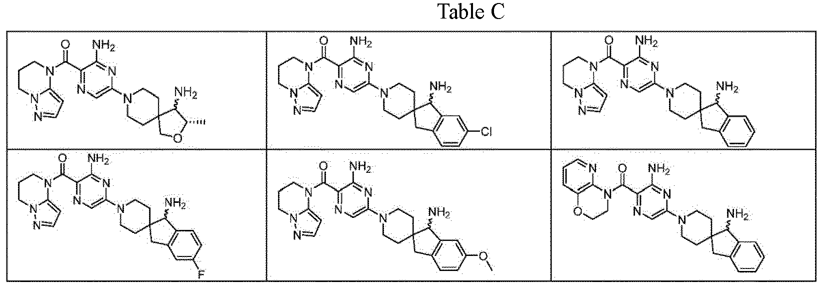

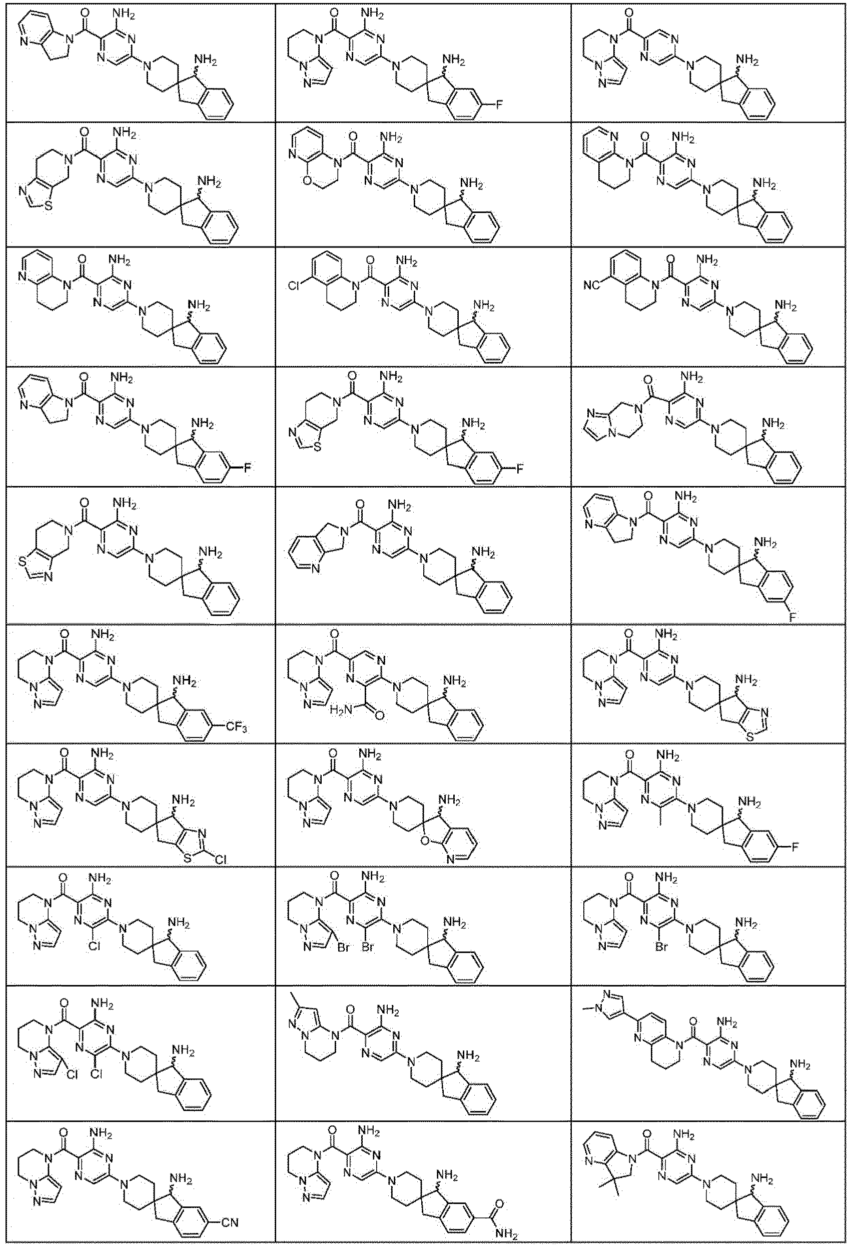

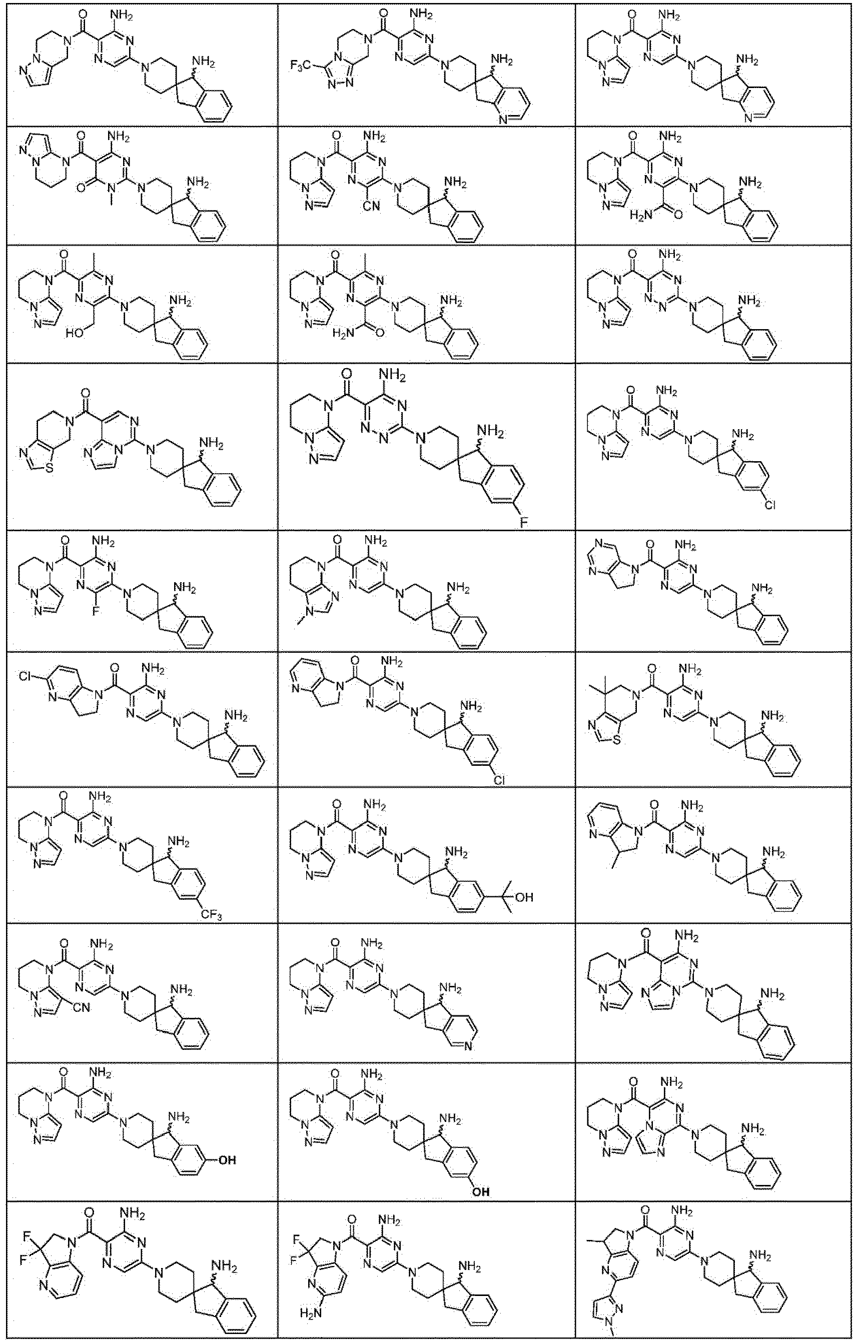

In some embodiments, the compound of formula (I) is at lease one compound selected from those in Table D.

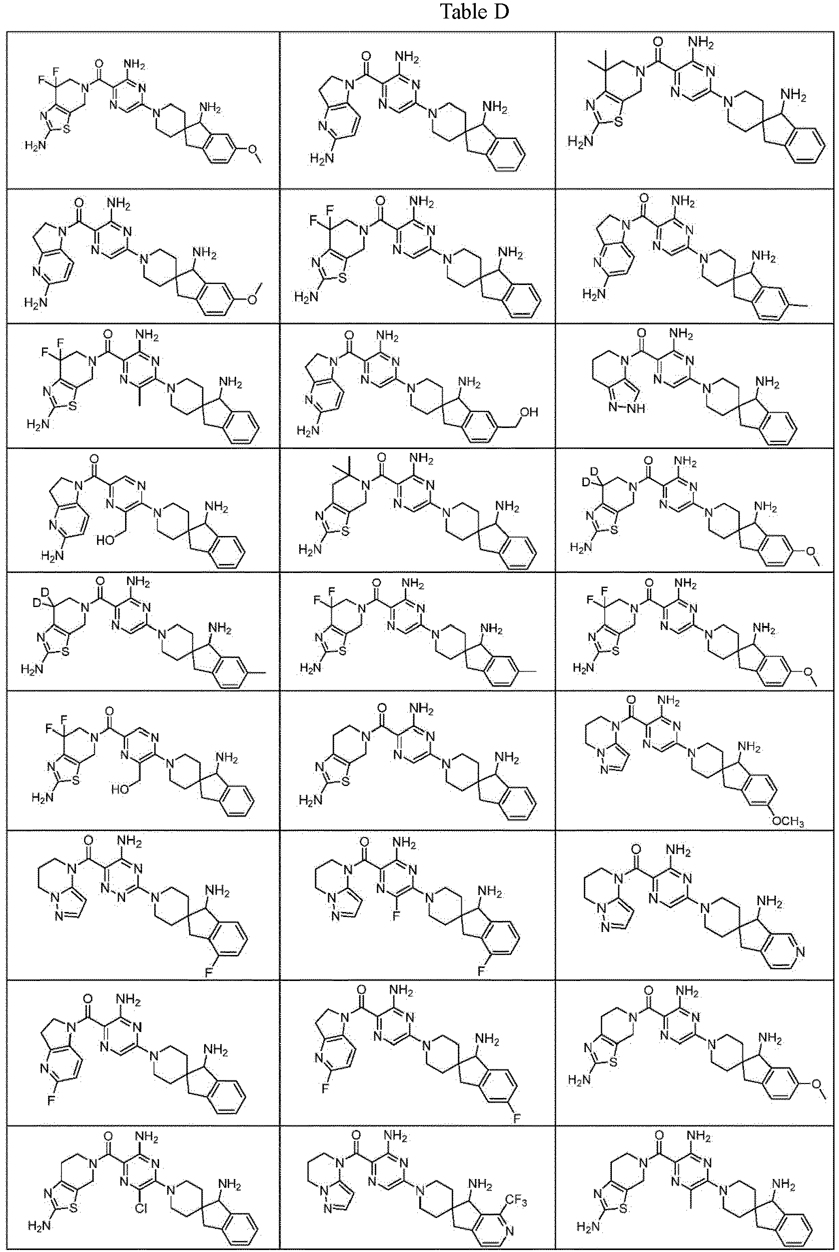

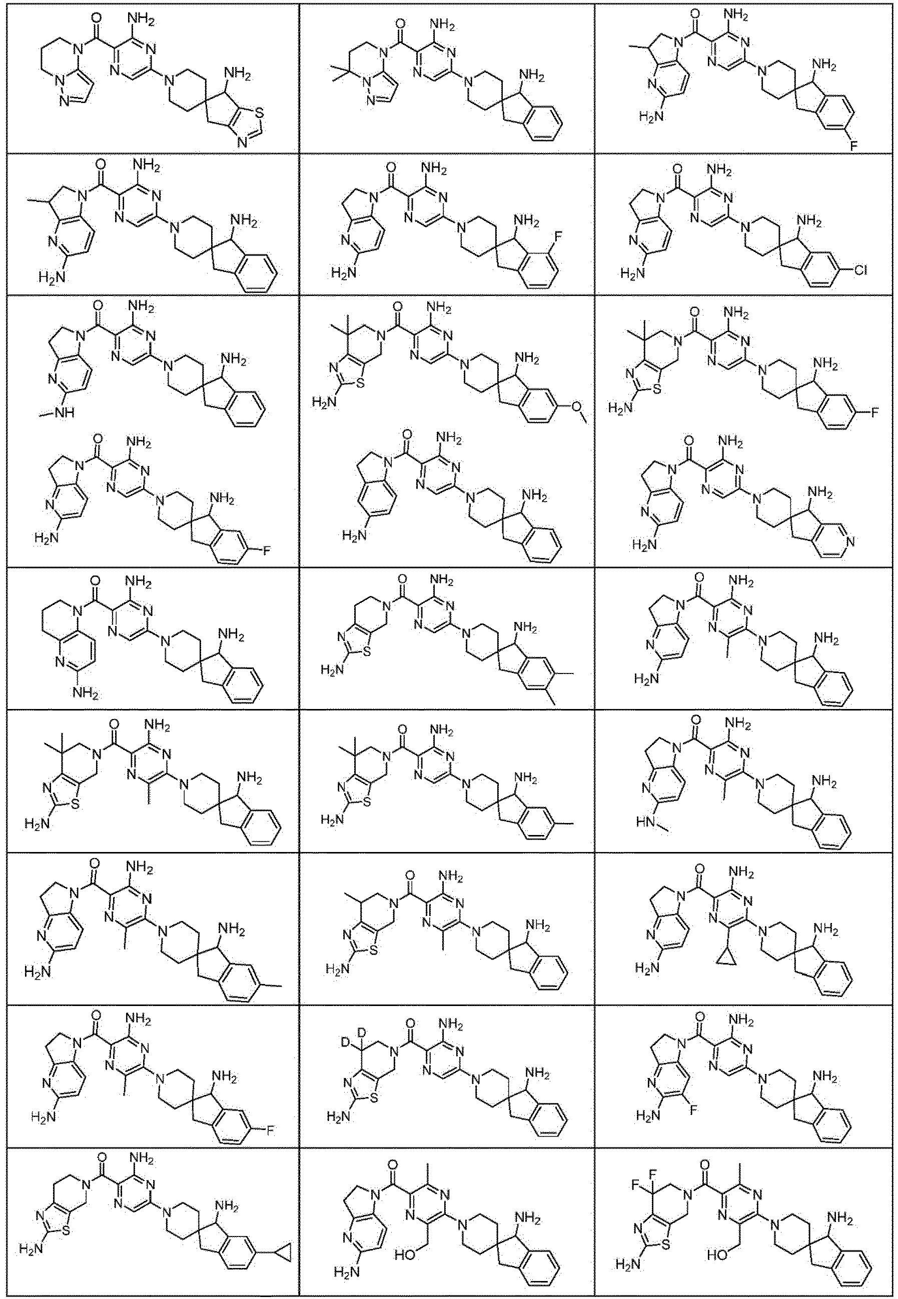

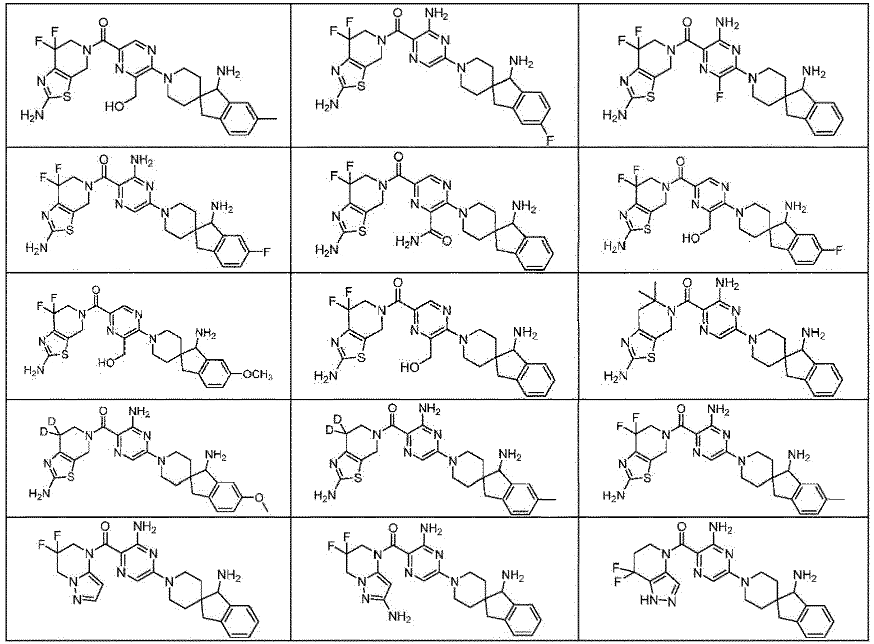

In some embodiments, the compound of formula (I) is at lease one compound selected from those in Table E.

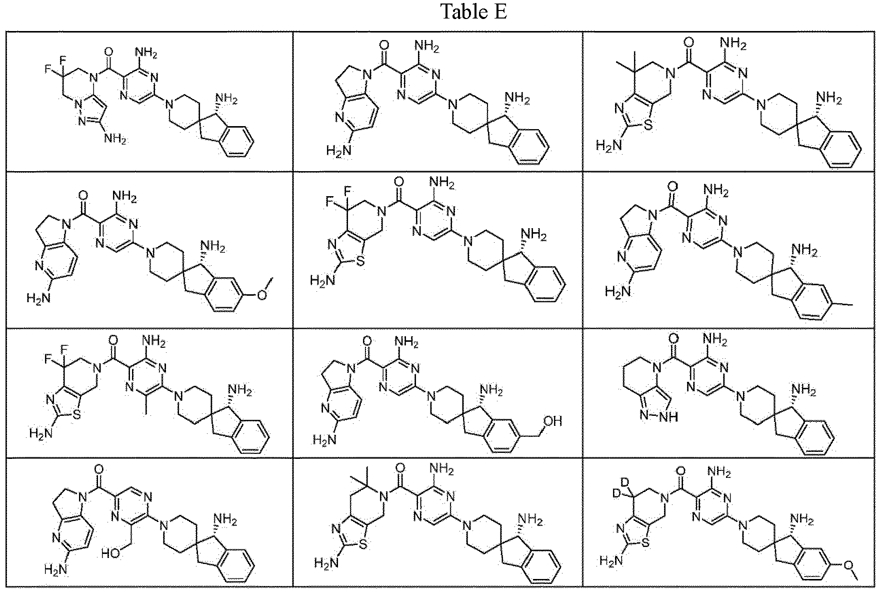

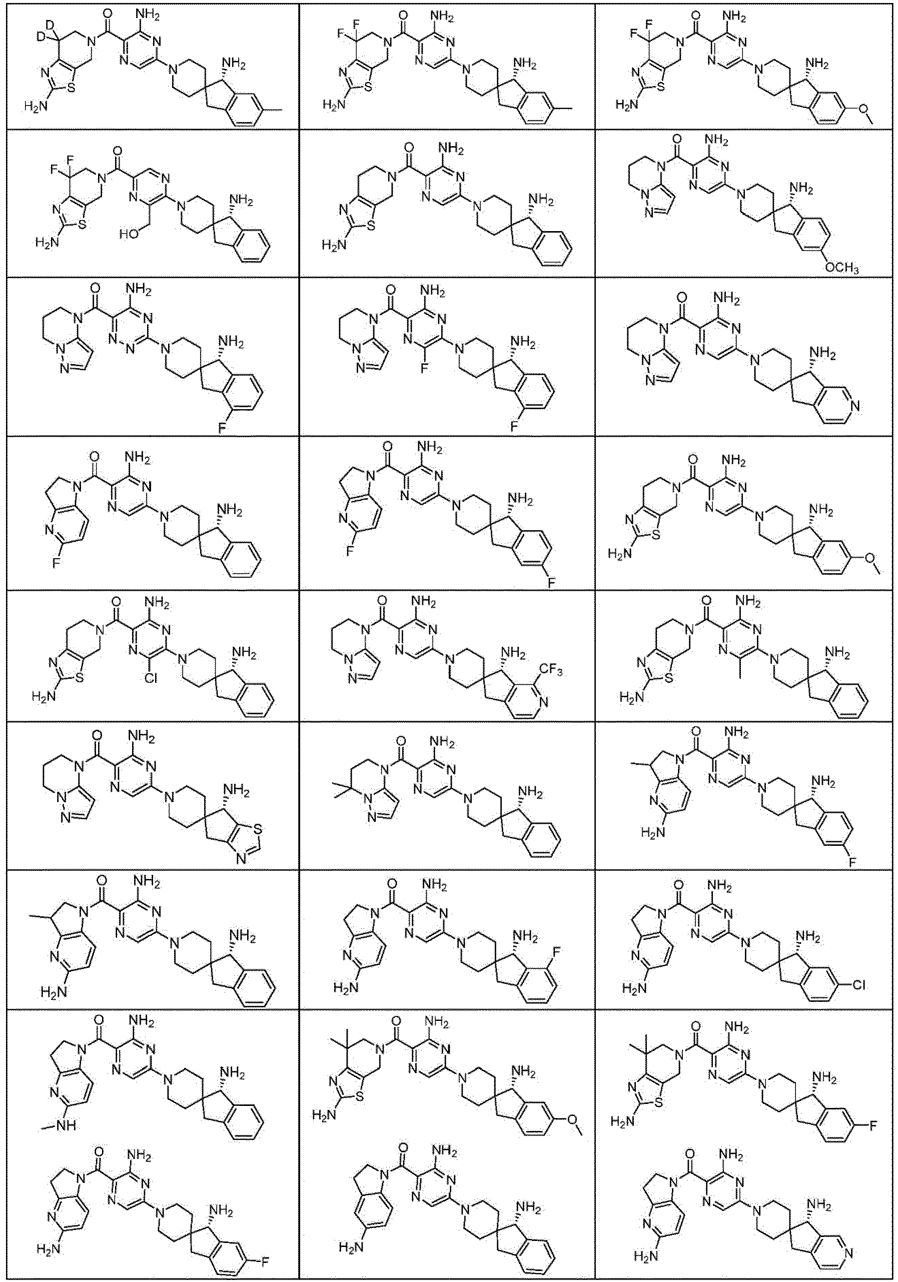

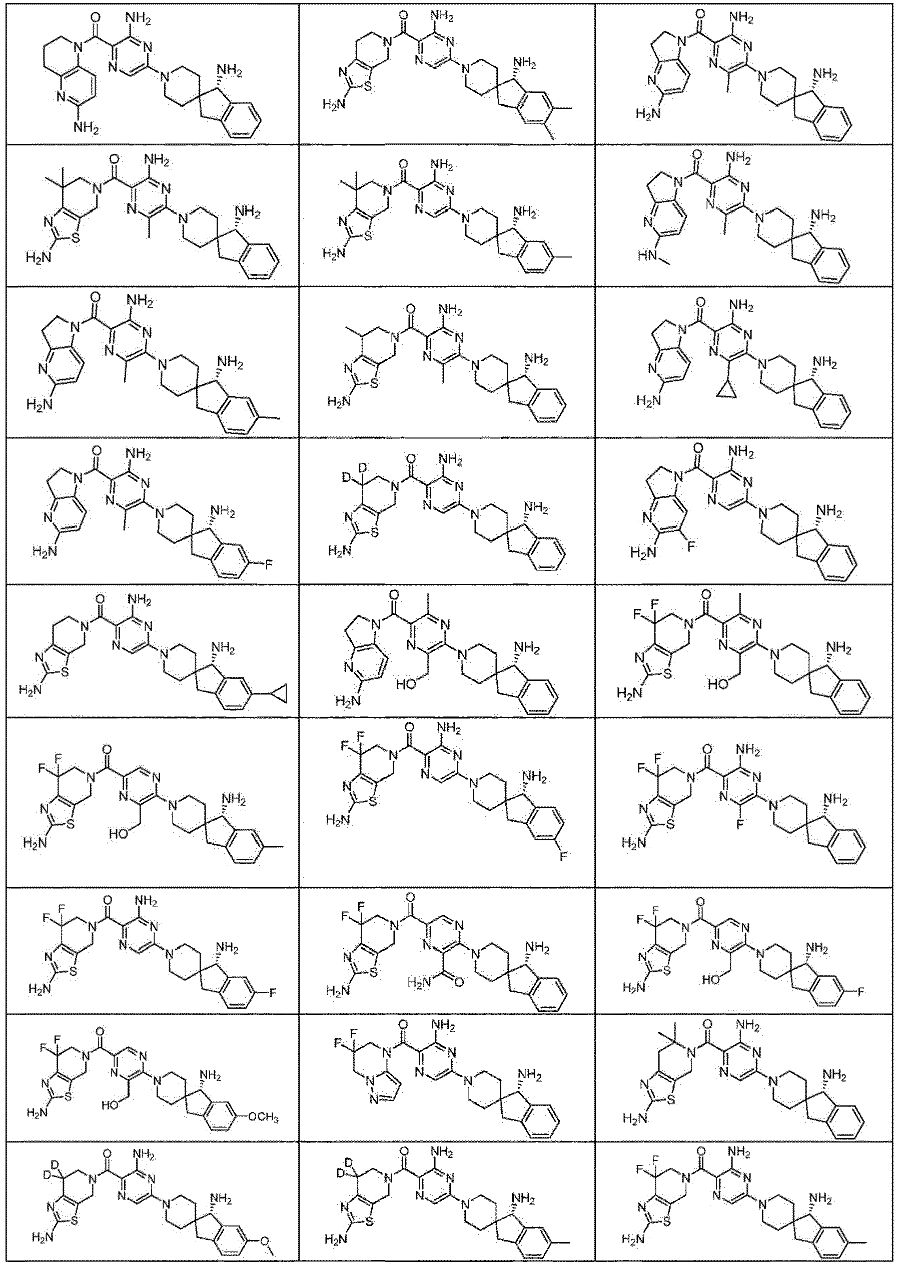

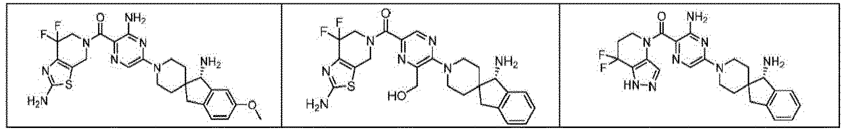

In some embodiments, the compound of formula (I) is at lease one compound selected from those in Table F, and wavy line 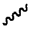 in Table F represents 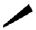 or 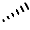 .

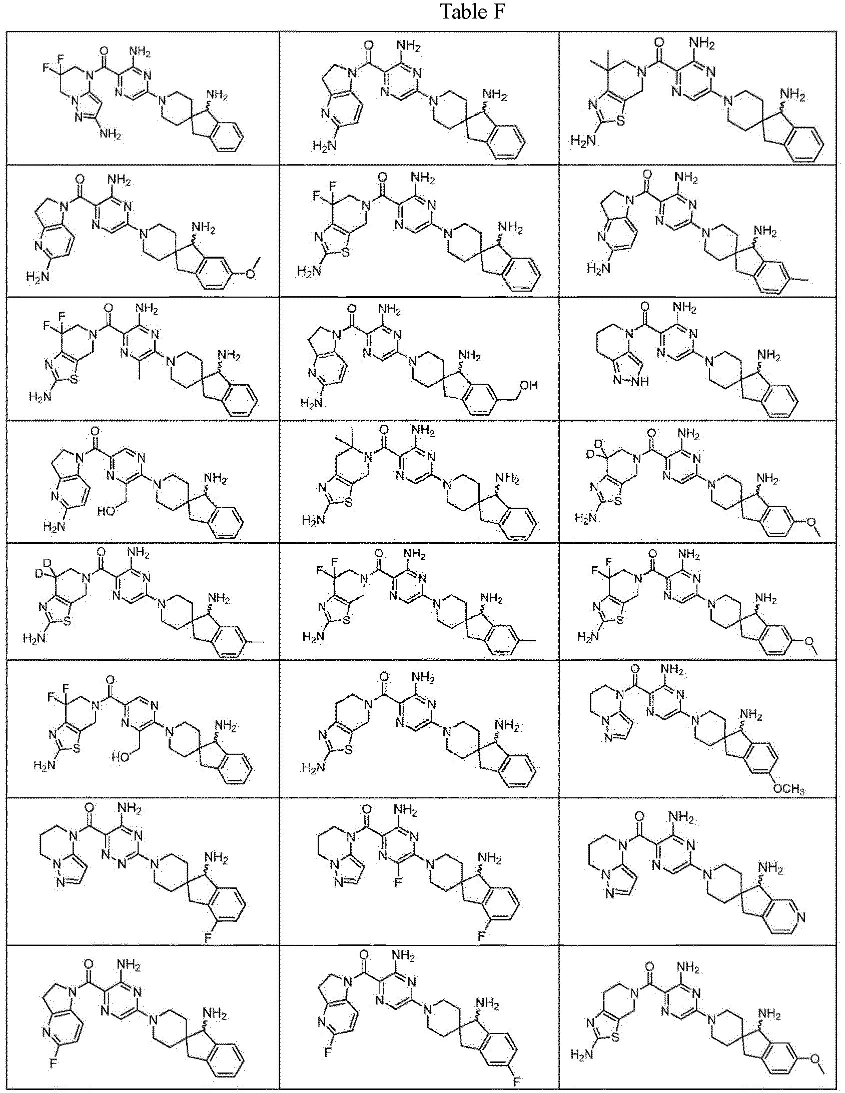

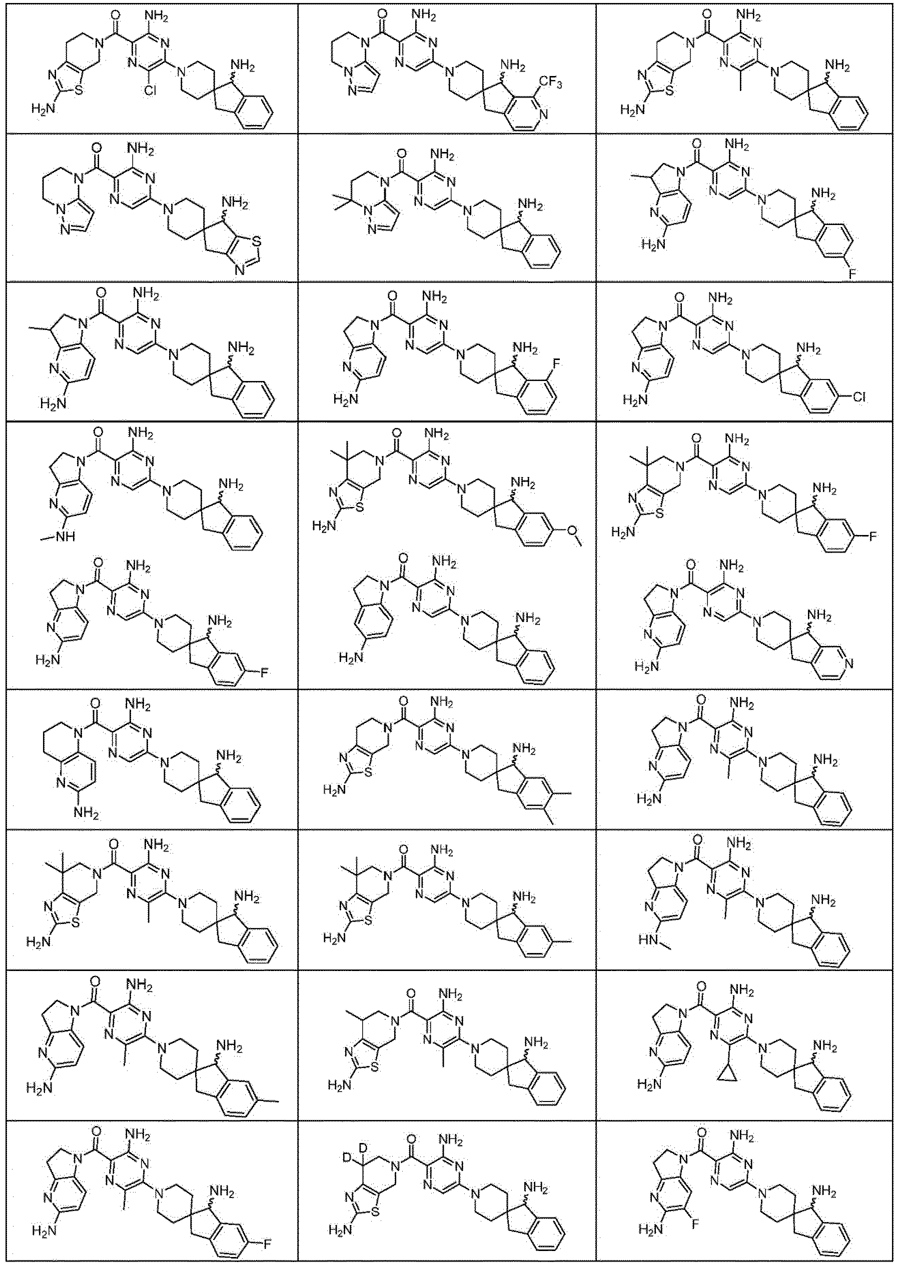

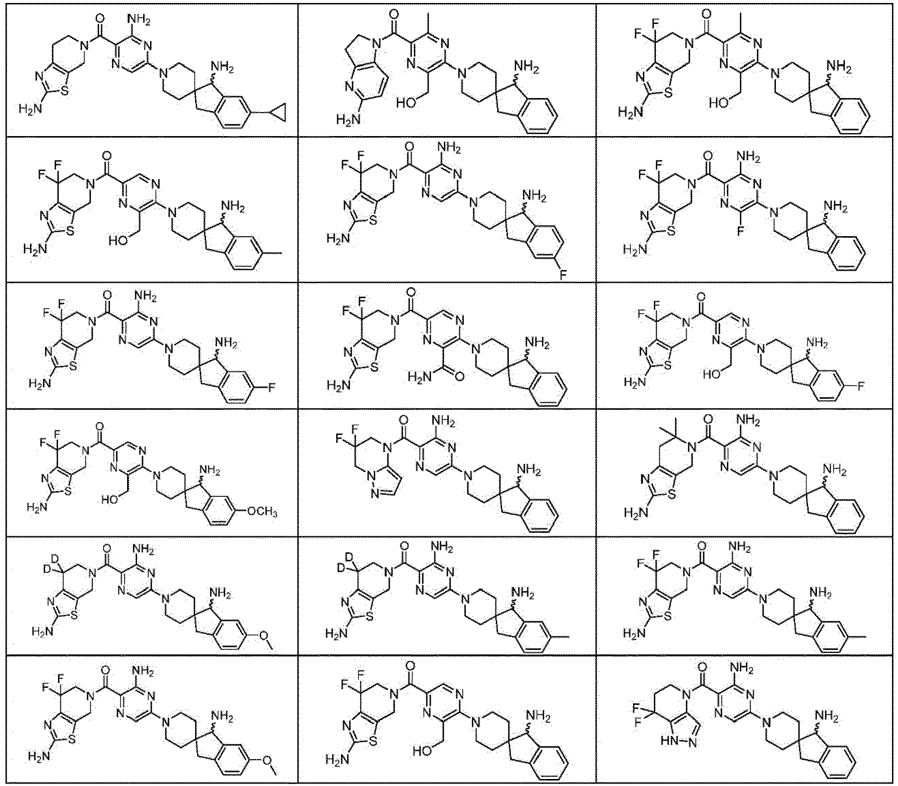

A second aspect of the present invention provides a pharmaceutical composition comprising a compound described in the first aspect of the present invention, or a pharmaceutically acceptable salt thereof, or a stereoisomer thereof, and a pharmaceutically acceptable carrier.

A third aspect of the present invention provides use of the compound described in the first aspect of the present invention, or a pharmaceutically acceptable salt thereof, or a stereoisomer thereof, and the pharmaceutical composition described in the second aspect of the present invention, in the preparation of a medicament for the treatment of a disease or condition mediated by SHP2 or associated with aberrant SHP2 activity.

A fourth aspect of the present invention provides a method for the treatment of a disease or condition mediated by SHP2 or associated with aberrant SHP2 activity, wherein the method comprises administering to a patient a therapeutically effective amount of the compound described in the first aspect of the present invention, or a pharmaceutically acceptable salt thereof, or a stereoisomer thereof, or the pharmaceutical composition as described in the second aspect of the present invention.

Src homology-2 phosphatase (SHP2) that contributes to a variety of cellular functions including proliferation, differentiation, cell cycle maintenance and migration is a protein tyrosine phosphatase encoded by PTPN11 gene. SHP2 is involved in signaling through Ras-mitogen-activated protein kinases, i.e., JAK-STAT or phosphatidylinositol 3-kinase-AKT pathway. SHP2 mediates the activation of Erk1 and Erk2 (Erk1/2, Erk) MAP kinases through receptor tyrosine kinases such as ErbBl, ErbB2 and c-Met.

SHP2 has two N-terminal Src homology-2 domains (N-SH2 and C-SH2), a catalytic domain (PTP) and a C-terminal tail. These two SH2 domains control the subcellular localization and functional regulation of SHP2. The molecule of SHP2 exists in an inactive conformation and inhibits its own activity by binding network inhibition involving residues from both the N-SH2 and PTP domains. In response to growth factor stimulation, SHP2 binds to specific tyrosine-phosphorylated sites on docking proteins such as Gab1 and Gab2 through the SH2 domain thereof, which induces conformational changes leading to SHP2 activation.

In some embodiments, the disease or condition associated with aberrant SHP2 activity includes: solid tumors and hematologic tumors. In some embodiments, the SHP2-mediated disease or condition is cancer, including, but not limited to: juvenile myelomonocytic leukemia (JMML), acute myeloid leukemia (AML), B-cell acute lymphoblastic leukemia (B-ALL), neuroblastoma, esophageal cancer, breast cancer, lung cancer, colon cancer, gastric cancer and head and neck cancer.

It should be understood that each of the above-described technical features of the present invention and each of the technical features specifically described below (e.g., in the Examples) can be combined with each other within the scope of the present invention, thereby constituting new or preferred technical solutions. For the sake of limitation of space, not all of them will be described herein.

### BRIEF DESCRIPTION OF THE DRAWINGS

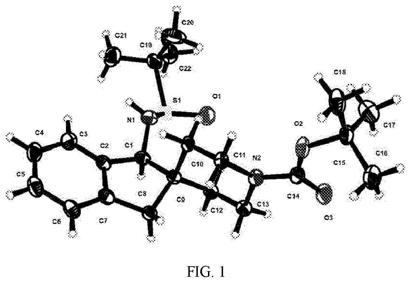

FIG. 1 is the molecular stereostructural ellipsoid diagram of Compound 3d.

### DETAILED DESCRIPTION

The inventors, after extensive and in-depth studies, have unexpectedly discovered the heterocycle-substituted ketone derivatives with significant SHP2 enzyme inhibitory activity and MV-4-11 cell inhibitory activity. This series of compounds is thus expected to be developed as medicaments for the treatment and/or prevention of diseases or conditions mediated by SHP2 or associated with aberrant SHP2 activity. On this basis, the inventors have accomplished the present invention.

### Definition of Terms

In order to enable a clearer understanding of the technical content of the present invention, the terms of the present invention are further described below.

"Alkyl" refers to linear and branched saturated aliphatic hydrocarbon groups. "C₁₋₈ alkyl" refers to alkyl having 1 to 8 carbon atoms, preferably C₁₋₆ alkyl, more preferably C₁₋₃ alkyl. Non-limiting examples of alkyl include: methyl, ethyl, n-propyl, iso-propyl, n-butyl, iso-butyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched chain isomers thereof, and the like.

"Alkenyl" refers to linear or branched unsaturated aliphatic hydrocarbon groups having one or more carbon-carbon double bonds (C=C), and "C₂₋₄ alkenyl" refers to alkenyl having 2 to 4 carbon atoms. The non-limiting examples of alkenyl include vinyl, propenyl, isopropenyl, n-butenyl, isobutenyl, and the like.

"Alkynyl" refers to linear and branched unsaturated aliphatic hydrocarbon groups having one or more carbon-carbon triple bonds, and "C₂₋₄ alkynyl" refers to alkynyl having 2 to 4 carbon atoms. The non-limiting examples of alkynyl include ethynyl, propynyl, n-butynyl, isobutynyl, and the like.

"Cycloalkyl" and "cycloalkyl ring" are used interchangeably to refer to a saturated monocyclic, bicyclic or polycyclic cyclic hydrocarbon group, which may be fused to aryl or heteroaryl. The cycloalkyl ring may optionally be substituted. In some embodiments, the cycloalkyl ring contains one or more carbonyls, e.g., a group containing oxo. "C₃₋₈ cycloalkyl" refers to monocyclic cycloalkyl having 3 to 8 carbon atoms. Non-limiting examples of cycloalkyl include cyclopropyl, cyclobutyl, cyclopentyl, cyclohexyl, cycloheptyl, cyclooctyl, cyclobutanone, cyclopentanone, cyclopentane-1,3-dione, and the like. C₃₋₆ cycloalkyl is preferred, including cyclopropyl, cyclobutyl, cyclopentyl and cyclohexyl. "Heterocycloalkyl" and "heterocycloalkyl ring" can be used interchangeably to refer to a cycloalkyl comprising at least one heteroatom selected from the group consisting of nitrogen, oxygen and sulphur, which may be fused to aryl or heteroaryl. Heterocycloalkyl ring may optionally be substituted. In some embodiments, the heterocycloalkyl ring contains one or more carbonyls or thiocarbonyls, for example, a group containing oxo or thio. "3- to 6-membered heterocycloalkyl" refers to heterocycloalkyl having 3 to 6 ring atoms, wherein 1 or 2 of ring atoms are heteroatoms selected from nitrogen, oxygen and sulfur. 4- to 6-membered heterocycloalkyl having 4 to 6 ring atoms is more preferred, wherein 1 or 2 ring atoms are heteroatoms selected from the group consisting of nitrogen, oxygen and sulfur. Non-limiting examples include aziridinyl, oxiranyl, azetidinyl, oxetanyl, tetrahydrofuranyl, tetrahydrothienyl, tetrahydropyrrolyl, oxazolidinyl, dioxolanyl, piperidinyl, piperazinyl, morpholinyl, dioxanyl, thiomorpholinyl, thiomorpholin-1,1-dioxide, tetrahydropyranyl, azetidin-2-carbonyl, oxetan-2-carbonyl, dihydrofuran-2(3H)-carbonyl, pyrrolidin-2-carbonyl, pyrrolidin-2,5-dicarbonyl, dihydrofuran-2,5-dicarbonyl, piperidin-2-carbonyl, tetrahydro-2H-pyran-2-carbonyl, piperazin-2-carbonyl, morpholin-3-carbonyl, and the like.

"Heteroaryl" and "heteroaryl ring" can be used interchangeably to refer to a group of a monocyclic, bicyclic, or polycyclic 4n+2 aryl ring system having cyclic carbon atom and cyclic heteroatom (e.g., having 6 or 10 π-electrons shared in a cyclic arrangement), wherein each heteroatom is independently selected from the group consisting of nitrogen, oxygen and sulfur. The heteroaryl ring may optionally be substituted. "5- to 6-membered heteroaryl" refers to monocyclic heteroaryl having 5 to 6 ring atoms, wherein 1, 2, 3 or 4 ring atoms are heteroatoms, and non-limiting examples include thienyl, furanyl, thiazolyl, isothiazolyl, imidazolyl, oxazolyl, pyrrolyl, pyrazolyl, triazolyl, 1,2,3-triazolyl, 1,2,4-triazolyl, 1,2,5-triazolyl, 1,3,4-triazolyl, tetrazolyl, isoxazolyl, oxadiazolyl, 1,2,3-oxadiazolyl, 1,2,4-oxadiazolyl, 1,2,5-oxadiazolyl, 1,3,4-oxadiazolyl, thiadiazolyl, pyridinyl, pyridazinyl, pyrimidinyl, pyrazinyl, triazinyl and tetrazinyl. "Heteroatom" refers to nitrogen, oxygen or sulfur. For heteroaryl containing one or more nitrogen atoms, the linkage position may be a carbon or nitrogen atom, as long as the valency allows. Heteroaryl bicyclic systems may include one or more heteroatoms in one or both rings.

"Fused" refers to a structure in which two or more rings share one or more bonds.

"Alkoxy" refers to -O-alkyl, wherein alkyl is as defined above, preferably C₁₋₈ alkoxy, more preferably C₁₋₆ alkoxy, and most preferably C₁₋₃ alkoxy. Non-limiting examples of alkoxy include methoxy, ethoxy, n-propoxy, isopropoxy, butoxy, tert-butoxy, isobutoxy, pentoxy and the like.

"Cycloalkyloxy" refers to -O-cycloalkyl, wherein cycloalkyl is as defined above, preferably C₃₋₈ cycloalkyloxy, and more preferably C₃₋₆ cycloalkyloxy. Non-limiting examples of cycloalkyloxy include cyclopropyloxy, cyclobutoxy, cyclopentyloxy, cyclohexyloxy and the like.

"Heteroatom" refers to nitrogen atom, oxygen atom, sulfur atom or phosphorus atom.

"A bond" refers to one covalent bond that linkes two groups.

"Halogen" refers to fluorine, chlorine, bromine or iodine.

"Halo" refers to halogen atom that substitutes one or more (e.g., 1, 2, 3, 4 or 5) hydrogens in the group.

For example, "haloalkyl" refers to an alkyl substituted by one or more (e.g., 1, 2, 3, 4 or 5) halogen atoms, and is preferably halo-C₁₋₈ alkyl, more preferably halo-C₁₋₆ alkyl, and more preferably halo-C₁₋₃ alkyl, wherein alkyl is as defined above. Examples of haloalkyl include, but are not limited to, monochloromethyl, dichloromethyl, trichloromethyl, monochloroethyl, 1,2-dichloroethyl, trichloroethyl, monobromoethyl, monofluoromethyl, difluoromethyl, trifluoromethyl, monofluoroethyl, difluoroethyl, trifluoroethyl and the like.

For example, "haloalkoxy" refers to an alkoxy substituted by one or more (e.g., 1, 2, 3, 4 or 5) halogen atoms, preferablyp halo-C₁₋₈ alkoxy, more preferably halo-C₁₋₆ alkoxy, and more preferably halo-C₁₋₃ alkoxy, wherein alkoxy is as defined above. Haloalkoxy includes (but is not limited to) trifluoromethoxy, trifluoroethoxy, monofluoromethoxy, monofluoroethoxy, difluoromethoxy, difluoroethoxy and the like.

For example, "halocycloalkyl" refers to a cycloalkyl substituted by one or more (e.g., 1, 2, 3, 4 or 5) halogen atoms, preferably halo-C₃₋₈ cycloalkyl, and more preferably halo-C₃₋₆ cycloalkyl, wherein cycloalkyl is as defined above. Halocycloalkyl include (but are not limited to) trifluorocyclopropyl, monofluorocyclopropyl, monofluorocyclohexyl, difluorocyclopropyl, difluorocyclohexyl and the like.

"Deuteroalkyl" refers to an alkyl substituted by one or more (e.g., 1, 2, 3, 4 or 5) deuterium atoms, preferably deutero-C₁₋₈ alkyl, more preferably deutero-C₁₋₅ alkyl, and more preferably deutero-C₁₋₃ alkyl, wherein alkyl is as defined above. Examples of deuteroalkyl include, but are not limited to, monodeuteromethyl, monodeuteroethyl, dideuteromethyl, dideuteroethyl, trideuteromethyl, trideuteroethyl and the like.

"Amino" refers to NH₂. "Cyano" refers to CN. "Nitro" refers to NO₂. "Phenylmethyl" refers to -CH₂-phenyl. "Oxo" refers to =O. "Carboxyl" refers to -C(O)OH. "Acetyl" refers to -C(O)CH₃. "Hydroxymethyl" refers to -CH₃. "Hydroxyethyl" refers to -CH₂CH₂OH or -CHOHCH₃. "Hydroxyl" refers to -OH. "Thiol" refers to SH. "Cyclopropylidene" is of the structure

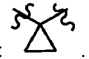

"Saturated or partially unsaturated monocycle" refers to a saturated or partially unsaturated all-carbon monocyclic system, wherein "partially unsaturated" refers to a ring moiety that includes at least one double or triple bond. "Partially unsaturated" is intended to encompass a ring moiety with multiple unsaturated sites, but is not intended to include aryl or heteroaryl moieties as defined herein. In some embodiments, the saturated or partially unsaturated monocycle contains one or more carbonyl, e.g., a group containing oxo. "3- to 7-membered saturated or partially unsaturated monocycle" has 3 to 7 ring carbon atoms, preferably a saturated or partially unsaturated monocycle having 3 to 6 ring carbon atoms, and more preferably a saturated monocycle having 3 to 6 ring carbon atoms. Non-limiting embodiments of saturated or partially unsaturated monocycle include cyclopropyl ring, cyclobutyl ring, cyclopentyl ring, cyclopentenyl ring, cyclohexyl ring, cyclohexenyl ring, cyclohexadienyl ring, cycloheptyl ring, cycloheptatrienyl ring, cyclopentanone ring, cyclopentan-1,3-dione ring and the like.

"Saturated or partially unsaturated heteromonocycle" refers to a saturated or partially unsaturated monocycle in which 1, 2 or 3 ring carbon atoms are substituted by heteroatoms selected from the group consisting of nitrogen, oxygen and S(O)ₜ (wherein t is an integer from 0 to 2), but not including a ring moiety of -O-O-, -O-S-, or -S-S-, and the rest ring atoms are carbon atoms. "3- to 7-membered saturated or partially unsaturated heteromonocycle" has 3 to 7 ring atoms, wherein 1, 2 or 3 ring atoms are heteroatoms as described above, which is preferably 3- to 6-membered saturated or partially unsaturated heteromonocycle having 3 to 6 ring atoms, wherein 1 or 2 ring atoms are heteroatoms as described above, more preferably 5- to 6-membered saturated or partially unsaturated heteromonocycle having 5 to 6 ring atoms, wherein 1 or 2 ring atoms are heteroatoms as described above, and most preferably 5- or 6-membered saturated heteromonocycle. Non-limiting examples of saturated heteromonocycle include epoxypropane ring, azetidine ring, oxetane ring, tetrahydrofuran ring, tetrahydrothiophene ring, tetrahydropyrrole ring, piperidine ring, pyrroline ring, oxazolidine ring, piperazine ring, dioxolane ring, dioxane ring, morpholine ring, thiomorpholine ring, thiomorpholin-1,1-dioxide, tetrahydropyran ring, azetidin-2-one ring, oxetan-2-one ring, pyrrolidin-2-one ring, pyrrolidin-2,5-dione ring, piperidin-2-one ring, dihydrofuran-2(3H)-one ring, dihydrofuran-2,5-dione ring, tetrahydro-2H-pyran-2-one ring, piperazin-2-one ring and morpholin-3-one ring. Non-limiting examples of partially unsaturated heteromonocycle include 1,2-dihydroazetidinyl ring, 1,2-dihydrooxetanyl ring, 2,5-dihydro-1H-pyrrole ring, 2,5-dihydrofuryl ring, 2,3-dihydrofuryl ring, 2,3-dihydro-1H-pyrrole ring, 3,4-dihydro-2H-pyran ring, 1,2,3,4-tetrahydropyridinyl ring, 3,6-dihydro-2H-pyran ring, 1,2,3,6-tetrahydropyridine ring, 4,5-dihydro-1H-imidazole ring, 1,4,5,6-tetrahydropyrimidine ring, 3,4,7,8-tetrahydro-2H-1,4,6-oxadiazolazine ring, 1,6-dihydropyrimidine ring, 4,5,6,7-tetrahydro-1H-1,3-diazepine ring, 2,5,6,7-tetrahydro-1,3,5-oxadiazepine ring, and the like.

"Substituted" refers to a group, in which one or more hydrogen atoms, preferably 1 to 5 hydrogen atoms are each independently substituted by a corresponding number of substituents, and more preferably 1 to 3 hydrogen atoms are each independently substituted by a corresponding number of substituents. It is self-evident that the substituents are only in their possible chemical positions and that those skilled in the art are able to determine (experimentally or theoretically) possible or impossible substitutions without undue effort. For example, amino or hydroxyl having a free hydrogen may be unstable when combined with a carbon atom having an unsaturated (e.g., alkenyl) bond.

Unless otherwise defined, "substituents each independently selected from the group consisting of ......" in the present invention refers to a group with more than one hydrogens substituted by substituents, and substituents may be of identical or different types, and the selected substituents are each of independent type.

Unless otherwise defined, "...... are identical or different and each independently ......" in the present invention refers to more than one identical substituent symbols in a general formula, and the substituents can be identical or different and be of each independent type. For example, when L is (CR₀₁R₀₂)ₛ, and s is 2, i.e., L is (CR₀₁R₀₂)-(CR₀₁R₀₂), wherein the two R₀₁ or R₀₂ can be identical or different and are of each independent type, and e.g., L can be C(CH₃)(CN)-C(CH₂CH₃)(OH), C(CH₃)(CN)-C(CH₃)(OH) or C(CN)(CH₂CH₃)-C(OH)(CH₂CH₃).

Unless otherwise defined, any group herein may be substituted or unsubstituted. When the above groups are substituted, the substituents are preferably 1 to 5 groups each independently selected from the group consisting of cyano, halogen (preferably fluorine or chlorine), C₁₋₈ alkyl (preferably C₁₋₆ alkyl, more preferably C₁₋₃ alkyl), C₁₋₈ alkoxy (preferably C₁₋₆ alkoxy, more preferably C₁₋₃ alkoxy), halo-C₁₋₈ alkyl (preferably halo-C₁₋₆ alkyl, more preferably halo-C₁₋₃ alkyl ), C₃₋₈ cycloalkyl (preferably C₃₋₆ cycloalkyl), halo-C₁₋₈ alkoxy (preferably halo-C₁₋₆ alkoxy, more preferably halo-C₁₋₃ alkoxy), C₁₋₈ alkyl-substituted amino, halo-C₁₋₈ alkyl-substituted amino, acetyl, hydroxyl, hydroxymethyl, hydroxyethyl, carboxyl, nitro, C₆₋₁₀ aryl (preferably phenyl), C₃₋₈ cycloalkyloxy (preferably C₃₋₆ cycloalkyloxy), C₂₋₈ alkenyl (preferably C₂₋₆ alkenyl, more preferably C₂₋₄ alkenyl), C₂₋₈ alkynyl (preferably C₂₋₆ alkynyl, more preferably C₂₋₄ alkynyl), -CONRₐ₀R_{b0}, -C(O)OC₁₋₁₀ alkyl (preferably -C(O)OC₁₋₆ alkyl, more preferably -C(O)OC₁₋₃ alkyl), -CHO, -OC(O)C₁₋₁₀ alkyl (preferably -OC(O)C₁₋₆ alkyl, more preferably -OC(O)C₁₋₃ alkyl), -S0₂C₁₋₁₀ alkyl (preferably -SO₂C₁₋₆ alkyl, more preferably -SO₂C₁₋₃ alkyl), -SO₂C₆₋₁₀ aryl (preferably -SO₂C₆ aryl, such as -SO₂-phenyl), -COC₆₋₁₀ aryl (preferably -COC₆ aryl, such as -CO-phenyl), 4- to 6- membered saturated or unsaturated monocycle, 4- to 6- membered saturated or unsaturated monocycle, 5- to 6- membered monocyclic heteroaryl ring, 8- to 10- membered bicyclic heteroaryl ring, spiro ring, spiro heterocycle, bridged ring and bridged heterocycle, wherein Rₐ₀ and R_{b0} are each independently hydrogen or C₁₋₃ alkyl.

The various types of substituents described herein above per se can be substituted by the groups described herein.

When 4- to 6-membered saturated heteromonocycles described herein are substituted, the positions of the substituents can be at their possible chemical positions, and exemplary substituted heteromonocycles with representative substitutions are as shown below: 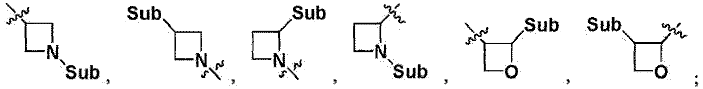 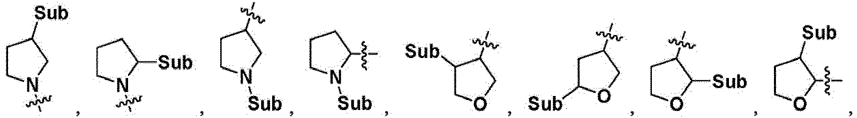 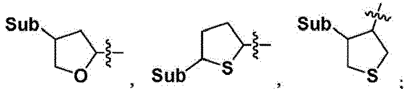 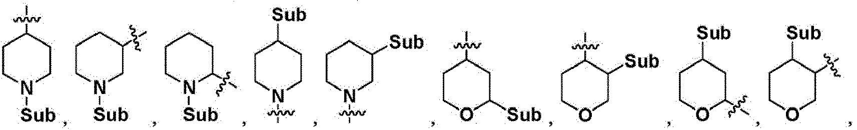 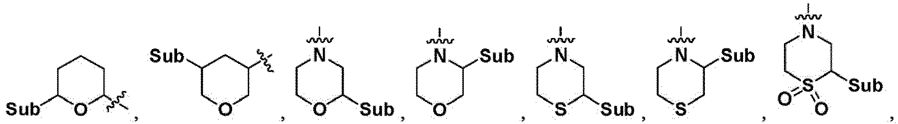 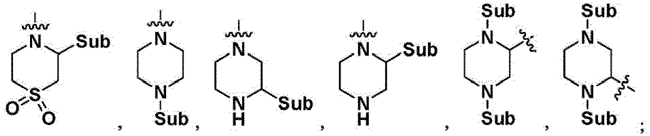 wherein "Sub" represents the various types of substituents described herein; and " 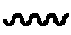 " represents a linkage to other atoms.

### Pharmaceutical Compositions

Generally the compound of the present invention, or the pharmaceutically acceptable salt thereof, or the stereoisomer thereof, can be administered in a suitable dosage form with one or more pharmaceutical carriers. These dosage forms are suitable for oral, rectal, topical, intraoral administration and parenteral administration (e.g., subcutaneous, intramuscular, intravenous administration, and the like). For example, dosage forms suitable for oral administration include capsules, tablets, granules and syrups. The compound of the present invention contained in these formulations may be solid powders or granules; solutions or suspensions in aqueous or non-aqueous liquids; emulsions in water-in-oil or oil-in-water, etc. The above dosage forms can be made from the active compound with one or more carriers or excipients by general pharmaceutical methods. The above carriers need to be compatible with the active compound or other excipients. For solid dosage forms, commonly used non-toxic carriers include, but are not limited to, mannitol, lactose, starch, magnesium stearate, cellulose, glucose, sucrose and the like. Carriers for liquid formulations include water, saline, aqueous glucose solution, ethylene glycol, polyethylene glycol and the like. The active compound can be formed into a solution or a suspension with the above carriers.

"Pharmaceutically acceptable carrier" refers to a non-toxic, inert, solid, semi-solid material or liquid filler, diluent, encapsulant or adjuvant or excipient of any type, which is compatible with a patient, preferably a mammal, more preferably a human, and is suitable for delivery of the active reagent to a target without terminating the activity of the reagent.

"Active material of the present invention" or "active compound of the present invention" refers to a compound of formula (I) of the present invention, or a pharmaceutically acceptable salt thereof, or a stereoisomer thereof, which has a high SHP2 enzyme inhibitory activity.

The compositions of the present invention are formulated, dosed and administered in a manner consistent with medical practice guideline. The "therapeutically effective amount" of the compound to be administered is determined by the specific condition to be treated, the individual to be treated, the cause of the condition, the target of the drug and the route of administration.

"Therapeutically effective amount" refers to the amount of the compound of the present invention that will cause a biological or medical response in an individual, such as decreasing or inhibiting enzyme or protein activity or ameliorating a symptom, alleviating a condition, slowing or delaying a disease process or preventing a disease, etc.

The therapeutically effective amount of the compound of the present invention, or a pharmaceutically acceptable salt thereof, or a stereoisomer thereof, contained in the pharmaceutical composition or medicinal composition of the present invention is preferably 0.1 mg/kg to 5 g/kg (body weight).

The term "patient" refers to an animal, preferably a mammal, and more preferably a human. The term "mammal" refers to a warm-blooded vertebrate mammal, including, for example, cats, dogs, rabbits, bears, foxes, wolves, monkeys, deer, mice, pigs and humans.

"Treatment" refers to alleviation, slowing of progression, attenuation, prevention, or maintenance of an existing disease or condition (e.g., cancer). Treatment also includes curing, preventing the progression of, or reducing to a certain level one or more symptoms of a disease or condition.

The "pharmaceutically acceptable salt" includes a pharmaceutically acceptable acid addition salt and a pharmaceutically acceptable base addition salt. Pharmaceutically acceptable acid addition salts refer to salts formed with inorganic or organic acids, which are capable of retaining the biological effectiveness of the free base without other side effects. These salts can be prepared by methods known in the art.

"Pharmaceutically acceptable base addition salts", include salts of inorganic bases and salts of organic bases, which can be prepared by methods known in the art.

When the compound represented by formula (I) of the present invention contains one or more chiral centers, the compound can exist in different optically active forms. When the compound of formula (I) contains one chiral center, the compound contains a pair of enantiomers. The two enantiomers of the compound and mixtures of the pair of enantiomers, such as racemic mixtures, are also within the scope of protection of the present invention. The enantiomers can be resoluted by methods known in the art, such as crystallization as well as methods such as chiral chromatography. When a compound of formula (I) contains more than one chiral center, the compound contains both enantiomers and diastereomers. All enantiomers and diastereomers of the compound, as well as mixtures of enantiomers, mixtures of diastereomers, and mixtures of enantiomers and diastereomers are also within the scope of protection of the present invention. The enantiomers and diastereomers can be resoluted by methods known in the art, such as crystallization as well as preparative chromatography. Unless otherwise indicated, the absolute configuration of a stereocenter is indicated by a wedge bond 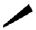 or 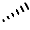 , and one of the absolute configurations of a stereocenter, e.g., one of 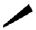 or 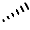 , is indicated by a wavy line 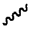 . When the compound described herein contain alkenyl double bonds or other geometrically asymmetric centers, E and Z geometrical isomers are included unless otherwise specified. Similarly, all tautomer forms are included within the scope of this application.

### Preparation method

The present invention provides preparation methods of the compound of formula (I), which can be synthesized using standard synthetic techniques known to those skilled in the art or using a combination of methods known in the art with the methods described in the present invention. The solvents, temperatures and other reaction conditions given in the present invention can be varied according to techniques in the art. The reactions described can be used sequentially to provide the compounds of the present invention, or can be used to synthesize fragments that are subsequently incorporated by the methods described herein and/or methods known in the art.

The compounds described herein may be synthesized using methods similar to those described below or exemplary methods described in the examples, or relevant disclosures used by those skilled in the art, by using appropriate optional starting materials. The starting materials used to synthesize the compounds described herein may be synthesized or may be obtained from commercial sources. The compounds described herein and other related compounds having different substituents can be synthesized using techniques and raw materials known to those skilled in the art. General methods for preparing the compounds disclosed by the present invention may be derived from reactions known in the art and the reactions may be modified in terms of reagents and conditions considered appropriate by those skilled in the art to introduce various moieties of the molecules provided by the present invention.

The main advantages of the present invention over the prior art include:
A series of structurally novel heterocycle-substituted methanone derivatives are provided, which have high inhibitory activity against SHP2 enzymes, with an IC₅₀ value of less than 100 nM, preferably less than 50 nM, and more preferably less than 10 nM. The compounds of the present invention exhibit significant tumor growth inhibition and have good antitumor efficacy, and thus can be used as medicaments for the treatment and/or prevention of SHP2-mediated diseases or diseases associated with aberrant SHP2 activity. In addition, the compounds of the present invention do not inhibit hERG potassium current, indicating a good safety profile for the cardiovascular system.

The present invention is further described below in conjunction with specific examples. It should be understood that these examples are used only to illustrate the invention and are not intended to limit the scope of the invention. Experimental methods for which specific conditions are not indicated in the following examples are generally in accordance with conventional conditions such as those described in Sambrook et al., Molecular Cloning: A Laboratory Manual (New York: Cold Spring Harbor Laboratory Press, 1989), or in accordance with conditions recommended by the manufacturer. Percentages and parts are by weight unless otherwise indicated. Unless otherwise defined, terms used herein have the same meaning as those familiar to those skilled in the art. In addition, any methods and materials similar or equivalent to what is recorded herein may be applied in the present invention.

Known starting materials can be synthesized using or according to methods known in the art or can be purchased from companies such as ABCR GmbH&Co.KG, Acros Organics, Aldrich Chemical Company, Accela ChemBio Inc and Darui Chemicals.

DCM: dichloromethane, ACN: acetonitril, DMF: dimethylformamide, DMSO: dimethyl sulfoxide, THF: tetrahydrofuran, DIEA: N,N-diisopropylethylamine, EA: ethyl acetate, PE: petroleum ether, BINAP: (2R,3S)-2,2'-bis(diphenylphosphino)-1,1'-binaphthalene, NBS: N-bromosuccinimide, NCS: N-chlorosuccinimide, CDI: N,N'-carbonyldiimidazole, Pd₂(dba)₃: tris(dibenzylideneacetone)-dipalladium, Pd(dppf)Cl₂: [1,1'-bis(diphenylphosphino)ferrocene]dichloropalladium, DPPA: diphenylphosphoryl azide, DBU: 1,8-diazabicycloundec-7-ene, TBAF: tetrabutylammonium fluoride, Na Ascorbate: sodium ascorbate, t-BuXPhos-Pd-G3: methanesulfonato(2-di-tert-butylphosphino-2',4',6'-triisopropyl-1,1'-biphenyl)(2'-amino-1,1'-biphenyl-2-yl)palladium(II), P(t-Bu)3 Pd G4: methanesulfonato(tri-t-butylphosphino)(2-methylamino-1,1 '-biphenyl-2-yl)palladium(II), DIBAL-H: diisobutylaluminum hydride, Ti(OEt)₄: ethyl titanate, LDA: lithium diisopropylamide, TBAB: tetrabutylammonium bromide, DIPEA: N,N-diisopropylethylamine, HATU: O-(7-azabenzotriazol-1-yl)-N,N,N',N'-tetramethyluronium hexafluorophosphate, EDCI: 1-(3-dimethylaminopropyl)-3-ethylcarbodiimide hydrochloride, BPO: dibenzoyl peroxide, TEA: triethanolamine, MO(CO)₆: molybdenum hexacarbonyl, TCFH: N,N,N',N'-tetramethylchloroformamidinium hexafluorophosphate, NIS: N-iodosuccinimide, m-CPBA: m-chloroperbenzoic acid, NMP: N-methylpyrrolidinone, Selectfluor: 1-chloromethyl-4-fluoro-1,4-diazobicyclo[2.2.2]octane bis(tetrafluoroborate), PyBOP: benzotriazol-1-yl-oxytripyrrolidinophosphonium hexafluorophosphate, TFA: trifluoroacetic acid, and T₃P: propylphosphonic anhydride. As used herein, and room temperature means about 20-30 °C.

In the following preparative examples 1-3, 6-9, 11 and 14-24, wavy line 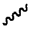 in the compounds represents the 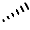 configuration.

### Preparative Example 1: Preparation of Compound 24g

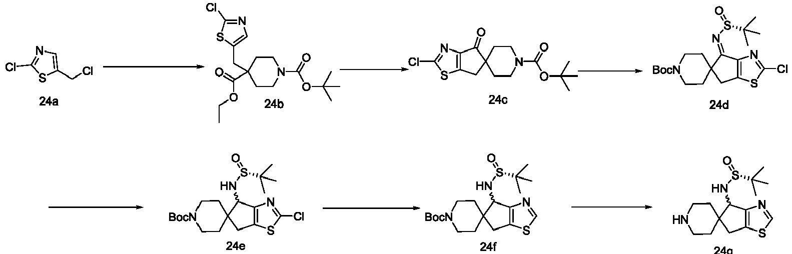

Step 1: After dissolving 1-(tert-butyl)4-ethylpiperidin-1,4-dicarboxylate (8.32 g, 34.22 mmol) in THF (100 mL), under nitrogen protection, the reaction system was cooled down to -78°C. Then LDA (2 M, 16.96 mL) was slowly added dropwise. The reaction was carried out at -78°C for 1 hour with stirring. Compound 24a (5 g, 29.75 mmol) dissolved in THF (10 mL) was added dropwise to the above reaction system, and the reaction was carried out at -78°C for 3 hours. After the reaction was completed, the reaction was quenched with 80 mL of saturated sodium chloride solution, and ethyl acetate was added. The organic phase was separated, and the aqueous phase was extracted with ethyl acetate. The organic phases were combined, dried with anhydrous sodium sulfate, then concentrated by spin-drying, and separated by column (petroleum ether : ethyl acetate=100:1 to 1:2) to give Compound 24b (6.1 g, 52.7% yield). MS m/z (ESI): 333.0 [M-56+H]⁺.

Step 2: Compound 24b (1 g, 2.57 mmol) was dissolved in THF (20 mL), and then the reaction was cooled down to -78°C. Then LDA (2 M, 3.34 mL) was added dropwise and the reaction was carried out at -78°C for 0.5 hour. After the reaction was completed, the reaction was quenched with saturated sodium chloride solution, and ethyl acetate was added. The organic phase was separated, and the aqueous phase was extracted with ethyl acetate. The organic phases were combined, dried with anhydrous sodium sulfate, then concentrated by spin-drying, and separated by column (0% to 30% petroleum ether/ethyl acetate) to give Compound 24c (280 mg, 31.7% yield). MS m/z (ESI): 287.0 [M-56+H]⁺.

Step 3: Compound 24c (1.2 g, 3.50 mmol) was dissolved in Ti(OEt)₄ (10 mL). After three argon replacements, the system was heated to a temperature of 120°C under argon protection, and reacted for 2 hours. After the reaction was cooled to room temperature, the resultant was diluted by adding 100 mL of ethyl acetate. The diluted reaction system was added to 300 mL of water with stirring, and a large amount of insoluble solid precipitated, which was filtered through diatomaceous earth. The filter cake was washed with ethyl acetate while the filtrate was extracted with ethyl acetate. The combined organic phases were dried with anhydrous sodium sulfate, concentrated, and separated by column (petroleum ether : ethyl acetate=100:1 to 1:1) to give Compound 24d (615 mg, 39.4% yield). MS m/z (ESI): 346.0[M-100+H]⁺.

Step 4: Compound 24d (551 mg, 1.24 mmol) was dissolved in THF (15 mL) and then the reaction was cooled down to -78°C. DIBAL-H (1 M, 2.47 mL) was added dropwise and the reaction was carried out at -78°C for 0.5 hour. After the reaction was completed, sodium sulfate decahydrate was added and the resultant was stirred for 20 min at low temperature and then filtered through diatomaceous earth. The filtrate was spin-dried to give Compound 24e (560 mg), MS m/z (ESI): 348.1 [M-100+H]⁺.

Step 5: Compound 24e (120 mg, 267.83 µmol) was dissolved in MeOH (15 mL) followed by the addition of Pd/C (100 mg, 10% purity) and triethylamine (0.1 mL), and hydrogen replacements were performed for 4 times. The system was heated to a temperature of 60°C under an oil bath and reacted for 12 hours. After the reaction was completed, the resultant was diluted with 20 mL of methanol, and then filtered through diatomaceous earth. The filtrate was concentrated by spin-drying to give Compound 24f (85 mg). MS m/z (ESI): 314.1[M-100+H]⁺.

Step 6: Compound 24f (68 mg, 164.7 µmol) was dissolved in DCM (6 mL), then TFA (1 mL) was added and the reaction was carried out at room temperature 20°C for 2 hours. After spin-drying, Compound 24 g (75 mg) was obtained. MS m/z (ESI): 314.1[M+1]⁺.

### Preparative Example 2: Preparation of Compound 25a

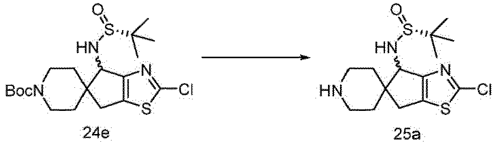

Compound 24e (70 mg, 152.1 µmol) was dissolved in DCM (6 mL), followed by the addition of TFA (1 mL), and the reaction was carried out at room temperature 20°C for 2 hours. After the reaction was completed, the reaction system was directly concentrated by spin-drying to give Compound 25a (90 mg). MS m/z (ESI): 348.1 [M+1]⁺.

### Preparative Example 3: Preparation of Compound 26h

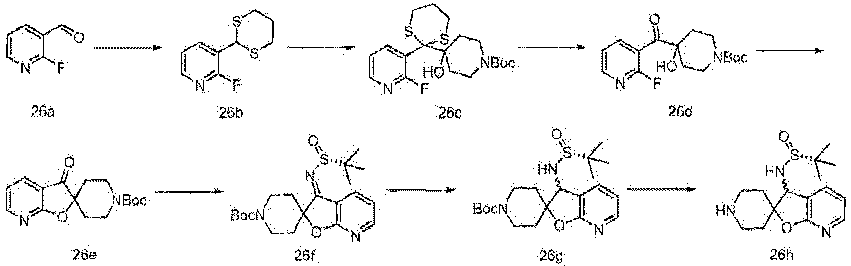

Step 1: 2-Fluoropyridin-3-carboxaldehyde 26a (10 g, 79.94 mmol) and ethane-1,2-dithiol (8.28 g, 87.93 mmol) were dissolved in DCM (150 mL), to which BF₃.Et₂O (3.52 g, 24.78 mmol) was then added. The reaction was carried out at room temperature for 1.0 hour with stirring. After the reaction was completed, the reaction was quenched with 30 mL of aqueous solution, and the reaction system was extracted 3 times with 60 mL of ethyl acetate. The organic phase was washed with 30 mL of saturated saline and dried with anhydrous sodium sulfate, and the solvent was spin-dried under reduced pressure. The sample was mixed with silica gel and purified by silica gel column chromatography (petroleum ether : ethyl acetate=1:1) to give Compound 26b (12 g, 63.9% yield). MS m/z (ESI): 216.0 [M+H]⁺.

Step 2: Compound 26b (10 g, 51.09 mmol) was dissolved in THF (200 mL), and the solution was cooled to -78°C, to which LDA (2M) (10.95 g, 102.18 mmol) was added dropwise. The reaction was carried out at -78°C for 1.0 hour with stirring, then tert-butyl 4-oxopiperidin-1-carboxylate (20.36 g, 102.18 mmol) was added, and the reaction was continued at -78°C for 1.0 hour. After the reaction was completed, the reaction was quenched with 30 mL of aqueous solution, and the reaction system was extracted 3 times with 60 mL of ethyl acetate. The organic phase was washed one time with 30 mL of saturated saline and dried with anhydrous sodium sulfate, and the solvent was spin-dried under reduced pressure. The sample was mixed with silica gel and purified by silica gel column chromatography (petroleum ether : ethyl acetate=4:1) to obtain Compound 26c (10g, 47.2% yield). MS m/z (ESI): 315.1[M-100+H]⁺.

Step 3: Compound 26c (10 g, 24.12 mmol), tribromopyridine (15.43 g, 48.24 mmol), pyridine (2.86 g, 36.18 mmol, 2.91 mL), and TBAB (2.33 g, 7.24 mmol) were dissolved in DCM (30 mL) and H₂O (5 mL). The reaction was carried out at room temperature for 2.0 hours with stirring. After the reaction was completed, the reaction was quenched with 30 mL of aqueous solution, and the reaction system was extracted 3 times with 60 mL of ethyl acetate. The organic phase was washed with 30 mL of saturated saline and dried with anhydrous sodium sulfate, and the solvent was spin-dried under reduced pressure. The sample was mixed with silica gel and purified by silica gel column chromatography (petroleum ether : ethyl acetate=3:1 ) to give Compound 26d (6.1 g, 78.0% yield). MS m/z (ESI): 225.0 [M-100+H]⁺.

Step 4: Compound 26d (6.1 g, 18.83 mmol) was dissolved in dioxane (50 mL) and potassium tert-butoxide (4.21 g, 37.61 mmol) was added to it. The reaction was carried out at 25°C for 2.0 hours with stirring. After the reaction was completed, the reaction was quenched with 30 mL of aqueous solution, and the reaction system was extracted 3 times with 60 mL of ethyl acetate. The organic phase was washed with 30 mL of saturated saline and dried with anhydrous sodium sulfate, and the solvent was spin-dried under reduced pressure. The sample was mixed with silica gel and purified by silica gel column chromatography (petroleum ether : ethyl acetate=1:1) to give Compound 26e (3.5 g, 61.2% yield). MS m/z (ESI): 249.0 [M+1-56]⁺.

Step 5: Compound 26e (4.8 g, 15.77 mmol) and R-(+)-tert-butylsulfinamide (1.91 g, 15.77 mmol) were dissolved in Ti(EtO)₄ (50 mL). The reaction was carried out at 90°C for 2.0 hours with stirring. After the reaction was completed, the reaction was quenched with 30 mL of aqueous solution, and the reaction system was extracted 3 times with 60 mL of ethyl acetate. The organic phase was washed one time with 30 mL of saturated saline and dried with anhydrous sodium sulfate, and the solvent was spin-dried under reduced pressure. The sample was mixed with silica gel and purified by silica gel column chromatography (petroleum ether : ethyl acetate=10:1) to give Compound 26f (4.6 g, 71.6% yield). MS m/z (ESI): 408.0 [M+1]⁺.

Step 6: Compound 26f (4.6 g, 11.29 mmol) was dissolved in THF (50 mL), to which then a hexane solution of DIBAL-H (11 mL) was added to at -78°C. The reaction was stirred at -78°C for 2.0 hours. After the reaction was completed, the reaction was quenched with 30 mL of aqueous solution, extracted 3 times with 60 mL of ethyl acetate, the combined organic phase was washed with 30 mL of saturated saline, dried with anhydrous sodium sulfate, and the solvent was spin dried under reduced pressure. The sample was mixed with silica gel and purified by silica gel column chromatography (petroleum ether :ethyl acetate=1:1) to give Compound 26g (3.5g, 75.7% yield). MS m/z (ESI): 354.0 [M+H]⁺.

Step 7: Compound 26g (100 mg, 244.18 µmol) was dissolved in TFA (1.00 mL) and DCM (5.00 mL). The reaction was carried out at 25°C for 1.0 hour with stirring. After completion of the reaction, the solvent was directly spin-dried to give Compound 26h (60 mg, 79.4% yield). MS m/z (ESI): 310.0 [M+H]⁺.

### Preparative Example 4: Preparation of Compound 32c

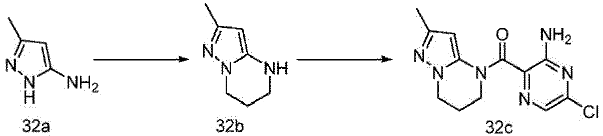

Step 1: At 0°C, 1,3-dibromopropane (1.25 g, 6.18 mmol) was added to a solution of compound 32a (500 mg, 5.15 mmol) and triethylamine (2.60 g, 25.74 mmol, 3.59 mL) in dioxane (10.30 mL), and the reaction system was warmed up to 100°C and stirred for 4 hours. The reaction system was concentrated, separated and purified by column chromatography (DCM : MeOH=15:1) to give Compound 32b (350 mg, 49% yield). MS m/z (ESI): 138.1 [M+1]⁺.

Step 2: A solution of DIPEA (190 mg, 1.47 mmol) and HATU (221.72 mg, 587.70 µmol) in DMF (2 mL) was added to Compound 32b (85 mg, 489.75 µmol) and 3-amino-5-chloro-pyrazin-2-carboxylic acid (873.90 mg, 538.73 µmol), and the reaction system was stirred for 2 hours at room temperature. The reaction system was concentrated and separated by column chromatography (dichloromethane: methanol=15:1) to give Compound 32c (25.96 mg, 41% yield). MS m/z (ESI): 293.0 [M+1]⁺.

### Preparative Example 5: Preparation of Compound 33d

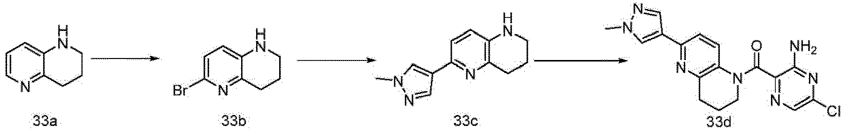

Step 1: NBS (1.27 g, 7.15 mmol) was added to a reaction system of compound 33a (950 mg, 7.08 mmol) in acetonitrile (10 mL), and the mixture was stirred at room temperature for 2 hours. The reaction system was concentrated, separated and purified by column chromatography (PE : EA=2:1) to give Compound 33b (1.20 g, 79% yield). MS m/z (ESI): 215.0 [M+1]⁺.

Step 2: A solution of K₂CO₃ (416.43 mg, 3.01 mmol) and Pd(dppf)Cl₂ (73.5 mg, 100.43 µmol) in dioxane (5 mL) and water (1.5 mL) was added to Compound 33b (214 mg, 1.00 mmol) and 1-methyl-4-(4,4,5,5-tetramethyl-1,3,2-dioxaborolan-2-yl)-1H-pyrazole (250 mg, 1.21 mmol), the resultant was stirred at 100°C under nitrogen protection for 5 hours. The reaction system was concentrated, separated and purified by column chromatography (PE: EA=1:1) to give Compound 33c (163 mg, 75% yield). MS m/z (ESI): 215.1 [M+1]⁺.

Step 3: The reaction system of compound 33c (83 mg, 387.37 µmol), 3-amino-5-chloropyrazin-2-carboxylic acid (67 mg, 387.37 µmol) and EDCI (148 mg, 774.74 µmol) in pyridine (5 mL) was stirred at room temperature for 5 hours. The reaction system was concentrated, separated and purified by column chromatography (DCM : MeOH=15:1) to give Compound 33d (115 mg, 80% yield). MS m/z (ESI): 370.1 [M+1]⁺.

### Preparative Example 6: Preparation of Compound 34a

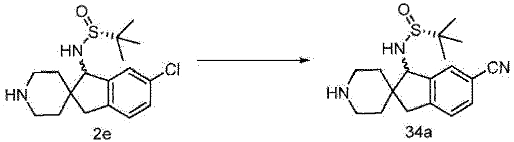

Step 1: Compound 2e (80 mg, 234.67 µmol) was dissolved in a solvent mixture of THF (1 mL) and H₂O (1 mL), to which Zn(CN)₂ (41.33 mg, 352.00 µmol) and t-BuXphos-Pd-G3 (18.63 mg, 23.47 µmol) was then added. The reaction was carried out at 100°C with stirring for 16 hours. When the reaction was finished, the solvent was evaporated under reduced pressure, dichloromethane was added, the resultant was mixed with silica gel, and purified by silica gel column chromatography to give Compound 34a (33 mg, 38.6% yield). MS m/z (ESI): 332.2 [M+1]⁺.

### Preparative Example 7: Preparation of Compound 35c

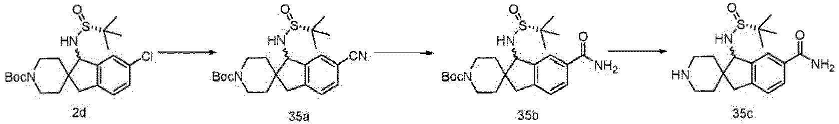

Step 1: Under argon protection, Compound 2d (500 mg, 1.13 mmol) was dissolved in a solvent mixture of THF (10 mL) and H₂O (10 mL), to which Zn(CN)₂ (266.24 mg, 2.27 mmol) and t-BuXphos-Pd-G3 (90.02 mg, 113.37 µmol) were then added. The reaction was carried out at 100°C for 16 hours with stirring. After the reaction was completed, the solvent was evaporated under reduced pressure, dichloromethane was added, and the sample was mixed with silica gel and purified by silica gel column chromatography (petroleum ether : ethyl acetate=2:3). After purification, Compound 35a (403 mg, 82.4% yield) was obtained. MS m/z (ESI): 332.1[M-100+H]⁺.

Step 2: Compound 35a (200 mg, 463.40 µmol) was dissolved in CH₃OH (5 mL), to which H₂O₂ (30%, 1.5 mL) and NH₄OH (28%, 2 mL) were added. The reaction was carried out at 20°C for 1 hour with stirring. When the reaction was completed, it was quenched by adding saturated sodium bicarbonate and saturated sodium thiosulfate solution, then the reaction system was extracted by adding ethyl acetate, the phases were separated, and the organic phase was collected and spin-dried. Dichloromethane was added, and the sample was mixed with silica gel and purified by silica gel column chromatography (petroleum ether : ethyl acetate=1:4 to ethyl acetate (100%) to dichloromethane: methanol=10:1). After purification, Compound 35b (95 mg, 45.6% yield) was obtained. MS m/z (ESI): 350.2 [M-100+H]⁺.

Step 3: Compound 35b (90 mg, 200.18 µmol) was dissolved in DCM (5 mL), to which TFA (0.7 mL) was then added. The reaction was carried out at 20°C for 0.5 hour with stirring. When the reaction was finished, the solvent was evaporated under reduced pressure to give Compound 35c (73 mg). MS m/z (ESI): 350.1 [M+1]⁺.

### Preparative Example 8: Preparation of Compound 38h

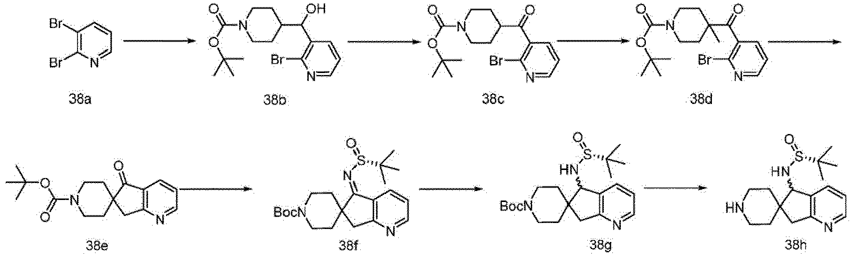

Step 1: Isopropylmagnesium chloride (2.8 M, 24.9 mL, 69.65 mmol) was added dropwise to a solution of 2,3-dibromopyridine (15.0 g, 63.32 mmol) in THF (200 mL), the resultant was stirred for 1 hour at room temperature, then a solution of 1-tert-butoxycarbonylpiperidin-4-carbaldehyde (14.85 g, 69.65 mmol) in THF (50 mL) was added dropwise to the reaction system and it was stirred for 1 hour. The reaction was quenched with saturated ammonium chloride solution (5 mL), and the reaction system was concentrated, and separated by column chromatography (petroleum ether : ethyl acetate=4:1) to give Compound 38b (23.5 g, 99% yield). MS m/z (ESI): 317.1 [M-56+H]⁺.

Step 2: Dess-Martin oxidizer (29.53 g, 69.63 mmol) was added batchwise to a solution of Compound 38b (23.5 g, 63.30 mmol) in dichloromethane (300 mL), and the resultant was stirred at room temperature for 1 hour. The reaction system was concentrated and separated by column chromatography (petroleum ether : ethyl acetate=3:1) to give Compound 38c (22.1 g, 94% yield). MS m/z (ESI): 313.0 [M-56+H]⁺.

Step 3: At -30°C, lithium hexamethyldisilanamide (38 mL, 38.02 mmol) was added dropwise to a solution of Compound 38c (11.7 g, 31.69 mmol) in THF (120 mL), the resultant was stirred at -30°C for 30 min, then iodomethane (6.75 g, 47.53 mmol) was added dropwise, and the reaction system was slowly warmed up to room temperature and stirred for 3 hours. The reaction was quenched with aqueous ammonium chloride (10 mL), and the reaction system was concentrated, and separated by column chromatography (dichloromethane : methanol=30:1) to give Compound 38d (10.3 g, 84% yield). MS m/z (ESI): 329.0 [M-56+H]⁺.

Step 4: A reaction system of Compound 38d (10.3 g, 26.87 mmol), tricyclohexylphosphine tetrafluoroborate (1.14 g, 2.69 mmol), cesium carbonate (8.76 g, 26.87 mmol), pivalic acid (823.37 mg, 8.06 mmol), and palladium(III) acetate (301.67 mg, 1.34 mmol) in 1,3,5-mesitylene (80 mL) was stirred at 140°C for 10 hours. The reaction system was diluted with ethyl acetate (50 mL) and filtered through a diatomaceous earth layer, and the filtrate was concentrated and separated by column chromatography (petroleum ether : ethyl acetate=3:1) to give Compound 38e (5.8 g, 71% yield). MS m/z (ESI): 303.1 [M+1]⁺.

Step 5: (R)-(+)-tert-butylsulfinamide (4.88 g, 40.28 mmol) was added to a solution of Compound 38e (5.8 g, 19.18 mmol) in Ti(OEt)₄ (50 mL), and the reaction system was stirred at 100°C for 3 hours. The reaction system was diluted with ethyl acetate (50 mL), and the reaction was quenched with water. The reaction system was filtered through a diatomaceous earth layer, and the filtrate was concentrated , separated by column chromatography (dichloromethane : methanol=20:1) to give Compound 38f (7.6 g, 97% yield). MS m/z (ESI): 406.2[M+1]⁺.

Step 6: At -78°C, DIBAL-H (40.14 mmol, 40.14 mL) was added dropwise to a solution of Compound 38f (7.4 g, 18.25 mmol) in THF (70 mL), the reaction system was stirred at -78°C for 4 hours, and the temperature was slowly raised to 0°C. The reaction was quenched with water (1 mL), and the reaction system was concentrated, and separated by column chromatography (petroleum ether : ethyl acetate=1:1) to give Compound 38g (5.8 g, 78% yield). MS m/z (ESI): 408.3 [M+1]⁺.

Step 7: At room temperature, trifluoroacetic acid (5 mL) was added to a solution of Compound 38g (1.5 g, 3.68 mmol) in dichloromethane (15 mL), and the reaction system was stirred at room temperature for 2 hours. The reaction system was concentrated, and separated by column chromatography (dichloromethane : methanol=10:1) to give Compound 38h (1.05 g, 92% yield). MS m/z (ESI ): 308.2 [M+1]⁺.

### Preparative Example 9: Preparation of Compound 48g

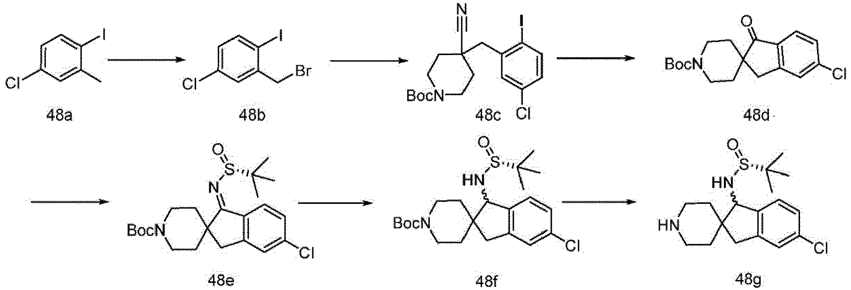

Step 1: BPO (242 mg, 1 mmol) was added to a solution of Compound 48a (2.51 g, 10 mmol) and NBS (1.77 g, 10 mmol) in dichloroethane (35 mL), and the reaction system was stirred under N₂ at 80°C for 12 hours and then concentrated. The resultant was separated and purified by column chromatography (0-16% ethyl acetate/petroleum ether) to give Compound 48b (2.8 g, 84.8% yield).

Step 2: LDA (6.4 ML, 12.7 mmol) was added dropwise to a solution of tert-butyl 4-cyanopiperidin-1-carboxylate (1.77 g, 8.45 mmol) in THF (40 mL) at -78°C under N₂, and the reaction system was stirred for 1 hour. A solution of Compound 48b (2.8 g, 8.45 mmol) in THF (10 mL) was slowly added, and the reaction system was stirred for 1 hour. 100 mL of saturated aqueous ammonium chloride solution was added to quench the reaction, and the resultant was extracted with ethyl acetate (40 mL*3). The organic phases were combined, washed with saline (50 mL*2), dried with anhydrous sodium sulfate, concentrated, separated and purified by column chromatography (petroleum ether/ethyl acetate=3:1) to give Compound 48c (2.6 g, 68.4% yield). MS m/z (ESI): 361.0 [M-100+H]⁺.

Step 3: Compound 48c (2.6 g, 5.65 mmol) was dissolved in 15 mL of DMF and 1.5 mL of water, then P(t-Bu)3 Pd G4 (364 mg, 0.56 mmol) and triethylamine (1.2 g, 11.3 mmol) were added. Nitrogen replacement was performed three times, and the mixture was heated to 130°C. The reaction system was stirred for 12 hours, then cooled to room temperature, and concentrated to dryness under reduced pressure, and the residue was purified by column chromatography (eluent: petroleum ether/ethyl acetate=1:2) to give Compound 48d (1.3 g, 68.8% yield). MS m/z (ESI): 280.2 [M-56+H]⁺.

Step 4: R-(+)-tert-butylsulfinamide (1.89 g, 15.5 mmol) was added to a solution of Compound 48d (1.3 g, 3.88 mmol) in Ti(EtO)₄ (50 mL), and the mixture was stirred at 90°C for 20 hours. To the reaction system, ethyl acetate (50 mL) and 200 mL of saline were added and the resultant was stirred for 5 min and filtrated through diatomaceous earth. The filtrate was layered, and the organic layer was washed with saline (50 mL*2), dried with anhydrous sodium sulfate, and concentrated. It was separated and purified by column chromatography (0-35% ethyl acetate/petroleum ether) to give Compound 48e (0.93 g, 58.6% yield). MS m/z (ESI): 339.1 [M-100+H]⁺.

Step 5: At -30°C, NaBH₄ (121 mg, 3.18 mmol) was added batchwise to a solution of Compound 48e (0.93 g, 2.12 mmol) in THF (15 mL), then the reaction system was stirred at room temperature for 12 hours. To the reaction system, ethyl acetate (30 mL) and 300 mL of saline were added and the resultant was stirred for 5 min. The filtrate was layered, and the organic layer was washed with saline (30 mL*2), dried with anhydrous sodium sulfate, and concentrated to give Compound 48f (0.94 g, 100% yield). MS m/z (ESI): 341.1 [M-100+H]⁺.

Step 6: TFA (2 mL) was added to a solution of Compound 48f (0.94 g, 2.13 mmol) in dichloromethane (10 mL) and the mixture was stirred for 2 hours. The reaction system was separated and purified by column chromatography (DCM : MeOH=7:1) to give Compound 48g (0.65 g, 87.7% yield). MS m/z (ESI): 341.1 [M+1]⁺.

### Preparative Example 10: Preparation of Compound 54e

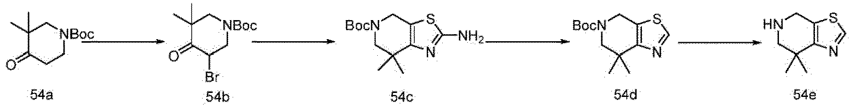

Step 1: Compound 54a (300 mg, 1.32 mmol) was dissolved in CHCl₃ (5 mL), and the solution was cooled to 0°C, to which Br₂ (1.05 g, 6.60 mmol) was added dropwise. The reaction system was stirred at 25°C for 1.0 hour to give Compound 54b (50 mg), which was used directly in the next step.

Step 2: Compound 54b (50 mg, 163.29 µmol) and thiourea (37.29 mg, 489.88 µmol) were dissolved in EtOH (5 mL). The reaction systemwas stirred at 80°C for 3 hours. After the reaction was completed, it was quenched with 40 mL of aqueous solution, and 60 mL of ethyl acetate was added. The reaction system was filtered through diatomaceous earth, and the resultant was extracted 3 times with 50 mL of ethyl acetate. The organic phase was washed with 40 mL of saturated saline and dried with anhydrous sodium sulfate, and the solvent was spin-dried under reduced pressure. The sample was mixed with silica gel and purified by silica gel column chromatography (petroleum ether : ethyl acetate=4:1) to give Compound 54c (14 mg, 30.2% yield). MS m/z (ESI): 284.0[M+1]⁺.

Step 3: Compound 54c (14 mg, 49.40 µmol) and tert-butyl nitrite (25.47 mg, 247.01 µmol) were dissolved in THF (3 mL). The reaction system was stirred at 80°C for 3 hours. The sample was mixed with silica gel and purified by silica gel column chromatography (petroleum ether : ethyl acetate=2:1) to give Compound 54d (6 mg, 95.7% yield). MS m/z (ESI): 269.0 [M+1]⁺.

Step 4: Compound 54d (10 mg, 37.26 µmol) was dissolved in MeOH (5 mL) to which HCl in MeOH (1 mL) was added. The reaction system was stirred at 25°C for 1.0 hour. After the reaction was completed, it was quenched with 30 mL of aqueous solution, and reaction system was extracted 3 times with 60 mL of ethyl acetate. The organic phase was washed with 30 mL of saturated saline and dried with anhydrous sodium sulfate, and the solvent was spin-dried under reduced pressure. The sample was mixed with silica gel and purified by silica gel column chromatography (dichloromethane : methanol=10:1) to give Compound 54e (45 mg, 49.8% yield). MS m/z (ESI):169.0 [M+1]⁺.

### Preparative Example 11: Preparation of Compound 56h

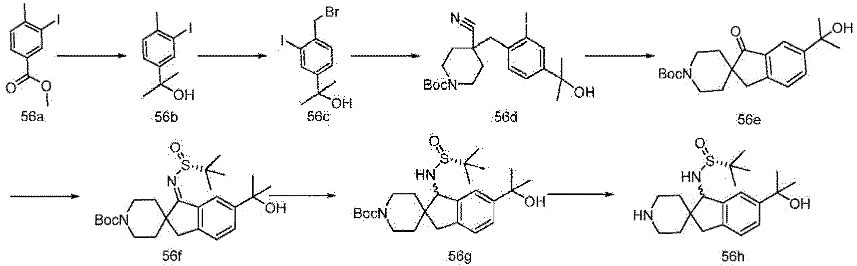

Step 1: A solution of 2N methylmagnesium bromide in THF (30 mL, 60 mmol) was added to a solution of Compound 56a (2.76 g, 10 mmol) in THF (40 mL), and the reaction system was stirred at room temperature for 12 hours. The reaction was quenched by adding 100 mL of saturated aqueous ammonium chloride, and the resultant was extracted with ethyl acetate (40 mL*3). The organic phases were combined, washed with saline (100 mL*2 ), dried with anhydrous sodium sulfate, concentrated, separated and purified by column chromatographic (petroleum ether/ethyl acetate 3:1) to give Compound 56b (2.6 g, 94.2% yield).

Step 2: BPO (230 mg, 0.95 mmol) was added to a solution of Compound 56b (2.6 g, 9.42 mmol) and NBS (1.67 g, 9.42 mmol) in DCE (35 mL), and the reaction system was stirred at 80°C under N₂ for 12 hours. The reaction system was concentrated, separated and purified by column chromatography (0-25% ethyl acetate/petroleum ether) to give Compound 56c (2.8 g, 84.1% yield).

Step 3: LDA (6.0 ML, 11.9 mmol) was added dropwise to a solution of tert-butyl 4-cyanopiperidin-1-carboxylate (1.66 g, 7.9 mmol) in THF (40 mL) at -78°C under N₂, and the reaction system was stirred for 1 hour. A solution of Compound 56c (2.8 g, 7.9 mmol) in THF (10 mL)was added slowly, and the reaction system was stirred for 1 hour. 100 mL of saturated aqueous ammonium chloride solution was added to quench the reaction, and the resultant was extracted with ethyl acetate (40 mL*3). The organic phases were combined, washed with saline (50 mL*2), dried with anhydrous sodium sulfate, concentrated, separated and purified by column chromatography (petroleum ether/ethyl acetate=3:1). Compound 56d (2.5 g, 65.7% yield) was obtained, MS m/z (ESI): 385.0 [M-100+H]⁺.

Step 4: Compound 56d (2.5 g, 5.15 mmol) was dissolved in 15 mL of DMF and 1.5 mL of water, to which P(t-Bu)3 Pd G4 (326 mg, 0.51 mmol) and triethylamine (1.2 g, 11.3 mmol) were added, and nitrogen replacement was performed three times. The reaction system was heated to 130°C, stirred for 12 hours, then cooled to room temperature, and concentrated to dryness under reduced pressure, and the residue was purified by column chromatography (eluent: petroleum ether/ethyl acetate=37%) to give Compound 56e (1.1 g, 59.4% yield). MS m/z (ESI): 304.2 [M-56+H]⁺.

Step 5: R-(+)-tert-butylsulfinamide (1.48 g, 12.3 mmol) was added to a solution of Compound 56e (1.1 g, 3.06 mmol) in Ti(EtO)₄ (50 mL), and the mixture was stirred at 90°C for 20 hours. To the reaction system, ethyl acetate (50 mL) and 200 mL of saline were added and the resultant was stirred for 5 min and filtrated through diatomaceous earth. The filtrate was layered, and the organic layer was washed with saline (50 mL*2), dried with anhydrous sodium sulfate, and concentrated. The resultant was separated and purified by column chromatography (0-45% ethyl acetate/petroleum ether) to give Compound 56f (0.81 g, 57.4% yield). MS m/z (ESI): 363.1 [M-100+H]⁺.

Step 6: At -30°C, NaBH₄ (100 mg, 2.62 mmol) was added batchwise to a solution of Compound 56f (0.81 g, 1.75 mmol) in THF (15 mL), and then the reaction system was stirred at room temperature for 12 hours. To the reaction system, ethyl acetate (30 mL) and 300 mL of saline were added and the resultant was stirred for 5 min. The filtrate was layered, and the organic layer was washed with saline (30 mL*2), dried with anhydrous sodium sulfate, and concentrated to give Compound 56g (0.56 g, 69.1% yield). MS m/z (ESI): 365.1 [M-100+H]⁺.

Step 7: TFA (2 mL) was added to a solution of Compound 56g (0.56g, 1.2 mmol) in dichloromethane (10 mL) and the mixture was stirred for 2 hours. The reaction system was separated and purified by column chromatography (DCM : MeOH=6:1) to give Compound 56h (0.11g, 25.2% yield). MS m/z (ESI): 365.1 [M+1]⁺.

### Preparative Example 12: Preparation of Compound 57f

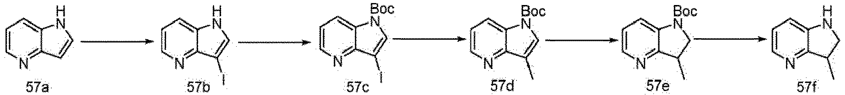

Step 1: NIS (5.20 g, 23.11 mmol) was added to a solution of Compound 57a (2.6 g, 22.01 mmol) in THF (40 mL), and the mixture was stirred at room temperature for 1 hour. The reaction system was concentrated, separated and purified by column chromatography (DCM : MeOH 10:1) to give Compound 57b (5.37 g, 100% yield). MS m/z (ESI): 244.9 [M+1]⁺.

Step 2: Di-tert-butyl dicarbonate (4.8 g, 22.01 mmol) was added dropwise to a solution of Compound 57b (5.37 g, 22.01 mmol) and 4-dimethylaminopyridine (2.69 g, 22.01 mmol ) in triethylamine (10 mL) and tetrahydrofuran (50 mL), and the reaction system was stirred at room temperature for 1 hour. The reaction system was concentrated, separated and purified by column chromatography (petroleum ether : ethyl acetate 4:1) to give Compound 57c (7.57 g, 100% yield). MS m/z (ESI): 344.9 [M+1], 288.9 [M-56+H]⁺.

Step 3: To a reaction system of Compound 57c (7.37 g, 21.42 mmol) in tetrahydrofuran (100 mL), n-BuLi (23.56 mmol, 14.73 mL) was added dropwise at -78°C, and the mixture was stirred for 1 hour, Then iodomethane (3.65 g, 25.70 mmol) was added dropwise and the reaction system was slowly warmed to room temperature. The reaction was quenched with saturated ammonium chloride (5 mL) and the reaction system was concentrated. The resultant was separated by column chromatography (petroleum ether : ethyl acetate 4:1) to give Compound 57d (950 mg, 19% yield). MS m/z (ESI): 233.1 [M+1]⁺.

Step 4: The reaction system of compound 57d (900 mg, 3.87 mmol) and Pd/C (450 mg, 10% purity) in ethanol was hydrogenated with hydrogen at 60°C, and then the reaction system was stirred for 12 hours. The reaction system was filtered and concentrated to give Compound 57e (900 mg, 99% yield). MS m/z (ESI): 235.1 [M+1]⁺.

Step 5: A solution of Compound 57e (200 mg, 853.63 µmol) in TFA (2 mL) and DCM (3 mL) was stirred at RT for 1 hour. The reaction system was concentrated and separated by column chromatography (DCM : MeOH 15:1) to give Compound 57f (90 mg, 78% yield). MS m/z (ESI): 135.1 [M+1]⁺.

### Preparative Example 13: Preparation of Compound 58c

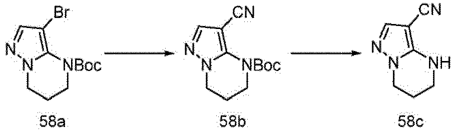

Step 1: Compound 58a (200 mg, 661.89 µmol) and TEA (200.93 mg, 1.99 mmol, 276.95 µL) were dissolved in DMF (4 mL), to which CuCN (309.76 mg, 3.31 mmol) was then added. The reaction system was microwave stirred at 120°C for 3 hours. After the reaction was completed, it was quenched with 30 mL of aqueous solution, and 80 mL of ethyl acetate was added. The resultant was filtrated through diatomaceous earth and extracted 3 times with 50 mL of ethyl acetate, and the organic phase was washed one time with 30 mL of saturated saline and dried with anhydrous sodium sulfate. The solvent was spin-dried under reduced pressure. The sample was mixed with silica gel and purified by silica gel column chromatography (petroleum ether : ethyl acetate=6:1) to give Compound 58b (130 mg, 79.1% yield). MS m/z (ESI): 193.1 [M-56+H]⁺.

Step 2: Compound 58b (130 mg, 523.60 µmol) was dissolved in MeOH (3.0 mL), to which a solution of HCl in MeOH (523.60 µmol, 1.0 mL) was added. The reaction system was stirred at 25°C for 1.0 hour. After the reaction was completed, it was quenched with 20 mL of aqueous solution, and 30 mL of ethyl acetate was added. The resultant was filtrated through diatomaceous earth and extracted 3 times with 40 mL of ethyl acetate, and the combined organic phase was washed with 40 mL of saturated saline, dried with anhydrous sodium sulfate. The solvent was spin-dried under reduced pressure. The sample was mixed with silica gel and purified by silica gel column chromatography (dichloromethane : methanol=15:1) to give Compound 58c (60 mg, 77.3% yield). MS m/z (ESI): 149.1[M+1]⁺.

### Preparative Example 14: Preparation of Compound 3e

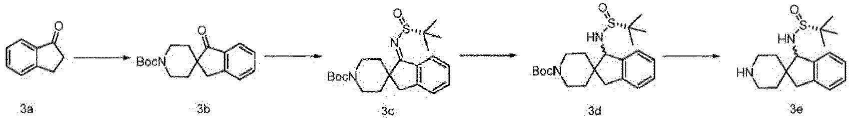

Step 1: Sodium hydride (10 g, 250 mmol) was added to a solution of Compound 3a (13.2 g, 100 mmol) and tert-butyl bis(2-chloroethyl)carbamate (2.42 g, 100 mmol) in DMF (100 mL) at room temperature, and the reaction system was stirred at room temperature for 0.5 hour, then at 60°C for 4 hours. The reaction was cooled to room temperature and quenched by adding 270 mL of saline, and the reaction system was extracted with ethyl acetate (80 mL*3). The organic phases were combined, dried with anhydrous sodium sulfate, concentrated, and purify by column chromatography (petroleum ether/ethyl acetate 5:1). Compound 3b (11.3 g, 37.6% yield) was obtained. MS m/z (ESI): 246.0 [M-56+H]⁺.

Step 2: R-(+)-tert-butylsulfinamide (18.3 g, 150.1 mmol) was added to a solution of Compound 3b (11.3 g, 37.5 mmol) in Ti(EtO)₄ (150 mL), and the mixture was stirred at 90°C for 20 hours. To the reaction system, ethyl acetate (100 mL) and 400 mL of saline were added and the mixture was stirred for 5 min. The reaction system was filtrated through diatomaceous earth, and the filtrate was layered. The organic layer was washed with saline (100 mL*2), dried with anhydrous sodium sulfate, and concentrated. The resultant was separated and purified by column chromatography (0-35% ethyl acetate/petroleum ether) to give Compound 3c (9.8 g, 64.6% yield). ms m/z (ESI): 305.1 [M-100+H]⁺.

Step 3: At -30 °C, NaBH₄ (1.4 g, 36.4 mmol) was added batchwise to a solution of compound 3c (9.8 g, 24.3 mmol) in THF (70 mL), and then the mixture was stirred at room temperature for 12 hours. To the reaction system, ethyl acetate (50 mL) and 100 mL of saline were added, and the mixture was stirred for 5 min. The filtrate was layered and the organic layer was washed with saline (50 mL*2), dried with anhydrous sodium sulfate, and concentrated to give Compound 3d (10.1 g, 100% yield). MS m/z (ESI): 307.1 [M-100+H]⁺.

Step 4: TFA (10 mL) was added to a solution of compound 3d (10.1 g, 24.8 mmol) in dichloromethane (40 mL) and the mixture was stirred for 2 hours. The reaction system was separated and purified by column chromatography (DCM : MeOH=7:1) to give Compound 3e (6.8 g, 89.4% yield). MS m/z (ESI): 307.1 [M+1]⁺.

### Preparative Example 15: Preparation of Compound 3e

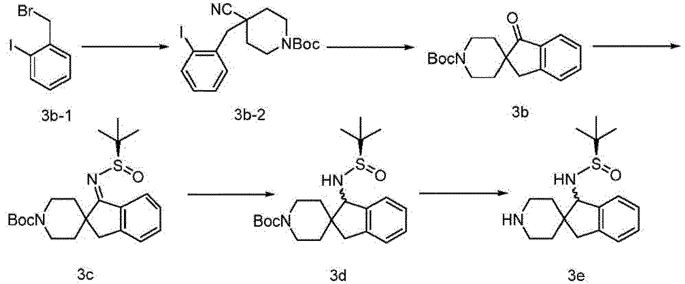

Step 1: N-Boc-4-cyanopiperidine (648 mg, 3.08 mmol) was dissolved in THF (10 mL), and LDA (373.03 mg, 3.48 mmol, 1.74 mL) was added slowly at -78°C under N₂ protection. The mixture was stirred for 45 min. Compound 3b-1 (3.70 mmol) dissolved in THF (3 mL) was slowly added dropwise to the reaction system. When the addition was complete, the reaction was continued at 25°C with stirring for 45 min. The reaction was monitored by LC-MS and TLC (PE/EA=10/1) until completion. The reaction mixture was purified by silica gel column chromatography (12 g, 0-14% ethyl acetate/petroleum ether) to give compound 3b-2.

Step 2: Compound 3b-2 (1.34 mmol) was dissolved in DMF (5 mL) and water (500 µL), and P(tBu)₃ Pd G2 (138 mg, 269.32 µmol) and triethylamine (326.48 mg, 3.23 mmol, 450 µL) were added under N₂ protection. The reaction system was heated and stirred under an oil bath at 130°C for 12 hours. After the reaction system was cooled to room temperature, the reaction was quenched by adding 150 mL of water, poured into a separatory funnel, and extracted by adding ethyl acetate (30 mL*2), and the organic phases were combined, washed with saline (40 mL), dried with anhydrous sodium sulfate, filtered, concentrated and spin-dried. The resultant was purified by silica gel column (4 g, 0-16% ethyl acetate/petroleum ether) to give Compound 3b.

Step 3: Compound 3b (1.20 mmol), (R)-(+)-tert-butylsulfinamide (219.00 mg, 1.81 mmol) and Ti(OEt)₄ (882 mg, 4.82 mmol, 1.6 mL) were reacted in a reaction vial under N₂ at 80°C. The reaction was monitored by LC-MS and TLC (PE/EA=5/1). The reaction was completed when the raw material disappeared. After the reaction was cooled down to room temperature, the reaction was quenched by adding water, and the reaction system was dissolved by adding ethyl acetate (30 mL) and filtered through a diatomaceous earth layer. The filtrate was poured into a separatory funnel and extracted by adding EtOAc (30 mL*2), and the organic phases were combined, washed with saline (40 mL), dried with anhydrous sodium sulfate, filtered, concentrated and spin-dried. The resultant was purified by silica gel column (12g, 0-21% ethyl acetate/petroleum ether) to give Compound 3c.

Step 4: Compound 3c (929.39 µmol) was dissolved in THF (5.0 mL) and DIBAL-H (199.50 mg, 1.41 mmol, 250 µL) was added dropwise. The reaction system was stirred for 2 hours. The reaction was monitored by LC-MS and TLC (PE/EA=5/1) until the completion. The reaction was quenched by adding appropriate amount of water, and the reaction system was filtered through a diatomaceous earth layer and washed with EtOAc (40 mL). The filtrate was washed with saline (40 mL), dried with anhydrous sodium sulfate, filtered, concentrated and spin-dried. The resultant was purified by silica gel column (4 g, 0-32% ethyl acetate/petroleum ether) to give Compound 3d.

Step 5: Compound 3d (533.72 µmol) was dissolved in DCM (3 mL) and trifluoroacetic acid (471 mg, 4.13 mmol) was added dropwise, and the reaction system was stirred for 2 hr. The reaction was monitored by LC-MS and TLC (DCM/MeOH=10/1) until the completion. The reaction system was spin-dried under reduced pressure, and DCM (10 mL) was added. The resultant was concentrated, spin-dried, and purified by silica gel column (4 g, 0-20% methanol/dichloromethane) to give Compound 3e.

### Confirmation of the Absolute Configuration of Compound 3d

To a white transparent glass bottle, 100mg of Compound 3d (prepared from Preparative Examples 14 and 15), 1mL of ethyl acetate, and 5mL of heptane were added, and the mixture was shaked until dissolved. The solution was filtered and slowly evaporated to precipitate crystals. The absolute configuration of the crystal was confirmed to be (S) configuration through single crystal X-ray analysis using D8 venture X-ray single crystal diffractometer from BRUKER, and the structural diagram was shown in Figure 1. Therefore, the absolute configuration of intermediate Compound 3d is the (S) configuration, and the absolute configuration of intermediate Compound 3e is also the (S) configuration.

### Instrument parameters:

| | |
|---|---|
| Light source: Cu target | Current and voltage: 50 kV, 1.2 mA |
| X-ray: Mo-K α (*λ*=1.54178 Å) | Exposure time: 10 s |
| Detector: CMOS surface detector | Distance from surface detector to sample: 40 mm |
| Resolution: 0.80 Å | Test temperature: 170(2) K |

### Preparative Examples 16 to 24: Preparation of Compounds 3f to 3n

Compounds 3f to 3n were prepared with reference to the method of Preparative Example 15.

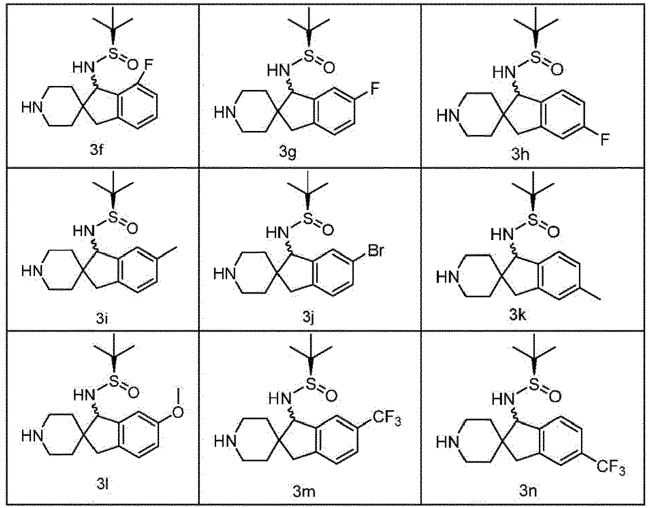

### Preparative Examples 25 to 26: Preparation of Compounds 3o and Compound 3p

(R)-(+)-tert butylsulfonamide was replaced by tert-butylsulfonamide, and Compound 3o and Compound 3p can be prepared with reference to the method of Preparative Example 15.

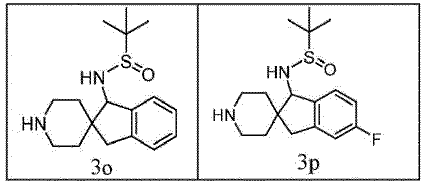

In the following examples 2-126, wavy line 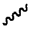 in the compounds represents the 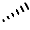 configuration.

### Example 1: Preparation of Compound 1

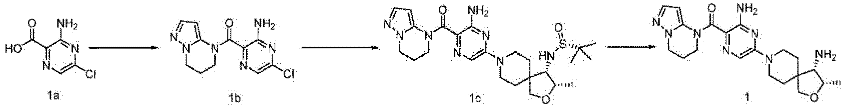

Step 1: Compound 1a (173 mg, 1 mmol) and 4,5,6,7-tetrahydropyrazolo[1,5-a]pyrimidine (123 mg, 1 mmol) were dissolved in ultra-dry DMF (10 mL), HATU (570 mg, 1.5 mmol) and TEA (131 mg, 1.3 mmol) were added. The mixture was stirred for 1 hour at room temperature, and the reaction was monitored with LC-MS until the raw material was consumed up. Then 50 mL of water was added, and the resultant was extracted with ethyl acetate (30 mL*3). The organic phase was washed with water (50 mL*2) and saturated saline (50 mL), respectively, and added with anhydrous sodium sulfate (5 g). The resultant was stirred for 5 min and filtrated to obtain the filtrate, which was concentrated under reduced pressure to obtain Compound 1b (231 mg). MS m/z(ESI): 279 [M+1]⁺.

Step 2: (3S,4S)-tert-butyl-4-((R)-1,1-dimethylethylsulfinylamino)-3-methyl-2-oxa-8-azaspiro [4.5]decane-8-carboxylate (123 mg, 327 µmol) was dissolved in DCM (5 mL), to which TFA (1 mL) was added. The reaction was carried out at 20°C with stirring for 0.5 hour. After the reaction was finished, the solvent was evaporated under reduced pressure, Compound 1b (70 mg, 251 µmol) dissolved in DMF (8 mL) was added, followed by the addition of K₂CO₃ (87 mg, 628 µmol). The system was heated to a temperature of 80°C and reacted for 15 hours. The resultant was concentrated by spin-drying, and separated by silica gel column (dichloromethane : methanol=100:1 to 15:1) to give Compound 1c (80 mg). MS m/z (ESI): 517.2[M+1]⁺.

Step 3: Compound 1c (75 mg, 145 µmol) was dissolved in methanol (8.0 mL), and then a solution of hydrochloric acid in dioxane (4 M, 0.5 mL) was added, and the reaction was carried out at room temperature 25°C for 0.5 hour. It was concentrated by spin-drying, separated and purified by preparative liquid chromatography (acidic), and then freeze-dried to give Compound 1 (47 mg, 77% yield). MS m/z (ESI): 413.3[M+1] ⁺. ¹H NMR (400 MHz, DMSO-d6) δ 8.24 (s, 1H), 7.60 (s, 1H), 7.31 (d, J = 2.0 Hz, 1H), 6.72 (s, 2H), 6.26 (d, J = 2.0 Hz, 1H), 4.14 - 4.10 (m, 3H), 4.03 - 3.85 (m, 4H), 3.71 (d, J = 8.6 Hz, 1H), 3.51 (d, J = 8.6 Hz, 1H), 3.36 - 3.30 (m, 2H), 2.98 (d, J = 5.3 Hz, 1H), 2.11 - 2.06 (m, 2H), 1.78 - 1.39 (m, 4H), 1.10 (d, J = 6.4 Hz, 3H).

### Example 2: Preparation of Compound 2

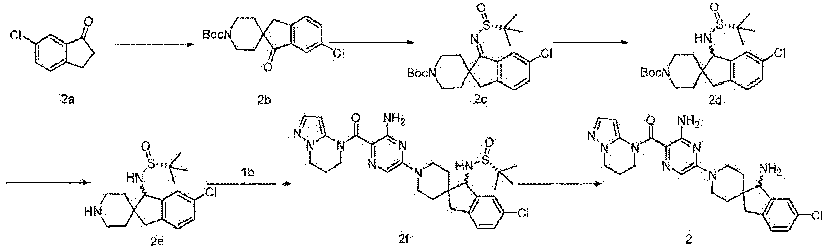

Step 1: Compound 2a (10 g, 60 mmol) was dissolved in DMF (200 mL), the solution was cooled to 0°C, to which NaH (60% purity, 6 g, 150 mmol) was then added batchwise. The reaction system was stirred for 0.5 hour at 0°C. Then, tert-butyl N,N-bis(2-chloroethyl)carbamate (16 g, 66 mmol) was added. The system was heated to a temperature of 60°C and reacted for another 16 hours. After the reaction was completed, saturated saline was added to the reaction system, and then the resultant was extracted with ethyl acetate. The organic phase was collected and dried by adding anhydrous sodium sulfate. The organic phase was evaporated under reduced pressure, and dichloromethane was added. The sample was mixed with silica gel, and purified by silica gel column chromatography (petroleum ether : ethyl acetate=5:1) to give Compound 2b (2.5 g). MS m/z (ESI):280.1[M-56+H]⁺.

Step 2: Compound 2b (1.46 g, 4.35 mmol) was dissolved in tetraethyl titanate (10 mL), to which R-(+)-tert-butylsulfinamide (1.05 g, 8.70 mmol) was then added. The reaction system was stirred at 120°C for 1.5 hours. After the reaction was completed, the reaction system was cooled to room temperature, diluted by adding ethyl acetate, and then the reaction system was added to water. The mixture was filtered, and the filtrate was collected. Then the filter cake was washed with ethyl acetate and all the filtrates were combined. The filtrate was extracted with ethyl acetate and the organic phase was collected. The organic phase was dried with anhydrous sodium sulfate, and the solvent ethyl acetate was spin-dried under reduced pressure. Then the resultant was purified by silica gel column chromatography (petroleum ether : ethyl acetate=9:1 to 4:1) to give Compound 2c (1.8 g). MS m/z (ESI): 339.1[M-100+H]⁺.

Step 3: Compound 2c (800 mg, 1.82 mmol) was dissolved in THF (10 mL), the solution was cooled to -78°C. DIBAL-H (1 M, 2 mL) was added dropwise. The reaction system was stirred at - 78°C for 0.5 hour. When the reaction was completed, 5 mL of THF was added for dilution, and then sodium sulfate decahydrate was added. Then ethyl acetate was added, and the resultant was layered. The organic phase was collected and the solvent was evaporated under reduced pressure to give Compound 2d (960 mg). MS m/z (ESI):341.1[M-100+H]⁺.

Step 4: Compound 2d (225 mg, 510 µmol) was dissolved in DCM (5 mL), to which TFA (1 mL) was added. The reaction system was stirred at 20°C for 0.5 hour. When the reaction was completed, the solvent was evaporated under reduced pressure to give Compound 2e (245 mg). MS m/z (ESI):341.1 [M+H]⁺.

Step 5: Compound 1b (100 mg, 179 µmol) and Compound 2e (61 mg, 179 µmol) were dissolved in DMF (3 mL), to which potassium carbonate (74 mg, 538 µmol) was added. The reaction system was stirred at 80°C for 16 hours. After the reaction was completed, DMF was evaporated under reduced pressure, and dichloromethane was added. The sample was mixed with silica gel and purified by silica gel column chromatography (petroleum ether : ethyl acetate=1:1 to ethyl acetate (100%) to dichloromethane : methanol=85%:15%) to give Compound 2f (110 mg). MS m/z (ESI): 583.2 [M+H]⁺.

Step 6: Compound 2f (110 mg, 189 µmol) was dissolved in methanol (5 mL), to which a solution of HCl in dioxane (4 M, 1 mL) was added. The reaction system was stirred for 0.5 hour at 20 °C. The solvent was spin-dried, and the resultant was separated and purified by preparative liquid chromatography (alkaline) to give Compound 2 (26 mg, 28% yield). MS m/z (ESI): 479.1[M+H]⁺. ¹H-NMR(400 MHz, DMSO-d6): δ 7.60 (s, 1H), 7.30 (s, 2H), 7.19 (s, 2H), 6.72 (s, 2H), 6.25 (s, 1H), 4.27 (s, 2H), 4.12 (s, 2H), 3.97 (s, 2H), 3.83 (s, 1H), 3.14-3.04 (m, 3H), 2.59 (d, *J =* 15.4 Hz, 1H), 2.07 (s, 2H), 1.74-1.49 (m, 3H), 1.05 *(d, J=* 13.0 Hz, 1H).

### Example 3: Preparation of Compound 3

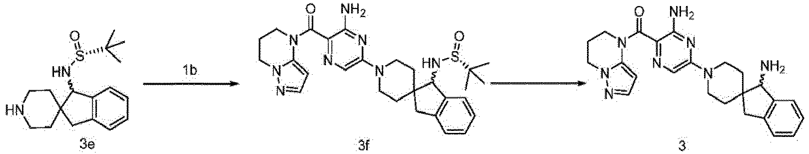

Compound 3 was prepared from Compound 3e as starting material with reference to the conditions of Steps 5 and 6 in Example 2. MS m/z(ESI): 445 [M+1]⁺. ¹H NMR (400 MHz, DMSO-*d*₆) 7.58 (s, 1H),7.29(d, *J =* 2.5 Hz, 1H), 7.31 - 7.27 (m, 2H), 7.16-7.14 (m, *J =* 5 Hz, 3H), 6.69 (s, 2H), 6.23 (s, 1H), 4.24 (d, *J =* 5.6 Hz, 2H), 4.11 - 4.08 (t, *J* = 6.0 Hz, 2H), 3.98 - 3.94 (m, 2H), 3.89 (s, 1H), 3.19 - 3.03 (m, 3H), 2.66 d, *J=* 16.0 Hz,1H), 2.06 (m, 2H), 1.75 - 1.67 (m, 1H), 1.66 - 1.58 (m, 1H), 1.47 (d, *J* = 13.2 Hz, 1H) , 1.15 (d, *J=* 13.2 Hz,1H).

### Example 4 to Example 14

For the preparation methods of Compound 4 to Compound 14, refer to Example 2.

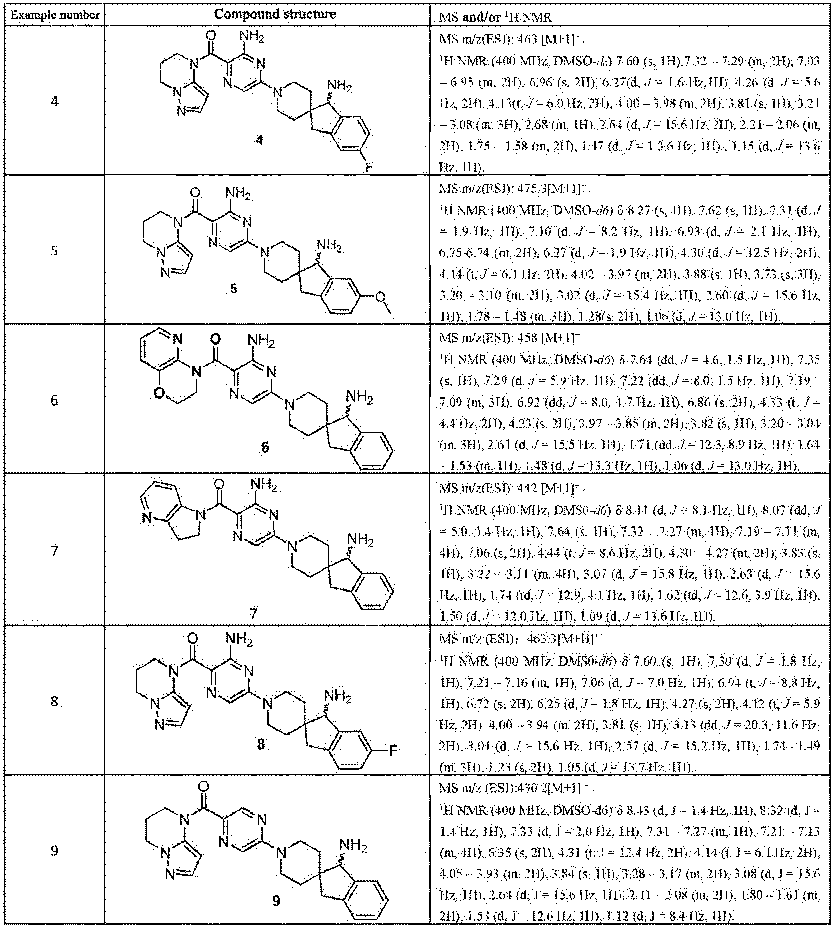

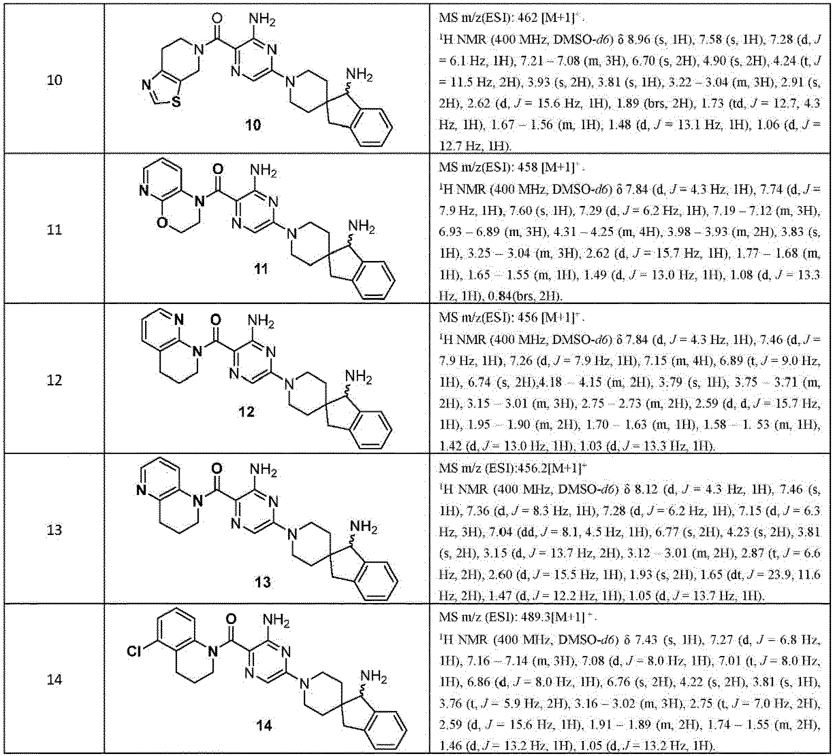

### Example 15: Preparation of Compound 15

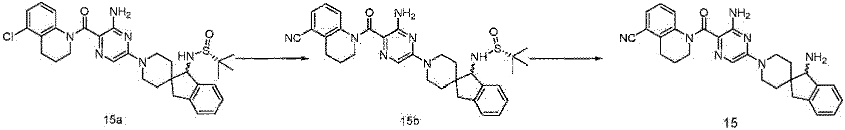

Step 1: After Compound 15a (120 mg, 202.30 µmol, synthesized with reference to the synthetic scheme of Compound 2f in Example 2) was dissolved in THF (5 mL) and H₂O (5 mL), t-Bu-XPhos-Pd-G3 (32.13 mg, 40.46 µmol) and Zn(CN)₂ (71.26 mg, 606.90 µmol) were added, and argon replacement was performed three times. Then the system was heated to a temperature of 90°C, reacted for 18 hours, cooled to room temperature, then diluted by adding dichloromethane, and filtered. The filter cake was washed with dichloromethane, and the filtrate was extracted with dichloromethane. The organic phases were combined, dried with anhydrous sodium sulfate and concentrated, and Compound 15b (95 mg, 80.5% yield) was obtained by silica gel chromatography (PE : EA=5:1 to 1:5). MS m/z (ESI): 584.3[M+1]⁺.

Step 2: Compound 15b (70 mg, 119.92 µmol) was dissolved in methanol (6 mL), and then a solution of hydrochloric acid in dioxane (4 M, 1 mL) was added. The reaction was carried out at room temperature 25°C for 0.5 hour. After the reaction was completed, it was directly concentrated by spin-drying to obtain a yellow oily material, and then it was dissolved again in methanol. The resultant was alkalized by adding triethylamine and spin-dried, separated by preparative liquid chromatography (alkaline method), and freeze-dried to give Compound 15 (32.4 mg, 55.9% yield). MS m/z (ESI): 480.3[M+1] ⁺. ¹H NMR (400 MHz, DMSO-*d6*) δ 7.50 - 7.39 (m, 2H), 7.32 - 7.20 (m, 2H), 7.19 - 7.13 (m, 4H), 6.82 (s, 2H), 4.23 (d, J = 10.0 Hz, 2H), 3.88 - 3.73 (m, 3H), 3.17 - 3.03 (m, 3H), 2.90 (t, J = 6.7 Hz, 2H), 2.63 (d, J = 15.7 Hz, 1H), 1.93 (p, J = 6.6 Hz, 2H), 1.78 - 1.40 (m, 3H), 1.09 (d, J = 13.3 Hz, 1H).

### Example 16 to Example 22

For the preparation methods of Compound 16 to Compound 22, refer to Example 2.

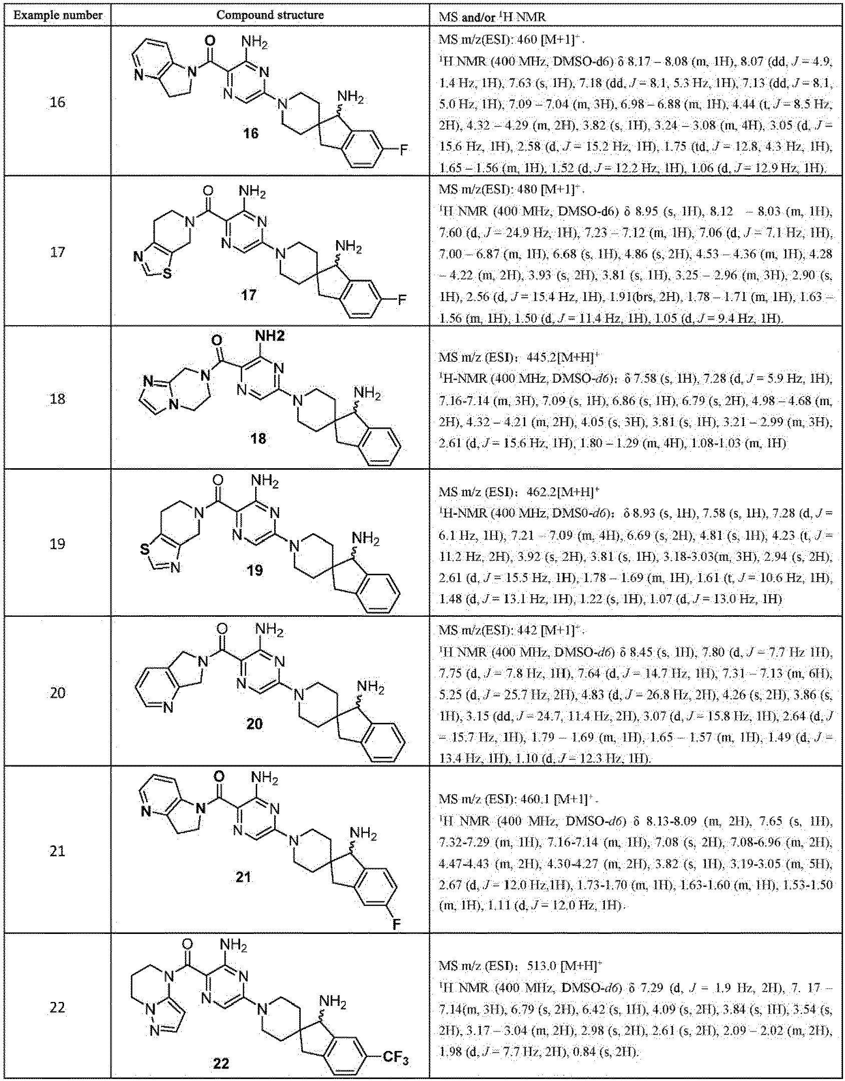

### Example 23: Preparation of Compound 23

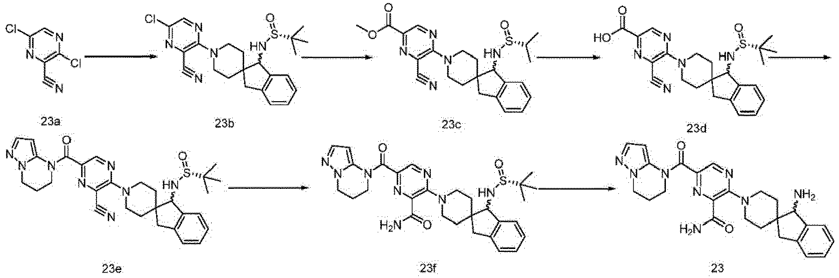

Step 1: Compound 23a (173 mg, 1 mmol) and Compound 3e (245 mg, 0.8 mmol) were dissolved in ultra-dry THF (15 mL), and then TEA (202 mg, 2 mmol) was added at room temperature. The mixture was stirred at room temperature for 0.5 hour. After concentration under reduced pressure, it was separated and purified by silica gel column chromatography (12 g, 0-50% ethyl acetate/petroleum ether) to give Compound 23b (235 mg 52.9% yield). MS m/z(ESI): 445 [M+1]⁺.

Step 2: Compound 23b (235 mg, 0.53 mmol) and molybdenum hexacarbonyl (278 mg, 1.06 mmol) were dissolved in MeOH (5 mL), and Pd(OAc)₂ (25 mg, 0.2 mmol), BINAP (68 mg, 0.2 mmol) and TEA (1 mL) were sequentially added to the reaction system. Argon replacement was performed three times. The reaction was carried out at 90°C with microwave stirring for 2 hours. The reaction liquid was cooled to room temperature and spin-dried, and separated by silica gel column chromatography (12 g, 0-60% ethyl acetate/petroleum ether) to give Compound 23c (135 mg, 46.1% yield). MS m/z(ESI): 468[M+1]⁺.

Step 3: Compound 23c (95 mg, 0.2 mmol) was dissolved in THF (8 mL) and water (4 mL), and LiOH (21 mg, 0.5 mmol) was added. The reaction system was stirred at room temperature (24°C) for 1 hour. Compound 23d (123 mg, 90% yield) was obtained by concentration. MS m/z(ESI): 454 [M+1]⁺.

Step 4: Compound 23d (115 mg, 0.25 mmol) and 4,5,6,7-tetrahydropyrazolo[1,5-a]pyrimidine (32 mg, 0.25 mmol) were dissolved in ultra-dry DMF (8 mL), and then HATU (142 mg, 0.38 mmol) and TEA (33 mg, 0.32 mmol) were added. The mixture was stirred at room temperature for 1 hour. Then 50 mL of water was added, and the resultant was extracted with ethyl acetate (30 mL*3). The organic phase was washed with water (50 mL*2) and saturated saline (50 mL), and the organic phase was added with anhydrous sodium sulfate (5 g) and stirred for 5 min, filtered to obtain the filtrate, which was concentrated under reduced pressure to give Compound 23e (80 mg, 63.1% yield). MS m/z (ESI): 559 [M+1]⁺.

Step 5: Compound 23e (80 mg, 0.143 mmol) was dissolved in ammonia water (1 mL), and then H₂O₂ (1 mL) was added. The reaction system was stirred at room temperature (24°C) for 0.5 hour. Compound 23f (73 mg, 87% yield) was obtained by concentration. MS m/z(ESI): 577 [M+1]⁺.

Step 6: Compound 23f (73 mg, 0.13 mmol) was dissolved in MeOH (5 mL), and then a solution of hydrochloric acid in dioxane (4M, 0.5 mL) was added to the reaction system. The reaction system was stirred for 20 min at room temperature (24°C). The final product Compound 23 (8 mg, 25.3% yield) was obtained by purification. MS m/z(ESI): 473 [M+1]⁺. ¹H NMR (400 MHz, DMSO-*d*₆) 8.51 (s, 1H), 7.98 (s, 1H), 7.67 (s, 1H), 7.37 (s, 1H), 7.29 (d, *J =* 5.9 Hz, 1H), 7.16 (m,3H), 6.50 (s, 1H), 4.12 (d, *J =* 6.1 Hz, 2H), 4.03 - 3.98 (m, 4H), 3.81 (s, 1H), 3.29 - 3.20 (m, 2H), 3.04 (d, *J =* 15.6 Hz, 1H), 2.62 (d, *J =* 15.8 Hz,1H), 2.11 - 2.07 (m, 2H), 1.79 -1.72 (m, 1H), 1.70 -1.64 (m, 1H), 1.48(d, *J* = 13.2 Hz,1H) , 1.16 (d, *J* = 13.5 Hz, 1H).

### Example 24 to Example 26

Compound 24 was prepared using Compound 24g as the raw material with reference to the method of Example 2. Compound 25 was prepared using Compound 25a as the raw material with reference to the method of Example 2. Compound 26 was prepared using Compound 26h as the raw material with reference to the method of Example 2. Compound 30 was prepared using Compound 30a as the raw material with reference to the method of Example 29.

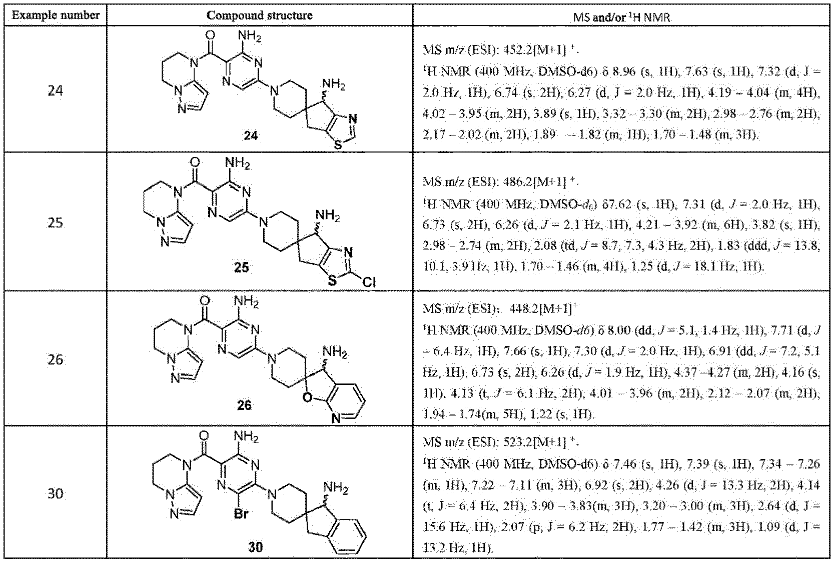

### Example 27: Preparation of Compound 27

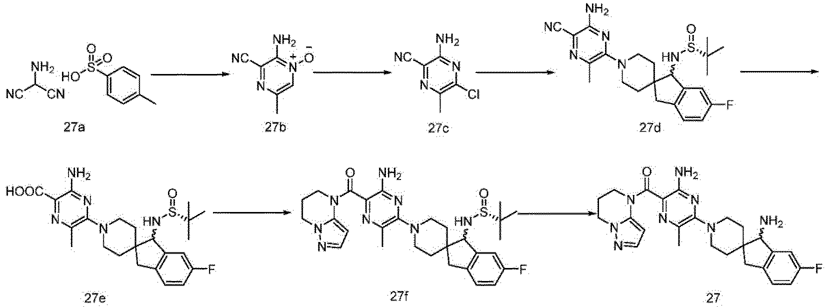

Step 1: Aminomalononitrile p-toluenesulfonate 27a (2.02 g, 8.04 mmol) and 2-oxopropanal-1-oxime (700 mg, 8.04 mmol) were dissolved in isopropanol (15 mL). The reaction system was stirred at 25°C for 12 hours. Upon completion of the reaction, the Compound 27b (1.02 g, 84.5% yield) was obtained by filtration. MS m/z (ESI): 151.0 [M+1]⁺.

Step 2: Compound 27b (700 mg, 4.66 mmol) was dissolved in DMF (20 mL) and then POCl₃ (1 mL) was added to it. The reaction system was stirred at 25°C for 1.0 hour. After the reaction was completed, it was quenched with 30 mL of aqueous solution, and 60 mL of ethyl acetate was added. The resultant was filtrated through diatomaceous earth and extracted 3 times with 60 mL of ethyl acetate, and the organic phase was washed with 30 mL of saturated saline, dried with anhydrous sodium sulfate. The solvent was spin-dried under reduced pressure. The sample was mixed with silica gel and purified by silica gel column chromatography (petroleum ether : ethyl acetate=3:1) to give Compound 27c (786 mg, 67.4% yield). MS m/z (ESI): 169.0[M+1]⁺.

Step 3: Compound 27c (100 mg, 593.18 µmol) and Compound 3g (192.46 mg, 593.18 µmol) were dissolved in DMF (5 mL), to which K₂CO₃ (245.95 mg, 1.78 mmol) was then added. The reaction system was stirred at 80°C for 2 hours. The reaction was quenched with 30 mL of aqueous solution, and reaction system was extracted 3 times with 30 mL of ethyl acetate. The organic phase was washed with 30 mL of saturated saline and dried with anhydrous sodium sulfate, and the solvent was spin-dried under reduced pressure. The sample was mixed with silica gel and purified by silica gel column chromatography (petroleum ether : ethyl acetate=3:1) to give Compound 27d (70 mg, 25.8% yield). MS m/z (ESI):457.0[M+1]⁺.

Step 4: Compound 27d (60 mg, 131.41 µmol) in MeOH (2 mL) was placed in a microwave tube and NaOH (105.13 mg, 2.63 mmol) was added. The reaction system was stirred in the microwave reactor at 100°C for 30 min. After the reaction was completed, it was neutralized by adding 4 M of aqueous hydrochloric acid to pH=4-6, extracted 3 times with 30 mL of ethyl acetate, and dried with anhydrous sodium sulfate, and the solvent was spin-dried. The sample was mixed with silica gel and purified by silica gel column chromatography (dichloromethane : methanol=7:1) to give Compound 27e (50 mg, 80.0% yield). MS m/z (ESI): 476.0 [M+1]⁺.

Step 5: Compound 27e (40 mg, 84.11 µmol) and 4,5,6,7-tetrahydropyrazolo[1,5-a]pyrimidine (15.54 mg, 126.16 µmol) in DMF (4 mL) were placed in a 25 ml eggplant-shaped bottle and HATU (47.60 mg, 126.16 µmol) was added. The reaction system was stirred at 30°C for 1.0 hour. After the reaction was completed, it was quenched with 30 mL of aqueous solution, and the reaction system was extracted 3 times with 60 mL of ethyl acetate. The organic phase was washed with 30 mL of saturated saline and dried with anhydrous sodium sulfate, and the solvent was spin-dried under reduced pressure. The sample was mixed with silica gel and purified by silica gel column chromatography (dichloromethane : methanol=15:1) to give Compound 27f (40 mg, 81.9% yield). MS m/z (ESI):581.0 [M+1]⁺.

Step 6: Compound 27f (40 mg. 68.88 µmol) was dissolved in a solution of HCl in MeOH (1 mL) and MeOH (5 mL). The reaction system was stirred for 0.5 hour at 25°C. The HCl-MeOH was spin-dried and the resultant was purified by prep-HPLC (alkaline) to give the final product Compound 27 (4.0 mg, 12.0% yield). MS m/z (ESI): 477.0[M+H]⁺. ¹H NMR (400 MHz, DMSO-*d6*) δ 7.31 (s, 1H), 7.19 - 7.18 (m, 1H), 7.09 (d, *J =* 8.5 Hz, 1H), 6.95 - 6.94 (m, 1H), 6.46 (s, 2H), 6.31 (s, 1H), 4.13 - 4.12 (m, 2H), 3.94 (s, 2H), 3.87 (s, 1H), 3.70-3.65 (m, 2H), 3.02 - 2.97 (m, 3H), 2.57 (d, *J* = 12.8 Hz, 1H), 2.29 (s, 3H), 2.08 (s, 2H),1.99 - 1.74 (m, 3H), 1.53 (d, *J* = 12.8 Hz, 1H).

### Example 28: Preparation of Compound 28

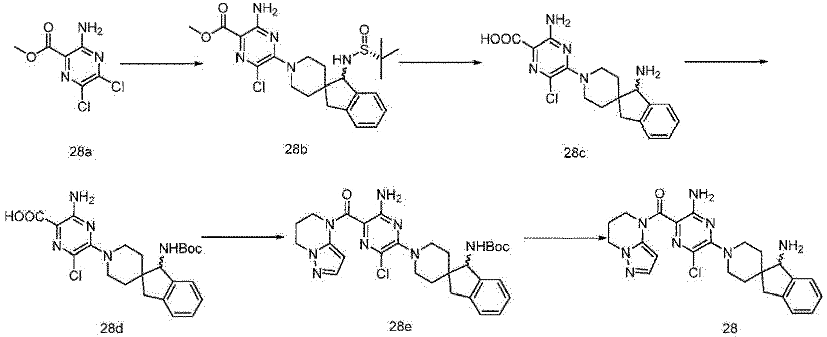

Step 1: A reaction system of Compound 28a (120 mg, 540.47 µmol), Compound 3e (198.76 mg, 648.57 µmol) and DIPEA (349.25 mg, 2.70 mmol, 470.69 µL) in DMF was stirred at room temperature for 2 hours. The reaction system was concentrated, separated and purified by column chromatography (DCM : MeOH 15:1) to give Compound 28b (100 mg, 37% yield). MS m/z (ESI): 492.1 [M+1]⁺.

Step 2: A reaction system of Compound 28b (125 mg, 254.05 µmol) and LiOH (18.25 mg, 762.14 µmol) in THF (5 mL) and H₂O (1 mL) was stirred at room temperature for 2 hours. The reaction system was acidified with concentrated hydrochloric acid (0.5 mL) and concentrated to give Compound 28c (90 mg, 94% yield). MS m/z (ESI): 375.1 [M+1]⁺.

Step 3: A reaction system of Compound 28c (90 mg, 1.33 mmol), DIPEA (93.34 mg, 722.24 µmol, 125.80 µL) and Boc₂O (78.81 mg, 361.12 µmol) in MeOH (5 mL) was stirred at room temperature for 2 hours. The reaction system was concentrated. The resultant was separated by column chromatography (eluent DCM : MeOH= 15:1) to give Compound 28d (105 mg, 92% yield). MS m/z (ESI): 474.1 [M+1]⁺.

Step 4: A solution of Compound 28d (105 mg, 221.54 µmol), 4,5,6,7-tetrahydropyrazolo[1,5-a]pyrimidine (30 mg, 243.70 µmol), HATU (83 mg, 221.54 µmol) and DIPEA (85 mg, 664.63 µmol) in DMF (2 mL) was stirred at room temperature for 4 hours. The reaction system was concentrated and separated by column chromatography (DCM : MeOH=15:1) to give Compound 28e (95 mg, 74% yield). MS m/z (ESI): 579.2 [M+1]⁺.

Step 5: To a solution of Compound 28e (95 mg, 587.58 µmol) in MeOH (2 mL) was added HCl/dioxane (4M, 2 mL), and the reaction system was stirred at room temperature for 2 hours. The reaction system was concentrated, and purified by preparative chromatography to give Compound 28 (12.25 mg, 15% yield). MS m/z (ESI): 479.1 [M+1]⁺. ¹H NMR (400 MHz, DMSO-d6) δ 7.34-7.30 (m, 2H), 7.18-7.16 (m, 3H), 6.92 (brs, 2H), 6.34 (s, 1H), 4.16-4.13 (m, 2H), 4.00-3.98 (m, 2H), 3.93 (s, 2H), 3.86 (s, 1H), 3.19-3.03 (m, 3H), 2. 62 (d, *J* = 16.0 Hz,1H), 2.15-2.06 (m, 2H), 1.91-1.85 (m, 1H), 1.79-1.75 (m, 1H), 1.55 (d, *J* = 12.0 Hz, 1H) ,1.14 (d, *J=* 12.0 Hz, 1H).

### Example 29: Preparation of Compound 29

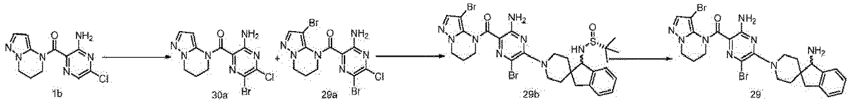

Step 1: Compound 1b (200 mg, 717.62 µmol) was dissolved in acetonitrile (10 mL), and then NBS (114.95 mg, 645.86 µmol) was added at once at room temperature. The reaction was carried out at room temperature 15°C for 0.5 hour. The reaction system was diluted by adding saturated sodium bicarbonate solution and water, and then extracted by adding of ethyl acetate. The organic phases were combined, dried with anhydrous sodium sulfate, concentrated, and purified by silica gel column chromatography (petroleum ether : ethyl acetate=1:10 to 1:5) to give Compound 29a (110 mg, 35.1% yield), MS m/z (ESI): 436.8[M+1]⁺ and Compound 30a (82 mg, 26.9% yield), MS m/z (ESI): 358.9[M+1]⁺.

Step 2: Compound 29a (150.00 mg, 161.52 µmol) and Compound 3e (74.95 mg, 244.55 µmol) were dissolved in DMF (6 mL) and then K₂CO₃ (92.18 mg, 666.96 µmol) was added. The system was heated to 90°C and reacted for 12 hours. The resultant was concentrated by spin-drying and separated by silica gel column chromatography (PE: EA=10:1 to 1:9) to give Compound 29b (40 mg, 25.5% yield). MS m/z (ESI): 707.0[M+1]⁺.

Step 3: Compound 29b (40 mg, 56.62 µmol) was dissolved in methanol (6 mL), and then a solution of hydrochloric acid in dioxane (4 M, 1 mL) was added. The reaction was carried out at room temperature 25°C for 0.5 hour. The reaction system was concentrated by spin-drying, and the resultant was dissolved in methanol again and alkalized by adding triethylamine, and then spin-dried. The resultant was separated by preparative chromatography (alkaline) and freeze-dried to give Compound 29 (25 mg, 73.1% yield). MS m/z (ESI): 601.0[M+1]⁺. ¹H NMR (400 MHz, DMSO-d6) δ 7.43 (s, 1H), 7.30 (d, J = 6.7 Hz, 1H), 7.18 - 7.13 (m, 5H), 4.16 (t, J = 6.5 Hz, 2H), 4.01 - 3.88 (m, 4H), 3.83(s, 1H), 3.21 - 2.96 (m, 3H), 2.60 (d, J = 15.7 Hz, 1H), 2.17 - 2.04 (m, 2H), 2.03 - 1.66 (m, 2H), 1.53 (d, J = 13.2 Hz, 1H), 1.13 (d, J = 13.1 Hz, 1H).

### Example 31: Preparation of Compound 31

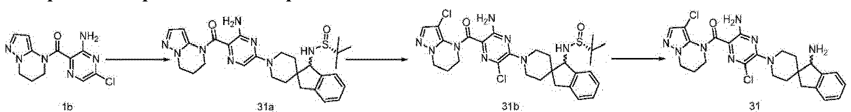

Step 1: Compound 1b (80 mg, 287.05 µmol) and Compound 3e (105.56 mg, 344.46 µmol) were dissolved in DMF (5 mL) and then K₂CO₃ (119.02 mg, 861.15 µmol) was added. The system was heated to 90°C for 6 hours. The resultant was concentrated by spin-drying and separated by silica gel column chromatography (PE: EA=10:1 to 1:9) to give Compound 31a (145 mg, 41.4% yield). MS m/z (ESI): 549.3 [M+1]⁺.

Step 2: Compound 31a (130 mg, 94.77 µmol) was dissolved in ACN (8 mL), followed by the addition of TEA (47.95 mg, 473.85 µmol, 66.09 µL), and then NCS (53.30 mg, 399.18 µmol) was added batchwise under an ice bath. The reaction was carried out at room temperature for 0.5 hour. After the reaction was completed, 5 ml of saturated sodium bicarbonate solution was added and the resultant was extracted with ethyl acetate. The combined organic phases were dried with anhydrous sodium sulfate and concentrated to give Compound 31b (60 mg). MS m/z (ESI): 617.2[M+1]⁺.

Step 3: Compound 31b (45 mg, 72.86 µmol) was dissolved in methanol (6 mL), and then a solution of hydrochloric acid in dioxane (4 M, 1 mL) was added. The reaction was carried out at room temperature 25°C for 0.5 hour. It was concentrated by spin-drying, dissolved in methanol again and alkalized by adding triethylamine, and then spin-dried. The resultant was separated by preparative chromatography (alkaline) and freeze-dried to give Compound 31 (20 mg, 51% yield). MS m/z (ESI): 513.1[M+1] ⁺. H NMR (400 MHz, DMSO-d6) δ 7.44 (s, 1H), 7.31 (d, J = 6.5 Hz, 1H), 7.23 - 7.06 (m, 5H), 4.15 - 4.02 (m, 4H), 3.92 - 3.84 (m, 3H), 3.25 - 2.99 (m, 3H), 2.62 (d, J = 15.7 Hz, 1H), 2.18 - 2.04 (m, 2H), 1.87 - 1.71(m, 2H), 1.53 (d, J = 13.3 Hz, 1H), 1.14 (d, J = 13.2 Hz, 1H).

### Example 32 to Example 39

Compound 32 was prepared using Compound 32c as the raw material with reference to the method of Example 2. Compound 33 was prepared using Compound 33d as the raw material with reference to the method of Example 2. Compound 34 was prepared using Compound 34a as the raw material with reference to the method of Example 2. Compound 35 was prepared using Compound 35c as the raw material with reference to the method of Example 2. Compound 36 and Compound 37 were prepared with reference to the method of Example 2. Compound 38 was prepared using Compound 38h as the raw material with reference to the method of Example 2. Compound 39 was prepared with reference to the method of Example 28.

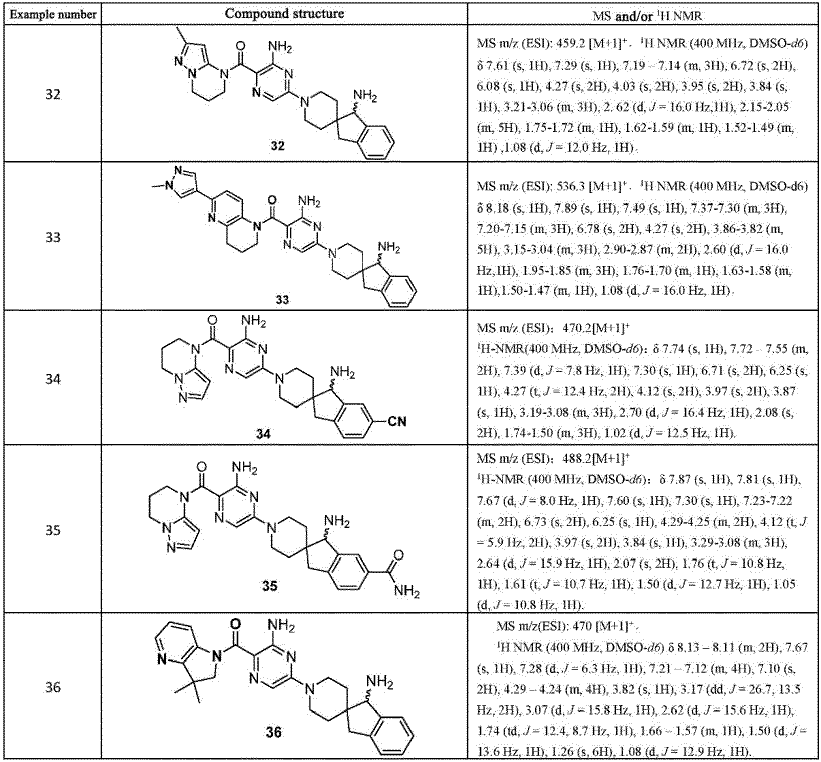

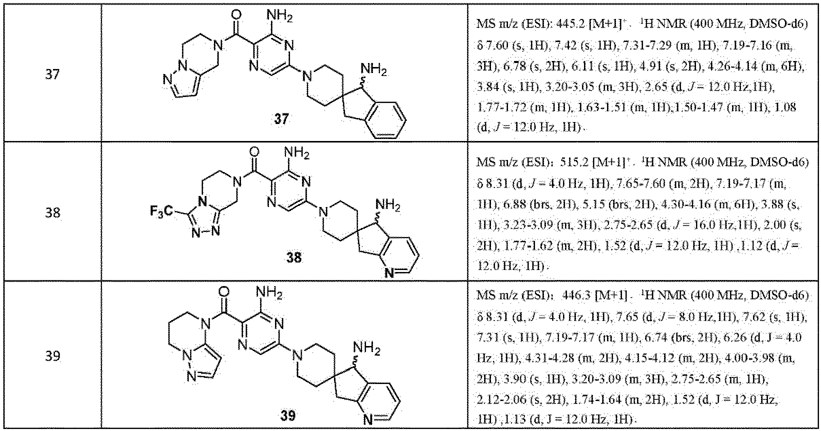

### Example 40: Preparation of Compound 40

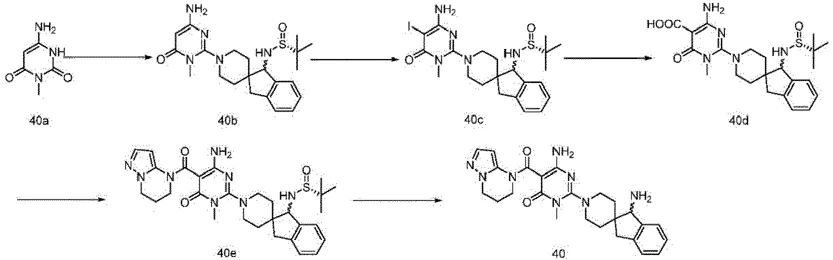

Step 1: 6-Amino-3-methyl-1H-pyrimidin-2,4-dione 40a (150 mg, 1.06 mmol) and PyBOP (1.41 g, 3.19 mmol) were dissolved in DMF (5 mL), and the solution was stirred for 10 min, to which Compound 3e (325.73 mg, 1.06 mmol) and DBU (1.62 g, 10.63 mmol, 1.59 mL) were then added. The reaction was stirred at 25°C for 3 hours. Then 30 mL of aqueous solution was added, and the resultant was extract 3 times with 30 mL of ethyl acetate, washed 3 times with 30 mL of saturated sodium chloride solution and dried with anhydrous sodium sulfate. The solvent was spin-dried. The sample was mixed with silica gel and purified by silica gel column chromatography (petroleum ether : ethyl acetate=2:1) to give Compound 40b (350 mg, 76.7% yield). MS m/z (ESI): 430.0 [M+1]⁺.

Step 2: Compound 40b (330 mg, 768.20 µmol) was dissolved in DMF (1.90 mL), to which TEA (77.73 mg, 768.20 µmol, 107.15 µL) and NIS (172.83 mg, 768.20 µmol) were then added. The reaction system was stirred at 25°C for 1.0 hour. After the reaction was completed, it was quenched with 30 mL of aqueous solution, and the reaction system was extracted 3 times with 50 mL of ethyl acetate. The organic phase was washed with 20 mL of saturated saline and dried with anhydrous sodium sulfate, and the solvent was spin-dried under reduced pressure. The sample was mixed with silica gel and purified by silica gel column chromatography (petroleum ether : ethyl acetate=3:1) to give Compound 40c (250 mg, 58.6% yield). MS m/z (ESI): 456.0[M+1]⁺.

Step 3: Compound 40c (220 mg, 396.06 µmol), Pd(OAc)₂ (17.78 mg, 79.21 µmol), BINAP (49.32 mg, 79.21 µmol), TEA (120.23 mg, 1.19 mmol, 165.72 µL) and MO(CO)₆ (209.12 mg, 792.12 µmol) were dissolved in dioxane (3 mL) and H₂O (3 mL). The reaction was carried out at 90°C for 1.0 hour with microwave stirring. After the reaction was completed, it was quenched with 20 mL of saturated aqueous sodium bicarbonate, and the reaction system was extracted 3 times with 30 mL of ethyl acetate, the organic phase was washed with 30 mL of saturated saline, dried with anhydrous sodium sulfate. The solvent was spin-dried under reduced pressure. The sample was mixed with silica gel and purified by silica gel column chromatography (petroleum ether : ethyl acetate=3:1) to give Compound 40d (130 mg, 69.3% yield). MS m/z (ESI): 474.0[M+1]⁺.

Step 4: Compound 40d (90 mg, 190.04 µmol) and 4,5,6,7-tetrahydropyrazolo[1,5-a]pyrimidine (35.11 mg, 285.06 µmol) were dissolved in acetonitrile (5 mL), to which TCFH (6.85 mg, 24.41 µmol) was then added. The reaction was carried out at 25°C for 4.0 hours with stirring. After the reaction was completed, it was quenched with 10 ml of aqueous solution, and the reaction system was extracted 3 times with 20 mL of ethyl acetate. The organic phase was washed one time with 20 mL of saturated saline and dried with anhydrous sodium sulfate, and the solvent was spin-dried under reduced pressure. The sample was mixed with silica gel and purified by silica gel column chromatography (petroleum ether : ethyl acetate=3:1) to give Compound 40e (45 mg, 40.9% yield). MS m/z (ESI): 579.0[M+1]⁺.

Step 5: Compound 40e (56.87 mg, 98.26 µmol) was dissolved in a solution of HCl in MeOH (1 mL) and MeOH (5 mL). The reaction was carried out at 25°C for 0.5 hour with stirring. The HCl-MeOH was spin-dried and the resultant was purified by prep-HPLC (alkaline) to give the final product Compound 40 (1.2 mg, 2.3% yield). MS m/z (ESI):475.0[M+1]⁺. ¹H NMR (400 MHz, DMSO-*d6*)δ 7.29 (d, *J* = 1.9 Hz, 2H), 7.15 (d, *J* = 5.7 Hz, 3H), 6.79 (s, 2H), 6.42 (s, 1H), 4.09 (s, 2H), 3.84 (s, 1H), 3.59 - 3.48 (m, 2H), 3.23 (s, 3H), 2.98 (s, 2H), 2.72 - 2.52 (m, 2H), 2.12 - 2.02 (m, 2H), 1.98 (d, *J* = 7.7 Hz, 2H), 1.56 - 1.42 (m, 2H), 0.84 (s, 2H).

### Example 41: Preparation of Compound 41

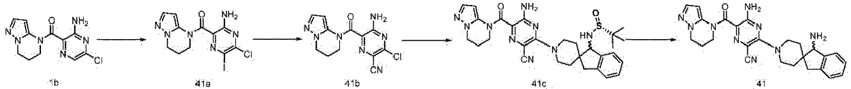

Step 1: Compound 1b (150 mg, 538.22 µmol) was dissolved in ACN (15 mL), and then NIS (90.82 mg, 403.66 µmol) was added batchwise at 0°C. The reaction was carried out at 0°C for 10 min. The reaction was quenched by adding saturated sodium bicarbonate solution, and the aqueous phase was extracted with ethyl acetate. The organic phases were combined, dried with anhydrous sodium sulfate, and concentrated to give Compound 41a (178 mg). MS m/z (ESI): 404.9[M+1]⁺.

Step 2: Compound 41a (148 mg, 365.80 µmol) was dissolved in DMF (6 mL) and then CuCN (49.14 mg, 548.70 µmol) was added. The system was heated to 130°C under argon protection and reacted for 3 hours. The resultant was concentrated by spin-drying and purified by silica gel chromatography (dichloromethane : methanol=1:100 to 1:20) to give Compound 41b (100 mg, 90% yield). MS m/z (ESI): 304.1[M+1]⁺.

Step 3: Compound 41b (80 mg, 263.41 µmol) and Compound 3e (88.80 mg, 289.75 µmol) were dissolved in ACN (10 mL) and then K₂CO₃ (91.02 mg, 658.53 µmol) was added. The system was heated to 70°C and reacted for 2 hours. The resultant was concentrated by spin-drying and separated by silica gel column chromatography (PE: EA=10:1 to 1:9) to give Compound 41c (115 mg, 76.1% yield) directly. MS m/z (ESI): 574.2[M+1]⁺.

Step 4: Compound 41c (25 mg, 43.58 µmol) was dissolved in methanol (5 mL), and then dioxane/hydrochloride (4 M, 0.5 mL) was added. The reaction was carried out at room temperature 25°C for 0.5 hour. It was concentrated by spin-drying, dissolved in methanol again and alkalized by adding triethylamine, and then spin-dried. The resultant was separated by preparative chromatography (alkaline) and freeze-dried to give Compound 41 (7.53 mg, 35.6% yield). MS m/z (ESI): 470.2[M+1] ⁺. ¹H NMR (400 MHz, DMSO-d6) δ 7.85 (s, 1H), 7.61 (s, 1H), 7.35 - 7.22 (m, 1H), 7.18 -7.11(m, 4H), 7.03 (s, 2H), 4.31 - 4.29 (m, 2H), 4.16 (t, J = 6.3 Hz, 2H), 3.94 (dd, J = 7.0, 4.1 Hz, 2H), 3.81 (s, 1H), 3.19 (q, J = 13.8 Hz, 2H), 3.06 (d, J = 15.6 Hz, 1H), 2.61 (d, J = 15.6 Hz, 1H), 2.13 - 2.06 (m, 2H), 1.81 - 1.42 (m, 3H), 1.12 - 1.01 (m, 1H).

### Example 42: Preparation of Compound 42

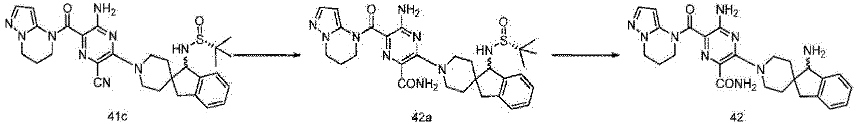

Step 1: Compound 41c (20 mg, 34.86 µmol) was dissolved in DMSO (3 mL) followed by the addition of K₂CO₃ (9.64 mg, 69.72 µmol) and H₂O₂ (0.2 mL, 30% purity), and the reaction was carried out at 15°C for 6 hours. The reaction system was quenched by adding saturated aqueous sodium thiosulphate and extracted by adding ethyl acetate, and the organic phases were combined, dried with anhydrous sodium sulfate and concentrated to give Compound 42a (15 mg). MS m/z (ESI): 592.2[M+1]⁺.

Step 2: Compound 42a (15 mg) was dissolved in methanol (5 mL), and then a solution of hydrochloric acid in dioxane (4 M, 0.2 mL) was added. The reaction was carried out at room temperature 25°C for 0.5 hour. It was concentrated by spin-drying, dissolved in methanol again and alkalized by adding triethylamine, and then spin-dried. The resultant was separated by preparative chromatography (alkaline) and freeze-dried to give Compound 42 (2.10 mg, 17% yield). MS m/z (ESI): 488.2[M+1]⁺.

### Example 43: Preparation of Compound 43

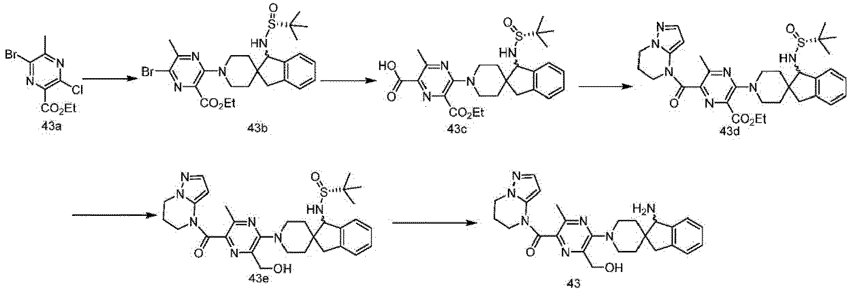

Step 1: Compound 43a (200 mg, 715.52 µmol) and Compound 3e (241.21 mg, 787.07 µmol) were dissolved in DMF (5 mL), and then K₂CO₃ (247.22 mg, 1.79 mmol) was added. The system was heated to 80°C and reacted for 12 hours. The resultant was concentrated by spin-drying and separated by silica gel column chromatography (petroleum ether: ethyl acetate=10:1 to 1:9) to give Compound 43b (270 mg, 68.7% yield). MS m/z (ESI):551.1[M+1]⁺.

Step 2: Compound 43b (200 mg, 363.95 µmol) was dissolved in water (5 mL) and dioxane (5 mL), followed by the addition of molybdenum hexacarbonyl (192.17 mg, 727.91 µmol), TEA (73.66 mg, 727.91 µmol, 101.53 µL), BINAP (45.32 mg, 72.79 µmol) and Pd(OAc)₂ (8.17 mg, 36.40 µmol), argon replacement was performed three times. The reaction system was microwave heated to 100°C and the reaction was carried out for 1 hour. The reaction was cooled to room temperature, and the reaction system was spin-dried and purified by silica gel column chromatography (dichloromethane: methanol=1:100 to 1:10) to give Compound 43c (145 mg, 77.41% yield). MS m/z (ESI):515.2[M+1]⁺.

Step 3: Compound 43c (145 mg, 281.75 µmol) and 4,5,6,7-tetrahydropyrazolo[1,5-A]pyrimidine (41.64 mg, 338.10 µmol) were dissolved in DCM (8 mL), followed by the addition of TEA (85.53 mg, 845.26 µmol, 117.89 µL), and then HATU ( 127.56 mg, 338.10 µmol) was added. The reaction was carried out at room temperature 15°C for 12 hours. The reaction system was diluted with 30 mL of dichloromethane, followed by adding 10 mL of water, and the aqueous phase was extracted with dichloromethane. The organic phases were combined, dried with anhydrous sodium sulfate, concentrated and purified by silica gel column chromatography (dichloromethane: methanol=1:100 to 1:10) to give Compound 43d (145 mg, 83 % yield). MS m/z (ESI):620.3[M+1]⁺.

Step 4: Compound 43d (35 mg, 56.47 µmol) was dissolved in anhydrous ethanol (1 mL) followed by the addition of THF (2 mL), and then NaBH₄ (6.41 mg, 169.42 µmol) and CaCl₂ (6.27 mg, 56.47 µmol) were added to the mixture in ice bath condition. The reaction was carried out at 0°C for 15 min, and a reaction system of Compound 43e was obtained. MS m/z (ESI). 578.2[M+1]⁺.

Step 5: The above-mentioned reaction system of Compound 43e was directly added to HCl-dioxane (4 M, 0.2 mL) and the pH was adjusted to about 2-3. The reaction system was directly spin-dried, dissolved in methanol again and alkalized by adding TEA, and then spin-dried. The resultant was separated by preparative chromatography (alkaline) to give Compound 43 (1.15 mg, 4.30% yield). MS m/z (ESI): 474.2[M+1]⁺.

### Example 44: Preparation of Compound 44

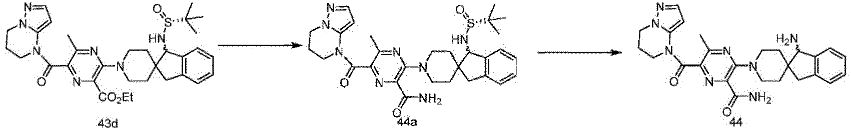

Step 1: Compound 43d (40 mg, 64.54 µmol) was dissolved in a solution of ammonia in methanol (7 M, 6 mL) and the reaction was carried out in a stainless steel tank at a temperature raised to 90°C for 7 hours. The reaction system was directly concentrated by spin-drying to give Compound 44a (35 mg, 22% yield). MS m/z (ESI):591.3 [M+1]⁺.

Step 2: Compound 44a (25 mg, 42.32 µmol) was dissolved in methanol (4 mL), and then a solution of hydrochloric acid in dioxane (4 M, 0.2 mL) was added. The reaction was carried out at room temperature 25°C for 0.5 hour. The reaction system was concentrated by spin-drying, dissolved in methanol again and alkalized by adding triethylamine, and then spin-dried. The resultant was separated by preparative chromatography (alkaline) and freeze-dried to give Compound 44 (3.50 mg, 17% yield). MS m/z (ESI):487.2 [M+1] ⁺. 1H NMR (400 MHz, DMSO-d6) δ 7.89 (s, 1H), 7.51 (s, 1H), 7.40 - 7.23 (m, 2H), 7.17 - 7.13 (m, 4H), 4.14 (t, J = 6.1 Hz, 2H), 3.98 (t, J = 10.9 Hz, 2H), 3.82 - 3.77 (m, 3H), 3.20 (q, J = 12.1 Hz, 2H), 3.04 (d, J = 15.7 Hz, 1H), 2.68 - 2.55 (m, 1H), 2.33 (s, 3H), 2.15 - 2.09 (m, 2H), 1.88 - 1.63 (m, 2H), 1.49 (d, J = 12.8 Hz, 1H), 1.10 (d, J = 13.1 Hz, 1H).

### Example 45: Preparation of Compound 45

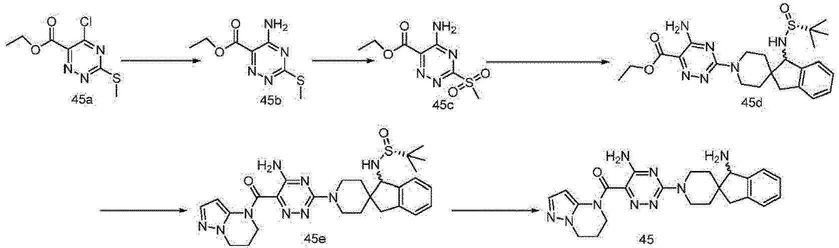

Step 1: Compound 45a (500 mg, 2.14 mmol) was dissolved in dioxane (10 mL), and then NH₄OH (2 mL, 28% purity) was added. The reaction was carried out at room temperature 15°C for 15 min. The reaction system was directly spin-dried to give Compound 45b (480 mg). MS m/z (ESI): 215.0 [M+1]⁺.

Step 2: Compound 45b (150 mg, 700.13 µmol) was dissolved in NMP (10 mL), and then m-CPBA (426.42 mg, 2.10 mmol, 85% purity) was added. The reaction was carried out at room temperature 15°C for 2 hours. The reaction system was not further processed and purified, and the reaction product Compound 45c in the NMP reaction system was used directly in the next step. MS m/z (ESI): 247.0 [M+1]⁺.

Step 3: DIPEA (393.64 mg, 3.05 mmol, 530.52 µL) was added to the reaction system from the previous step, followed by the addition of Compound 3e (186.68 mg, 609.15 µmol), and the reaction system was heated to 90°C and the reaction was carried out for 2 hours. The reaction liquid was added with saturated sodium bicarbonate solution and was diluted by adding ethyl acetate. The aqueous phase was extracted with ethyl acetate, and the organic phases were combined, washed twice with saturated sodium chloride solution, then dried with anhydrous sodium sulfate, concentrated, and purified by silica gel column chromatography (petroleum ether : ethyl acetate=10:1 to 1:10) to give Compound 45d (200 mg, 69.5% yield).MS m/z (ESI):473.2 [M+1]⁺.

Step 4: Compound 45d (85 mg, 179.86 µmol) and 4,5,6,7-tetrahydropyrazolo[1,5-A]pyrimidine (26.5 mg, 215.86 µmol) were dissolved in toluene (10 mL), and trimethylaluminum (1 M, 0.5 mL) was added under argon protection. The system was heated to 110°C and reacted for 3.5 hours under argon protection. After the reaction was completed, methanol was carefully added dropwise to quench the reaction until no bubble was formed, and then the reaction liquid was directly concentrated by spin-drying, and purified by column chromatography (dichloromethane : methanol=1:100 to 1:10) to give Compound 45e (45 mg, 45.5% yield). MS m/z (ESI): 550.3[M+1]⁺.

Step 5: Compound 45e (40 mg, 72.77 µmol) was dissolved in methanol (6 mL) and then a solution of hydrochloric acid in dioxane (4 M, 0.2 mL) was added. The reaction was carried out at room temperature 25°C for 0.5 hour. The reaction system was concentrated by spin-drying, dissolved in methanol again and alkalized by adding triethylamine, and then spin-dried. The resultant was separated by preparative chromatography (alkaline) and freeze-dried to give Compound 45 (19.73 mg, 60.8% yield). MS m/z (ESI):446.2 [M+1] ⁺. ¹H NMR (400 MHz, DMSO-d6) δ 7.35 (d, J = 2.0 Hz, 1H), 7.28 (dd, J = 6.5, 1.9 Hz, 1H), 7.19 - 7.10 (m, 3H), 6.36 (s, 1H), 4.53 (s, 2H), 4.14 (t, J = 6.1 Hz, 2H), 3.99 - 3.89 (m, 2H), 3.82 (s, 1H), 3.25 - 3.19 (m, 2H), 3.08 (d, J = 15.6 Hz, 1H), 2.62 (d, J = 15.6 Hz, 1H), 2.11 (p, J = 6.2 Hz, 2H), 1.78 - 1.43 (m, 3H), 1.07 (d, J = 13.4 Hz, 1H).

### Example 46: Preparation of Compound 46

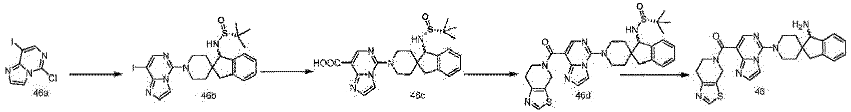

Step 1: Compound 46a (30 mg, 107.35 µmol) and Compound 3e (36.19 mg, 118.08 µmol) were dissolved in ACN (8 mL), and DIEA (27.75 mg, 214.70 µmol , 37.40 µL) was added. The system was heated to 90°C and reacted for 3 hours. The resultant was spin-dried and purified by silica gel column chromatography (petroleum ether : ethyl acetate=5:1 to 1: 9) to give Compound 46b (45 mg, 76.3% yield). m/z (ESI): 550.1 [M+1]⁺.

Step 2: Compound 46b (40 mg, 72.80 µmol) was dissolved in water (3 mL) and dioxane (3 mL), and then molybdenum hexacarbonyl (38.44 mg, 145.60 µmol), TEA (14.73 mg, 145.60 µmol, 20.31 µL), BINAP (9.07 mg, 14.56 µmol) and Pd(OAc)₂ (1.63 mg, 7.28 µmol) were added. Argon replacement was performed three times and the reaction system was microwave-heated to 90°C and the reaction was carried out for 1 hour. The reaction was cooled down to room temperature, then spin-dried and purified by silica gel column chromatography (dichloromethane : methanol=1:100 to 1:6) to give Compound 46c (25 mg, 73.5% yield). MS m/z (ESI):468.2 [M+1]⁺.

Step 3: Compound 46c (15 mg, 32.08 µmol) and 4,5,6,7-tetrahydrothiazolo[5,4-c]pyridine hydrochloride (5.40 mg, 38.50 µmol) were dissolved in DMF (4 mL), followed by the addition of TEA (6.49 mg, 64.16 µmol, 8.95 µL) and HATU (15.73 mg, 41.70 µmol), and the reaction was carried out at room temperature for 6 hours. The reaction system was added with ethyl acetate and water, and the aqueous phase was extracted with ethyl acetate. The combined organic phases were dried with anhydrous sodium sulfate, and concentrated to give Compound 46d (18 mg), which was used directly in the next step. MS m/z (ESI):590.2 [M+1]⁺.

Step 4: Compound 46d (15 mg, 25.43 µmol) was dissolved in methanol (5 mL) and then a solution of hydrochloric acid in dioxane (4 M, 0.3 mL) was added. The reaction was carried out at room temperature 25°C for 0.5 hour. After the reaction was completed, it was directly concentrated by spin-drying, dissolved in methanol again and alkalized by adding triethylamine, and then spin-dried. The resultant was separated by preparative chromatography (alkaline) and freeze-dried to give Compound 46 (3 mg, 22.8% yield). MS m/z (ESI):486.2 [M+1]⁺.

### Example 48: Preparation of Compound 48

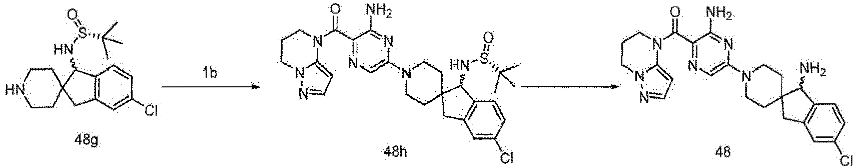

Compound 48 was prepared using Compound 48g as the raw material with reference to the methods and conditions of Step 5 and Step 6 in Example 2. MS m/z(ESI): 479 [M+1]⁺. ¹H NMR (400 MHz, DMSO-*d*₆): δ 7.59 (s, 1H), 7.30 - 7.28 (m, 2H), 7.23 - 7.19 (m, 2H), 6.72 (s, 2H), 6.25 (d, *J* = 2.0 Hz,1H), 4.29 - 4.25 (m, 2H), 4.11 (t, *J* = 6.1 Hz, 2H), 3.98 - 3.96 (m, 2H), 3.79 (s, 1H), 3.18 - 3.05 (m, 3H), 2.62 (d, *J* = 15.9 Hz, 1H), 2.10 - 2.07 (m, 2H), 1.72(td, *J* = 12.6, 4.2 Hz, 1H), 1.59 (td, J = 12.7, 4.2 Hz, 1H), 1.50 (d, J= 13.2 Hz, 1H), 1.05 (d, *J=* 12.9 Hz, 1H).

### Example 49: Preparation of Compound 49

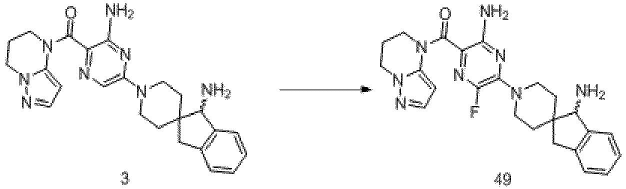

Compound 3 (40 mg, 89.98 µmol) was dissolved in acetonitrile (5 mL), to which Selectfluor^{®} Fluorine Reagent (63.75 mg, 179.96 µmol) was then added. The reaction was carried out at 0°C for 1 min with stirring. HCl-MeOH was spin-dried, and the resultant was purified by prep-HPLC (alkaline) to give the final product Compound 49 (1.18 mg, 2.6% yield). MS m/z (ESI): 463.0[M+1]⁺. ¹H NMR (400 MHz, DMSO-d6) δ 7.30 (s, 2H), 7.16 (d, *J* = 5.5 Hz, 3H), 6.84 (s, 2H), 6.25 (s, 1H), 4.24 (s, 2H), 4.12 (s, 2H), 3.98 (s, 2H), 3.74 (s, 1H), 3.07 (d, *J* = 15.4 Hz, 2H), 2.64 (d, *J* = 15.2 Hz, 2H), 1.98 (d, *J* = 7.7 Hz, 2H), 1.80 - 1.63 (m, 2H), 1.56 - 1.48 (m, 1H), 0.84 (s, 1H).

### Example 50 to Example 58

Compound 50 to Compound 52 were prepared with reference to the method of Example 2. Compound 53 was prepared using Compound 48g as the raw material with reference to the method of Example 2. Compound 54 was prepared using Compound 54e as the raw material with reference to the method of Example 2. Compound 55 was prepared with reference to the method of Example 2. Compound 56 was prepared using Compound 56h as the raw material with reference to the method of Example 2. Compound 57 was prepared using Compound 57f as the raw material with reference to the method of Example 2. Compound 58 was prepared using Compound 58c as the raw material with reference to the method of Example 2.

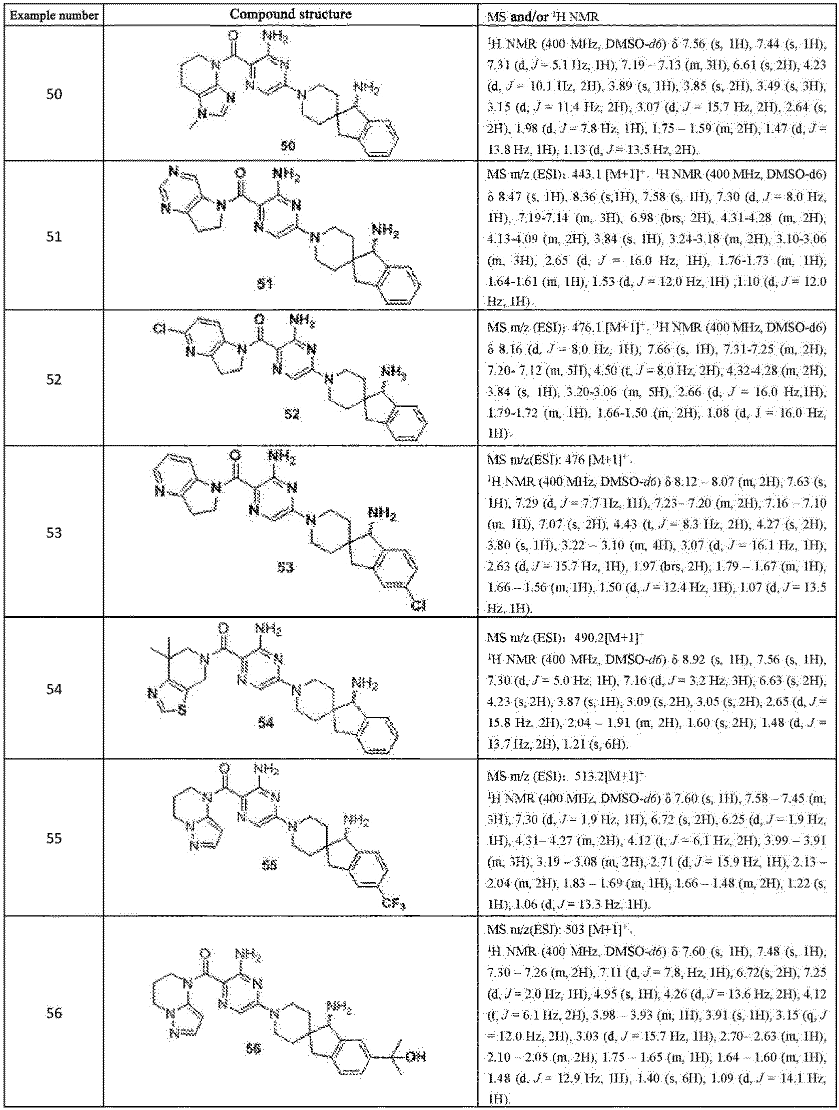

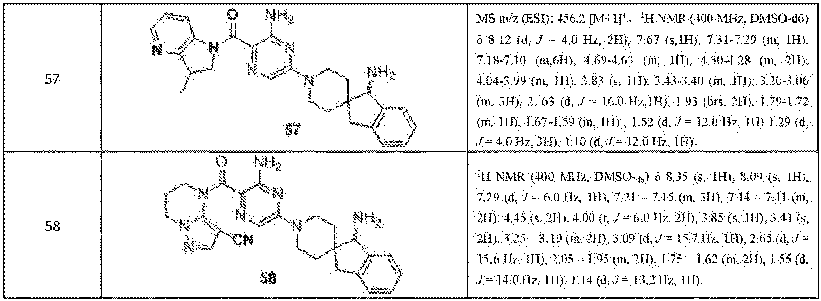

### Example 59: Preparation of Compound 59

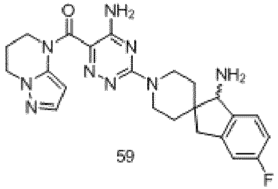

Compound 59 was prepared with reference to the method of Example 45. LC-MS: MS m/z(ESI): 464.2 [M+1]⁺.

### Example 60: Preparation of Compound 60

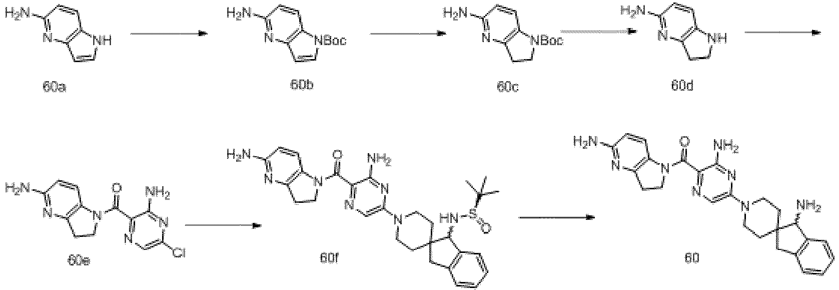

Step 1: A solution of Compound 60a (1 g, 7.52 mmol), triethylamine (1.52 g, 15.02 mmol), DMAP (275 mg, 2.25 mmol), and di-tert-butyl dicarbonate (233 mg, 2.3 mmol) in DCM (15 mL) was stirred at room temperature for 2 hours. The reaction system was concentrated, separated and purified by silica gel column chromatography (petroleum ether: ethyl acetate=5:1) to give Compound 60b (500 mg, yield: 28.5%). MS m/z (ESI): 234.1 [M+1]⁺.

Step 2: A solution of Compound 60b (500 mg, 2.14 mmol) and Pd/C (150 mg, 10%, wet) in ethanol (10 mL) was hydrogenated with hydrogen at 60 °C for 12 hours. The reaction system was filtered, concentrated, separated and purified by silica gel column chromatography (petroleum ether: ethyl acetate=5:1) to give Compound 60c (350 mg, yield: 69.4%). MS m/z (ESI): 236.1 [M+1]⁺.

Step 3: A solution of Compound 60c (300 mg, 1.28 mmol) in trifluoroacetic acid (2 mL) and dichloromethane (4 mL) was stirred at room temperature for 1 hour, and the reaction system was concentrated, separated and purified by silica gel column chromatography (dichloromethane: methanol=10:1) to give Compound 60d (150 mg, Yield: 87.0%). MS m/z (ESI): 136.1 [M +1]⁺.

Step 4: A solution of 3-amino-5-chloropyrazin-2-carboxylic acid (80 mg, 0.461 mmol), Compound 60d (74.76 mg, 0.533 mmol), triethylamine (233 mg, 2.3 mmol), and T₃P (880 mg, 1.38 mmol, 50% in THF) in THF (3 mL) was stirred at room temperature for 0.5 hour, and the reaction system was concentrated, separated and purified by silica gel column chromatography (dichloromethane: methanol=10:1) to obtain Compound 60e (120 mg, yield: 89.4%). MS m/z (ESI): 291.0[M+1]⁺.

Step 5: A reaction system of Compound 60e (60 mg, 0.206 mmol), Compound 3e (82.23 mg, 0.268 mmol) and potassium carbonate (85.58 mg, 0.619 mmol) in DMF (3 mL) was stirred at 70°C for 2 hours, and the reaction system was concentrated, separated and purified by silica gel column chromatography (dichloromethane : methanol=15:1) to give Compound 60f (90 mg, yield: 77.7%). MS m/z (ESI): 561.3 [M+1]⁺.

Step 6: A solution of Compound 60f (90 mg, 0.160 mmol) and HCl/dioxane (2 mL, 4M) in methanol (2 mL) was stirred at room temperature for 2 hours. The reaction system was concentrated, separated and purified by preparative chromatography to give Compound 60 (26 mg, yield: 35.6%). MS m/z (ESI):457.2 [M+1]⁺. ¹H NMR (400 MHz, DMSO-d6) δ 7.95 (s, 1H), 7.58 (s, 1H), 7.28 (d, *J* = 6.5 Hz, 1H), 7.18-7.11 (m, 3H), 6.96 (brs, 2H), 6.21 (d, *J=* 8.0 Hz,1H), 5.68 (brs, 2H), 4.34-4.21 (m, 4H), 3.81 (s, 1H), 3.15-3.03 (m, 3H), 2.94 (t, *J* = 8.4Hz, 2H), 2.62 (d, *J* = 15.7 Hz, 1H), 2.06 (brs, 2H), 1.78-1.72 (m, 1H), 1.66-1.63 (m, 1H) ,1.48 *(d, J=* 12.0 Hz, 1H), 1.06 (d, *J* = 12.0 Hz, 1H).

### Example 61: Preparation of Compound 61

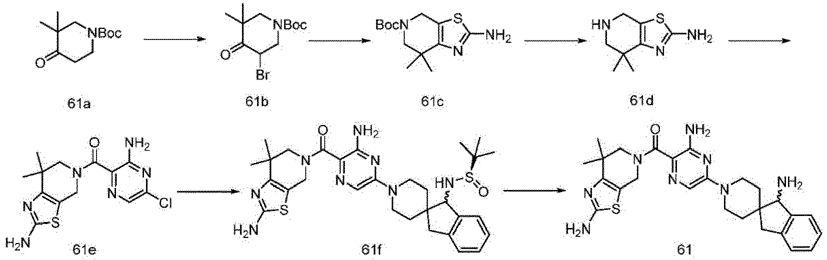

Step 1: Tert-butyl 3,3-dimethyl-4-oxopiperidin-1-carboxylate 61a (1.8 g, 7.92 mmol) was dissolved in CHCl₃ (15 mL), and the solution was cooled to 0°C, to which Br₂ (6.33 g, 39.60 mmol) was added dropwise. The reaction system was stirred at 25°C for 1.0 hour. The resultant was directly concentrated by spin-drying to give Compound 61b (1.8 g, 74.2% yield). MS m/z (ESI): 206.0 [M-100+H]⁺.

Step 2: Compound 61b (1.8 g, 5.88 mmol) and thiourea (1.34 g, 17.64 mmol) were dissolved in EtOH (15 mL). The reaction system was stirred at 80°C for 3 hours. After the reaction was completed, it was quenched with 40 mL of aqueous solution, and 60 mL of ethyl acetate was added. The resultant was filtrated through diatomaceous earth and the filtrate was extracted 3 times with 50 mL of ethyl acetate, and the combined organic phase was washed with 40 mL of saturated saline and dried with anhydrous sodium sulfate. The solvent was spin-dried under reduced pressure. The sample was mixed with silica gel and purified by silica gel column chromatography (petroleum ether : ethyl acetate=4:1) to give Compound 61c (600 mg, 36.0% yield). MS m/z (ESI): 284.0[M+1]⁺.

Step 3: Compound 61c (600 mg, 2.12 mmol) was dissolved in MeOH (5 mL), to which HCl in MeOH (1 mL) was added. The reaction system was stirred at 25°C for 1.0 hour. After the reaction was completed, it was quenched with 30 mL of aqueous solution, and the reaction system was extracted 3 times with 60 mL of ethyl acetate. The organic phase was washed with 30 mL of saturated saline and dried with anhydrous sodium sulfate, and the solvent was spin-dried under reduced pressure. The sample was mixed with silica gel and purified by silica gel column chromatography (dichloromethane : methanol=10:1) to give Compound 61d (320 mg, 82.5% yield). MS m/z (ESI): 184.1 [M+1]⁺.

Step 4: Compound 61d (105.60 mg, 576.18 µmol) and 3-amino-5-chloropyrazin-2-carboxylic acid (100 mg,0.58 mmol) were dissolved in ultra-dry DMF (5 mL), and then HATU (326 mg,0.86 mmol) and TEA (174 mg,1.72 mmol) were added. The mixture was stirred at room temperature for 1 hour. The reaction was monitored with LC-MS until the raw material was consumed up. Then 50 mL of water was added, and the resultant was extracted with ethyl acetate (30 mL * 3). The organic phase was washed with water (50 mL * 2) and saturated saline (50 mL), respectively, and the organic phase was added with anhydrous sodium sulfate (5 g) and stirred for 5 min, filtrated to give filtrate, which was concentrated under reduced pressure to give Compound 61e (123 mg). MS m/z (ESI): 339.1 [M +1]⁺.

Step 5: Compound 61e (123 mg, 363 µmol) and Compound 3e (111 mg, 363 µmol) were dissolved in DMF (3 mL), to which potassium carbonate (150 mg, 1089 µmol) was added. The reaction system was stirred at 80°C for 16 hours. After the reaction was completed, DMF was evaporated under reduced pressure, and dichloromethane was added. The sample was mixed with silica gel and purified by silica gel column chromatography (dichloromethane : methanol=85%:15%) to give Compound 61f (61 mg, 17.4% yield). MS m/z (ESI): 609.3 [M+1]⁺.

Step 6: Compound 61f (61 mg, 100 µmol) was dissolved in methanol (5 mL), to which a solution of HCl in dioxane (4 M, 1 mL) was added. The reaction system was stirred for 0.5 hour at 20°C. The solvent was spin-dried, and the resultant was separated and purified by preparative liquid chromatography (alkaline) to give the final product Compound 61 (9.0 mg, 17.8% yield). MS m/z (ESI): 505.2[M+1]⁺. 'H-NMR(400 MHz, DMSO-d6): δ 7.53 (s, 1H), 7.28 (d, *J* = 6.0 Hz, 1H), 7.18 - 7.11 (m, 3H), 6.81 (brs, 2H), 6.56 (brs, 2H), 4.64 (brs, 2H), 4.24 - 4.18(m, 2H), 3.81 (s, 1H), 3.68 (s, 2H) 3.17 - 3.09 (m, 2H), 3.05 (d, *J =* 15.6 Hz, 1H), 2.60 (d, *J =* 15.7 Hz, 1H), 2.05 - 1.76 (m, 1H), 1.73 (td, *J* = 12.9, 4.3 Hz, 1H), 1.60 (td, *J =* 12.9, 4.3 Hz,, 1H), 1.48 (d, *J* = 12.9 Hz, 1H), 1.22 - 1.09 (m, 8H).

### Example 62 Preparation of Compound 62

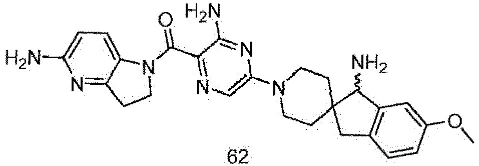

Compound 62 was prepared with reference to Example 60. MS m/z (ESI): 487.3[M+1]⁺. ¹H-NMR(400 MHz, DMSO-d6): δ 7.95 (s, 1H), 7.57 (s, 1H), 7.06 (d, *J =* 8.2 Hz, 1H), 6.95 (s, 2H), 6.87 (d, J = 2.1 Hz, 1H), 6.69 (dd, *J=* 8.1, 2.4 Hz, 1H), 6.21 (d, *J* = 8.7 Hz, 1H), 5.67 (s, 2H), 4.34 - 4.24 (m, 4H), 3.78 (s, 1H), 3.71 (s, 3H), 3.16 - 3.06 (m, 2H), 3.00 -2.92 (m, 3H), 2.52 (d, *J =* 12.3, 2H), 2.12 (s, 1H), 1.76 - 1.71 (m, 1H), 1.60 - 1.57 (m, 1H), 1.48 (d, *J* = 12.7 Hz, 1H), 1.07 (d, *J* = 13.5 Hz, 1H).

### Example 63: Preparation of Compound 63

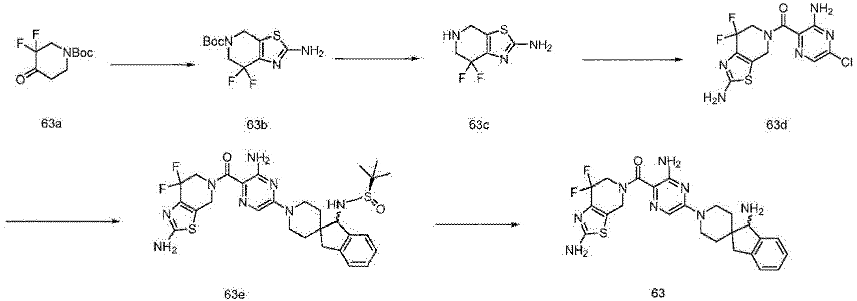

Step 1: Compound 63a (2.35 g, 10 mmol), cyanamide (840 mg, 20 mmol) and sulfur powder (640 mg, 20 mmol) were dissolved in pyridine (15 mL). The reaction was stirred at 130°C for 2 hours. After the reaction was completed, it was cooled to room temperature, 60 mL of ethyl acetate was added, the resultant was filtered through diatomaceous earth, washed 3 times with 50 mL of ethyl acetate, and the solvent was spin-dried under reduced pressure. Compound 63b (3 g, 100% yield) was obtained. MS m/z (ESI): 292.1[M+1]⁺.

Step 2: Compound 63b (3 g, 10.3 mmol) was dissolved in MeOH (40 mL), to which HCl in dioxane (25 mL) was added. The reaction system was stirred at 25°C for 2.0 hours. After the reaction was completed, the solvent was spin-dried under reduced pressure. The resultant was alkalized with DIPEA, separated and purified by silica gel column chromatography (20 g, 1-12% MeOH/DCM) to give Compound 63c (2.5 g, 100% yield). MS m/z (ESI):192.0 [M+1]⁺.

Step 3: 3-Amino-5-chloropyrazin-2-carboxylic acid (173 mg, 1 mmol) and Compound 63c (573 mg, 3 mmol) were dissolved in ultra-dry DMF (10 mL), and then HATU (570 mg, 1.5 mmol) and TEA (131 mg, 1.3 mmol) were added. The mixture was stirred for 1 hour at room temperature, and the reaction was monitored by LC-MS until the raw material was consumed up. Then 50 mL of water was added, and the resultant was extracted with ethyl acetate (30 mL*3). The organic phase was washed with water (50 mL*2) and saturated saline (50 mL), and the organic phase was added with anhydrous sodium sulfate (5 g) and stirred for 5 min, filtered to give filtrate, which was concentrated under reduced pressure to give Compound 63d (303 mg, 87.57% yield). MS m/z (ESI): 347.0 [M+1]⁺.

Step 4: Compound 63d (70 mg, 0.2 mmol) and Compound 3e (73 mg, 0.24 mmol) were dissolved in ultra-dry DMF (5 mL), and K₂CO₃ (83 mg, 0.6 mmol) was added. The mixture was stirred for 3 hours at 70°C. The resultant was concentrated under reduced pressure, separated and purified by silica gel column chromatography (4 g, 1-10% MeOH/DCM) to give Compound 63e (59mg, 47.9% yield). MS m/z(ESI): 617.2 [M+1]⁺.

Step 5: Compound 63e (59 mg, 0.096 mmol) was dissolved in MeOH (3 mL), and a solution of hydrochloric acid in dioxane (4 M, 0.3 mL) was added to the reaction system. The reaction system was stirred at room temperature (24°C) for 20 min. The resultant was separated and purified by preparative liquid chromatography to give the final product Compound 63 (21 mg, 42.85% yield). MS m/z(ESI): 513.2 [M+1]⁺. ¹H-NMR(400 MHz, DMSO-d6): δ 7.58 (s, 1H), 7.28 (d, *J =* 6.0 Hz, 1H), 7.22 - 7.09 (m, 5H), 6.77 (s, 2H), 4.81 (s, 2H), 4.45 (s, 2H),4.28 - 4.22 (m, 2H), 3.82 (s, 1H), 3.15 - 3.03 (m, 3H), 2.61 (d, *J =* 15.6 Hz, 1H), 1.79 - 1.70 (m, 3H), 1.66 - 1.57 (m, 1H), 1.49 (d, *J =* 12.5 Hz, 1H), 1.07 (d, *J* = 13.3 Hz, 1H).

### Example 64 Preparation of Compound 64

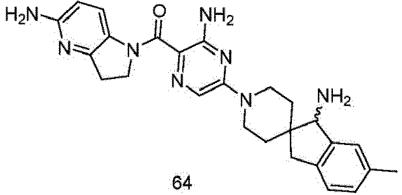

Compound 64 was prepared with reference to Example 60. MS m/z (ESI): 471.3[M+1]⁺. ¹H NMR (400 MHz, DMSO-d6) δ 7.95 (s, 1H), 7.57 (s, 1H), 7.10 (s, 1H), 7.05 (d, J = 7.6 Hz, 1H), 6.96 - 6.94 (m, 3H), 6.21 (d, J = 8.7 Hz, 1H), 5.67 (s, 2H), 4.35 - 4.22(m, 4H), 3.80 (s, 1H), 3.13 (q, J = 13.4 Hz, 2H), 3.03 - 2.89 (m, 3H), 2.66 - 2.64 (m, 1H), 2.56 (d, J = 15.5 Hz, 1H), 2.32 - 2.30 (m, 1H), 2.27 (s, 3H), 1.72 (td, J = 12.5, 4.1 Hz, 1H), 1.60 (td, J = 10.6, 4.0 Hz, 1H), 1.47 (d, J = 13.2 Hz, 1H), 1.09 (d, J = 13.3 Hz, 1H).

### Example 65: Preparation of Compound 65

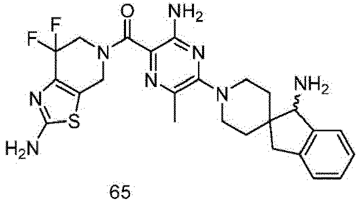

Compound 65 was prepared with reference to Example 63. MS m/z(ESI): 527.2 [M+1]⁺. ¹H NMR (400 MHz, DMSO-d6): δ 7.29 (d, *J* = 6.2 Hz, 1H), 7.27 - 7.00 (m, 3H), 6.51 (s, 2H), 4.80 (s, 2H), 4.33 (s, 4H), 3.84 (s, 2H), 3.68 (t, *J* = 13.3 Hz, 2H), 3.07 - 2.94 (m, 3H), 2.61 - 2.51 (m, 2H), 2.31 (s, 3H), 1.97 - 1.82 (m, 1H), 1.79 - 1.70 (m, 1H), 1.51 (d, *J* = 12.6 Hz, 1H), 1.12 (d, *J* = 14.8 Hz, 1H).

### Example 66: Preparation of Compound 66

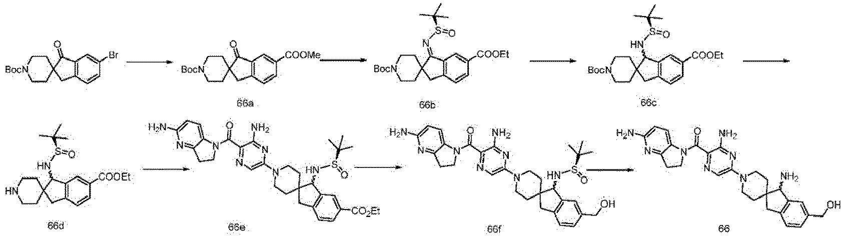

Step 1: Under N₂ protection, tert-butyl 6-bromo-1-oxo-1,3-dihydrospiro[indene-2,4'-piperidin]-1'-carboxylate (1.35 g, 3.55 mmol), Pd(OAc)₂ (159.40 mg, 710.01 µmol), BINAP (442.10 mg, 710.01 µmol),TEA (1.08 g, 10.65 mmol, 1.49 mL) and molybdenum hexacarbonyl (1.87 g, 7.10 mmol) were dissolved in MeOH (10 mL). The reaction system was microwave stirred at 90°C for 1.0 hour. After the reaction was completed, it was quenched with 40 mL of aqueous solution, and then 60 mL of ethyl acetate was added. The resultant was filtrated through diatomaceous earth, the filtrate was extracted 3 times with 50 mL of ethyl acetate, and the combined organic phase was washed with 40 mL of saturated saline, dried with anhydrous sodium sulfate, and spin-dried under reduced pressure. The resultant was purified by silica gel column chromatography (petroleum ether : ethyl acetate=4:1) to give Compound 66a (850 mg, 66.62% yield). MS m/z (ESI): 304.0 [M-56+1]⁺.

Step 2: Compound 66a (850 mg, 2.36 mmol) and R-(+)-tert-butylsulfinamide (572.32 mg, 4.73 mmol) were dissolved in Ti(EtO)₄ (10 mL). The reaction was carried out at 90°C for 2.0 hours with stirring. After the reaction was completed, it was quenched with 30 mL of aqueous solution, and the reaction system was extracted 3 times with 60 mL of ethyl acetate. The organic phase was washed one time with 30 mL of saturated saline, dried with anhydrous sodium sulfate, and spin-dried under reduced pressure. The resultant was purified by silica gel column chromatography (petroleum ether : ethyl acetate=10:1) to give Compound 66b (530 mg, 47.02% yield). MS m/z (ESI):377 [M-100+H]⁺.

Step 3: Compound 66b (530 mg, 1.11 mmol) was dissolved in THF (10 mL), to which a solution of DIBAL-H in hexane (2.2 mL) was added at -78°C. The reaction was carried out at -78°C for 2.0 hours with stirring. After the reaction was completed, it was quenched with 30 mL of aqueous solution, and the reaction system was extracted 3 times with 60 mL of ethyl acetate. The organic phase was washed with 30 mL of saturated saline, dried with anhydrous sodium sulfate, and spin-dried under reduced pressure. The resultant was purified by silica gel column chromatography (petroleum ether : ethyl acetate=1:1) to give Compound 66c (420 mg, 78.91% yield). MS m/z (ESI): 379 [M-100+H]⁺.

Step 4: Compound 66c (530 mg, 1.11 mmol) was dissolved in TFA (1.00 mL) and DCM (5.00 mL). The reaction was carried out at 25°C for 1.0 hour with stirring. After the reaction was completed, the solvent was spin-dried directly to give Compound 66d (200 mg, 47.72% yield). MS mS m/z (ESI): 379[M+1]⁺.

Step 5: Compound 66d (120 mg, 412.79 µmol) and Compound 60e (203.13 mg, 536.62 µmol) were dissolved in DMF (10 mL), to which K₂CO₃ (596.54 mg, 4.32 mmol) was added. The reaction was carried out at 80°C for 16 hours with stirring. After the reaction was completed, it was spin-dried under reduced pressure and purified by silica gel column chromatography (dichloromethane : methanol=85%:15%) to give Compound 66e (85 mg, 32.54% yield). MS m/z (ESI): 633[M+1]⁺.

Step 6: Compound 66e (85 mg, 134.33 µmol) was dissolved in THF (5 mL), to which a solution of DIBAL-H in hexane (1.0 mL) was added at 0°C. The reaction was carried out at 0°C for 2.0 hours with stirring. After the reaction was completed, it was quenched with 30 mL of aqueous solution, and the reaction system was extracted 3 times with 60 mL of ethyl acetate. The organic phase was washed with 30 mL of saturated saline, dried with anhydrous sodium sulfate, and spin-dried under reduced pressure. The resultant was purified by silica gel column chromatography (petroleum ether : ethyl acetate=1:1) to give Compound 66f (62 mg, 78.13% yield). MS m/z (ESI): 591 [M+1]⁺.

Step 7: Compound 66f (62 mg, 104.95 µmol) was dissolved in methanol (5 mL), to which a solution of HCl in dioxane (4 M, 1 mL) was added. The reaction was carried out for 0.5 hour at 20°C with stirring. The solvent was spin-dried and the resultant was separated and purified by preparative liquid chromatography (alkaline) to give the final product Compound 66 (4.20 mg, 8.22% yield). MS m/z (ESI): 487[M+1]⁺. ¹H-NMR(400 MHz, DMSO-d6): δ 7.95 (s, 1H), 7.58 (s, 1H), 7.25 (s, 1H), 7.12 - 7.16 (m, 2H), 6.95 (s, 2H), 6.21 (d, *J =* 8.8 Hz, 1H), 5.68 (s, 2H), 4.44 (d, *J =* 5.5 Hz, 2H), 4.32 - 4.25 (m, 4H), 3.79 (s, 1H), 3.15 -3.12 (m, 2H), 3.01 - 2.92 (m, 2H), 2.66 - 2.56 (m, 1H), 2.02 - 1.95 (m, 2H), 1.77 - 1.70 (m, 1H), 1.65 - 1.57 (m, 1H), 1.48 (d, *J =* 12.7 Hz, 1H), 1.07 (d, *J =* 12.3 Hz, 1H).

### Example 67: Preparation of Compound 67

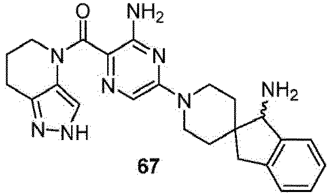

Compound 67 was prepared with reference to Example 2. MS m/z(ESI): 445 [M+1]⁺. ¹H NMR (400 MHz, DMSO-d6) δ 12.34 (s, 1H), 7.98 (s, 1H), 7.56 (s, 1H), 7.28 (d, *J=* 6.1 Hz, 1H), 7.21 - 7.09 (m, 3H), 6.55 (s, 2H), 4.26 - 4.19 (m, 2H), 3.91 (s, 2H), 3.82 (s, 1H), 3.20 - 3.09 (m, 2H), 3.05 (d, *J* = 15.6 Hz, 1H), 2.67 (t, *J* = 6.4 Hz, 2H), 2.61 (d, *J* = 15.7 Hz, 1H), 1.89 - 1.83 (m, 2H), 1.73 (td, *J* = 12.4, 4.0 Hz, 1H), 1.66 - 1.57 (td, *J =* 12.6, 4.3 Hz, 1H), 1.48 (d, *J =* 13.0 Hz, 1H), 1.08 (d, *J* = 12.9 Hz, 1H).

### Example 68: Preparation of Compound 68

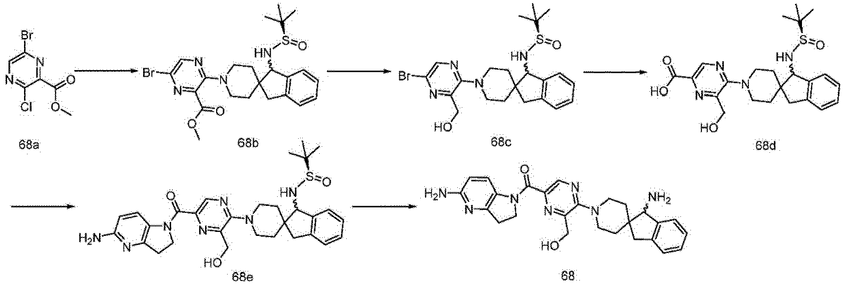

Step 1: Compound 68a (0.9 g, 3.58 mmol) and Compound 3e (1.10 g, 3.58 mmol) were dissolved in acetonitrile (15 mL), and then K₂CO₃ (1.24 g, 8.95 mmol) was added. The temperature of the reaction was raised to 90°C under an oil bath and carried out for 12 hours. The reaction system was concentrated, separated and purified by silica gel column chromatography (PE: EA=10:1 to 3:1) to give 1.2 g of Compound 68b (64.3%yield). MS m/z (ESI): 521.2[M+1]⁺.

Step 2: Compound 68b (1.2 g, 2.30 mmol) was dissolved in THF (40 mL) and the temperature of the reaction was decreased to 0°C, to which LiAlH₄ (174.68 mg, 4.60 mmol, 1.03 mL) was added batchwise. The reaction was carried out at 0°C for 0.5 hour, and the raw materials were consumed up. The reaction system was added with 0.2 mL of water, and then added with 0.2 mL of 3 M aqueous sodium hydroxide. The mixture was stirred, and added with 0.3 mL of water and excess sodium sulfate, and the resultant was then stirred for 15 min, and then filtered through diatomaceous earth. The filtrate was spin-dried, separated and purified by silica gel column chromatography (PE: EA=10:1 to 1:2) to give 300 mg of Compound 68c (26.4% yield). MS m/z (ESI):493.1[M+1]⁺.

Step 3: Compound 68c (300 mg, 607.95 µmol) was dissolved in water (7 mL) and 1,4-dioxane (7 mL), and then molybdenum hexacarbonyl (321.00 mg, 1.22 mmol), triethylamine (123.04 mg, 1.22 mmol, 169.59 µL), 1,1'-binaphthyl-2,2'-bisdiphenylphosphine ( 75.71 mg, 121.59 µmol) and Pd(OAc)₂ (13.65 mg, 60.80 µmol) was added. Argon replacement was performed three times, and the reaction system was microwave heated to 100°C and reacted for 1 hour. The reaction system was cooled to room temperature, then spin-dried, and separated by silica gel column chromatography (DCM: MeOH=1:100 to 1 :5) to give 150 mg of Compound 68d (53.5% yield). MS m/z (ESI): 459.2[M+1]⁺.

Step 4: Compound 68d (80 mg, 174.45 µmol), Compound 60d (32.94 mg, 191.90 µmol, HCl) were dissolved in DMF (4 mL), followed by the addition of N,N-diethylethylamine (52.96 mg, 523.36 µmol, 73.00 µL), and finally [dimethylamino-(1-formyl-3H-pyrazolo[3,4-b]pyridin-1-bromo-3-yl)methylene]-dimethylammonium hexafluorophosphate (85.56 mg, 226.79 µmol) was added. The reaction was carried out at room temperature for 1 hour. The reaction system was spin-dried and separated by silica gel column chromatography (DCM: MeOH=10:1) to give 40 mg of Compound 68e (39.8% yield). MS m/z (ESI). 576.3[M+1]⁺.

Step 5: Compound 68e (35.00 mg, 60.79 µmol) was dissolved in Methanol (8 mL), and then a solution of hydrochloric acid in dioxane (4 M, 0.2 mL) was added. The reaction was carried out at 15°C for 1 hour, and 8.6 mg of Compound 68 (29.8% yield) was obtained after purification. MS m/z (ESI): 472.2[M+1]⁺.

### Example 69: Preparation of Compound 69

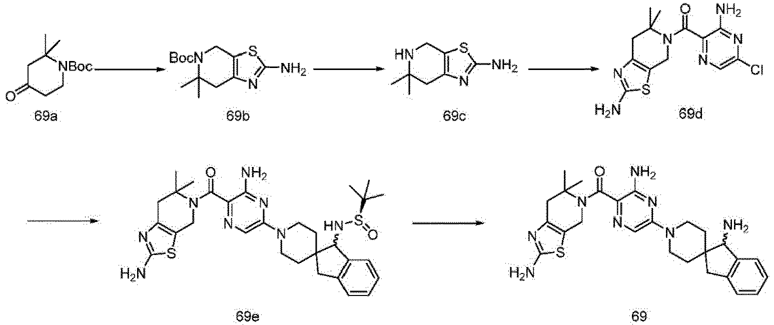

Step 1: Tert-butyl 2,2-dimethyl-4-oxopiperidin-1-carboxylate 69a (800 mg, 3.52 mmol), sulfur powder (239.90 mg, 7.04 mmol) and cyanamide (295.93 mg, 7.04 mmol) were dissolved in pyridine (15 mL). The reaction was carried out at 130°C for 1.0 hour with stirring. After the reaction was completed, it was quenched with 40 mL of aqueous solution, and 60 mL of ethyl acetate was added. The resultant was filtrated through diatomaceous earth and the filtrate was extracted 3 times with ethyl acetate 50 mL, and the combined organic phase was washed with 40 mL of saturated saline, and dried with anhydrous sodium sulfate. The solvent was spin-dried under reduced pressure. The resultant was purified by silica gel column chromatography (petroleum ether : ethyl acetate=4:1) to give Compound 69b (432 mg, 43.31% yield). MS m/z (ESI): 284.1[M+1]⁺.

Step 2: Compound 69b (200 mg, 705.74 mmol) was dissolved in MeOH (5 mL), to which a solution of HCl in methanol (1 mL) was added. The reaction was carried out at 25°C for 1.0 hour with stirring. After the reaction was completed, it was quenched with 30 mL of aqueous solution, and the reaction system was extracted 3 times with 60 mL of ethyl acetate. The organic phase was washed with 30 mL of saturated saline and dried with anhydrous sodium sulfate, and the solvent was spin-dried under reduced pressure. The resultant was purified by silica gel column chromatography (dichloromethane : methanol=10:1) to give Compound 69c (85 mg, 65.7% yield). MS m/z (ESI):184.1 [M+1]⁺.

Step 3: Compound 69c (85 mg, 463 µmol) and 3-amino-5-chloropyrazin-2-carboxylic acid (80.49 mg, 463.79 µmol) were dissolved in ultra-dry DMF (5 mL), and then HATU (209.97 mg, 556.55 µmol) and TEA (56.32 mg, 0.56 mmol) were added. The mixture was stirred at room temperature for 1 hour, and the reaction was monitored by LC-MS until the raw material was consumed up. Then 50 mL of water was added, and the resultant was extracted with ethyl acetate (30 mL * 3). The organic phase were washed with water (50 mL * 2) and saturated saline (50 mL), respectively, and the organic phase was added with anhydrous sodium sulfate (5 g) and stirred for 5 min, filtrated to obtain filtrate, which was concentrated under reduced pressure to give Compound 69d (123 mg). MS m/z (ESI): 339.0 [M +1]⁺.

Step 4: Compound 69d (80.50 mg, 262.68 µmol) and Compound 3e (80.50 mg, 262.68 µmol) were dissolved in DMF (3 mL), to which potassium carbonate (108.92 mg, 788.04 µmol) was then added. The reaction was carried out at 80°C for 16 hours with stirring. After the reaction was completed, DMF was evaporated under reduced pressure, and dichloromethane was added. The sample was mixed with silica gel and purified by silica gel column chromatography (dichloromethane : methanol=85%:15%) to give Compound 69e (62 mg). MS m/z (ESI): 609.3 [M+1]⁺.

Step 5: Compound 69e (89 mg, 262.68 µmol) and Compound 3e (80.50 mg, 262.68 µmol) were dissolved in methanol (5 mL), and then, a solution of HCl in dioxane (4M, 1 mL) was added to it. The reaction was carried out at 20°C for 0.5 hour with stirring. The solvent was spin-dried and the resultant was separated and purified by preparative liquid chromatography (alkaline) to give the final product Compound 69 (5.99 mg, 11.49% yield). MS m/z (ESI): 505.2[M+1]⁺. 'H-NMR(400 MHz, DMSO-d6): δ 7.56 (s, 1H), 7.27 (d, *J* = 6.8 Hz, 1H), 7.17 - 7.13 (m, 3H), 6.72 - 6.70 (m, 4H), 4.40 (s, 2H), 4.25 - 4.18 (m, 2H), 3.81 (s, 1H), 3.19 - 3.03 (m, 3H), 2.65 - 2.59 (m, 3H), 2.01 - 1.96 (m, 3H), 1.76 - 1.68 (m, 1H), 1.65 - 1.58 (m, 1H), 1.44 (s, 6H), 1.08 (d, *J* = 13.8 Hz, 1H).

### Example 70: Preparation of Compound 70

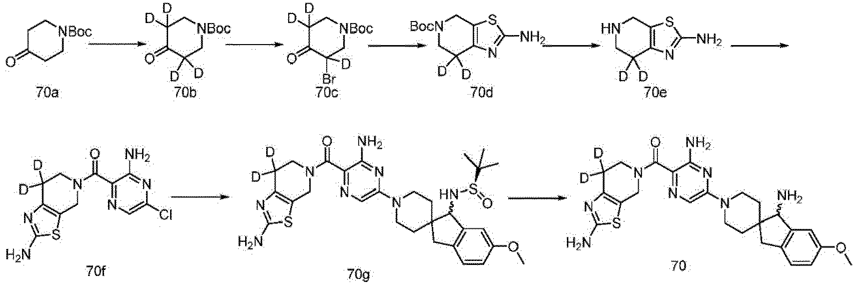

Step 1: Tert-butyl 4-oxopiperidin-1-carboxylate 70a (5 g, 25.09 mmol) was dissolved in CDCl₃ (50 mL), to which TBU (444.18 mg, 1.76 mmol) was added. The reaction was carried out at 25°C for 20 hours with stirring. After the reaction was completed, it was quenched with 40 mL of aqueous solution, and 60 mL of ethyl acetate was added. The resultant was filtrated through diatomaceous earth, and the filtrate was extracted 3 times with 50 mL of ethyl acetate. The organic phase was washed with 40 mL of saturated saline, and dried with anhydrous sodium sulfate, and the solvent was spin-dried under reduced pressure. The sample was mixed with silica gel and purified by silica gel column chromatography (petroleum ether : ethyl acetate=4:1) to give Compound 70b (4.8 g, 94.10% yield). MS m/z (ESI): 148[M-56+H]⁺.

Step 2: Compound 70b (4.6 g, 22.63 mmol) was dissolved in CHCl₃ (30 mL), and the solution was cooled to 0°C. Br₂ (3.62 g, 22.63 mmol) was added to it dropwise. The reaction was carried out at 25°C for 1.0 hour with stirring. The resultant was directly concentrated by spin-drying to give Compound 70c (5.1 g, 80.16% yield). MS m/z (ESI): 225 [M-56+H]⁺.

Step 3: Compound 70c (5.1 g, 18.14 mmol) and thiourea (4.14 g, 54.42 mmol) were dissolved in EtOH (5 mL). The reaction was carried out at 80°C for 3 hours with stirring. After the reaction was completed, it was quenched with 40 mL of aqueous solution, and 60 mL of ethyl acetate was added. The resultant was filtrated through diatomaceous earth, and the filtrate was extracted 3 times with 50 mL of ethyl acetate, and the organic phase was washed with 40 mL of saturated saline, dried with anhydrous sodium sulfate. The solvent was spin-dried under reduced pressure. The sample was mixed with silica gel and purified by silica gel column chromatography (petroleum ether : ethyl acetate=4:1) to give Compound 70d (1.2 g, 25.71% yield). MS m/z (ESI): 258[M+1]⁺.

Step 4: Compound 70d (1.2 g, 4.66 mmol) was dissolved in MeOH (5 mL), to which a solution of HCl in MeOH (1 mL) was added. The reaction was carried out at 25°C for 1.0 hour with stirring. After the reaction was completed, it was quenched with 30 mL of aqueous solution, and the reaction system was extracted 3 times with 60 mL of ethyl acetate. The organic phase was washed with 30 mL of saturated saline and dried with anhydrous sodium sulfate, and the solvent was spin-dried under reduced pressure. The sample was mixed with silica gel and purified by silica gel column chromatography (dichloromethane : methanol=10:1) to give Compound 70e (610 mg, 83.2% yield). MS m/z (ESI):158[M+1]⁺.

Step 5: Compound 70e (510 mg, 3.24 mmol) and 3-amino-5-chloropyrazin-2-carboxylic acid (562.95 mg, 3.24 mmol) were dissolved in THF (30 mL), to which T₃P (2.06 g, 6.49 mmol) was then added. The mixture was stirred at room temperature for 1 hour. The reaction was monitored by LC-MS until the raw materials were consumed up. Then 50 mL of water was added and the resultant was extracted with ethyl acetate (30 mL*3). The organic phase was washed with water (50 mL*2) and saturated saline (50 mL), respectively, and the organic phase was added with anhydrous sodium sulfate (5 g) and stirred for 5 min, filtered to obtain filtrate, which was concentrated under reduced pressure to give Compound 70f (520 mg, 51.26% yield). MS m/z (ESI): 313 [M+H]⁺.

Step 6: Compound 70f (450 mg, 1.44 mmol) and Compound 3l (484.12 mg, 1.44 mmol) were dissolved in DMF (10 mL), to which K₂CO₃ (596.54 mg, 4.32 mmol) was then added. The reaction was carried out at 80°C for 16 hours with stirring. After the reaction was completed, DMF was evaporated under reduced pressure, and dichloromethane was added. The sample was mixed with silica gel and purified by silica gel column chromatography (dichloromethane : methanol=85%:15%) to give Compound 70 g (78 mg). MS m/z (ESI): 613.0 [M+1]⁺.

Step 7: Compound 70g (77 mg, 125.65 µmol) was dissolved in methanol (5 mL), and then, a solution of HCl in dioxane (4 M, 1 mL) was added to it. The reaction was carried out at 20°C for 0.5 hour with stirring. The solvent was spin-dried and the resultant was separated and purified by preparative liquid chromatography (alkaline) to give the final product Compound 70 (21.34 mg, 30.22% yield). MS m/z (ESI): 507.3 [M-1]⁻. ¹H-NMR(400 MHz, DMSO-d6): δ 7.54 (s, 1H), 7.06 (d, *J* = 8.2 Hz, 1H), 6.87 (d, *J* = 2.1 Hz, 1H), 6.76 (s, 2H), 6.68 (dd, *J* = 8.2, 2.4 Hz, 1H), 6.63 (s, 2H), 4.57 (s, 2H), 4.25 -4.18 (m, 2H), 3.81 (s, 2H), 3.77 (s, 1H), 3.71 (s, 3H), 3.14 -3.04 (m, 2H), 2.97 (d, *J =* 15.2 Hz, 1H), 2.54 (s, 1H),2.01 - 1.96 (m, 1H), 1.73 (td, *J* = 13.0, 4.3 Hz, 1H), 1.59 (td, *J* = 13.4, 4.9 Hz, 1H), 1.47 (d, *J* = 13.5 Hz, 1H), 1.06 (d, J= 13.7 Hz, 1H).

### Example 71: Preparation of Compound 71

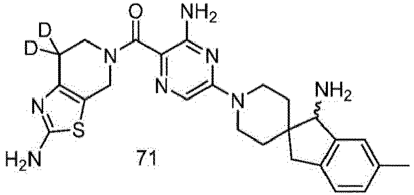

Compound 71 was prepared with reference to Example 70. MS m/z (ESI): 491.2 [M-1]⁻. ¹H-NMR(400 MHz, DMSO-d6): δ 7.54 (s, 1H), 7.08 (s, 1H), 7.04(d, *J =* 7.5 Hz, 1H), 6.93 (d, *J =* 7.6 Hz, 1H), 6.77 (s, 2H), 6.63 (s, 2H), 4.56 (s, 2H), 4.25 -4.18 (m, 2H), 3.81 (s, 2H), 3.76 (s, 1H), 3.15 - 3.06 (m, 2H), 2.99 (d, *J* = 15.5 Hz, 1H), 2.56 (s, 3H), 2.52 (s, 1H), 2.01 - 1.96 (m, 1H), 1.84 (s, 1H), 1.73 *(td, J=* 12.9, 4.1 Hz, 1H), 1.59 (td, *J* = 12.8, 3.6 Hz, 1H), 1.46 (d, *J* = 12.8 Hz, 1H), 1.06 (d*, J* = 13.7 Hz, 1H).

### Example 72: Preparation of Compound 72

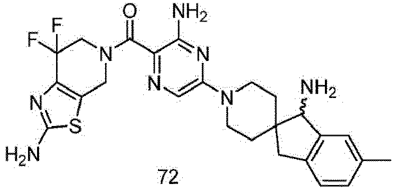

Compound 72 was prepared with reference to Example 63. MS m/z (ESI): 527 [M+1] ⁺. ¹H NMR (400 MHz, DMSO-d6) : δ 7.58 (s, 1H), 7.18 (s, 2H), 7.08 (s, 1H), 7.04 (d, J = 7.6 Hz, 1H), 6.93 (d, J = 7.6 Hz, 1H), 6.77 (s, 2H), 4.81 (s, 2H), 4.43(s, 2H), 4.25 - 4.22 (m, 2H), 3.77(s, 1H), 3.15 (q, J = 13.3 Hz, 2H), 2.99 (d, J = 15.5 Hz, 1H), 2.53 (d, J = 15.5 Hz, 1H), 2.26 (s, 3H), 1.72 (td, *J* = 12.5, 4.1 Hz, 1H), 1.59 (td, *J* = 12.7, 4.1Hz, 1H), 1.46 (d, *J* = 13.3 Hz, 1H), 1.06 (d, *J* = 13.4 Hz, 1H).

### Example 73: Preparation of Compound 73

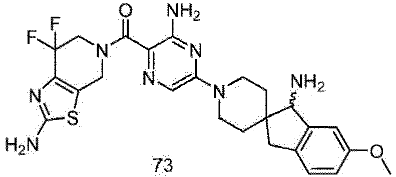

Compound 73 was prepared with reference to Example 63. MS m/z (ESI): 543[M+1] ⁺. ¹H NMR (400 MHz, DMSO-d6):δ 7.58 (s, 1H), 7.18 (s, 2H), 7.07 (d, J = 8.1 Hz, 1H), 6.88 (d, J = 2.5 Hz, 1H), 6.77 (s, 2H), 6.68 (dd, J = 8.1, 2.5 Hz, 1H), 4.81 (m, 2H), 4.40 (s, 2H), 4.26 - 4.22 (m, 2H), 3.77 (s, 1H), 3.71 (s, 3H), 3.21 - 3.07 (m, 2H), 2.99 (d, J = 15.3 Hz, 1H), 2.64 (s, 1H), 1.77 - 1.70 (m, 3H), 1.49 (td, J = 12.7, 4.2 Hz, 1H), 1.49 (d, J = 13.0 Hz, 1H), 1.06 (d, J = 13.3 Hz, 1H).

### Example 74: Preparation of Compound 74

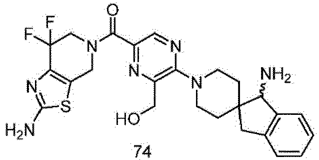

Compound 74 was prepared with reference to Example 68. MS m/z (ESI): 528 [M+1] . H NMR (400 MHz, DMSO-d6) δ 8.40 (s, 1H), 7.29 (d, J = 6.6 Hz, 1H), 7.21 (s, 2H), 7.18 - 7.11 (m, 3H), 5.39 (s, 1H), 4.90 - 4.78(m, 3H), 4.54 (d, J = 5.3 Hz, 2H), 4.31 (s, 2H), 3.94 - 3.83 (m, 3H), 3.19 (q, J = 13.5 Hz, 2H), 3.06 (d, J = 15.6 Hz, 1H), 2.62 (d, J = 15.6 Hz, 1H), 1.87 (td, J = 13.4, 4.3 Hz, 1H), 1.80 - 1.73 (m, 1H), 1.52 (d, J = 13.0 Hz, 1H), 1.12 (d, J = 13.2 Hz, 1H).

### Example 75: Preparation of Compound 75

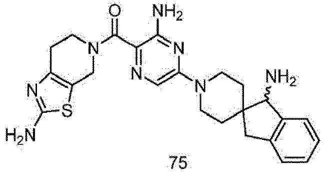

Compound 75 was prepared with reference to Example 2. MS m/z (ESI): 477.2[M+1] . ¹H NMR (400 MHz, DMSO-*d*₆) δ 7.56 (d, *J=* 4.3 Hz, 1H), 7.36 - 7.06 (m, 4H), 6.80 (brs, 2H), 6.66 (brs, 2H), 4.57 (brs, 2H), 4.27 - 4.10 (m, 3H), 3.83 - 3.77 (m, 3H), 3.33 -3.05(m, 4H), 2.82 - 2.49 (m, 3H), 1.77 - 1.71 (m, 1H), 1.66 - 1.58 (m, 1H), 1.49 (d,J= 13.3 Hz, 1H), 1.08 (d, *J=* 13.3 Hz, 1H).

### Example 114: Compound 114

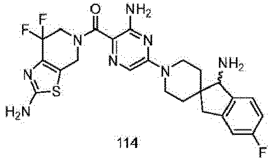

Compound 114 was prepared with reference to Example 63, with the difference that Compound 3e was replaced by Compound 3h. MS m/z (ESI):531.2 [M+1]⁺. ¹H NMR (400 MHz, DMSO-d6)δ 7.58 (s, 1H), 7.29 - 7.24(m, 1H), 7.18 (s, 2H), 7.00 -6.93 (m, 2H), 6.77 (s, 2H), 4.81 (s, 2H), 4.42 (s, 2H), 4.27 - 4.22 (m, 2H), 3.78 (s, 1H), 3.21 - 3.12 (m, 2H), 3.05 (d, J = 16.0 Hz, 1H), 2.62 (d, J = 15.9 Hz, 1H), 1.80 (s, 2H), 1.75 - 1.59 (m, 2H), 1.48 (d, J = 12.7 Hz, 1H), 1.09 (d, J = 12.7 Hz, 1H).

The compounds in the following Examples were synthesized with reference to the methods described above.

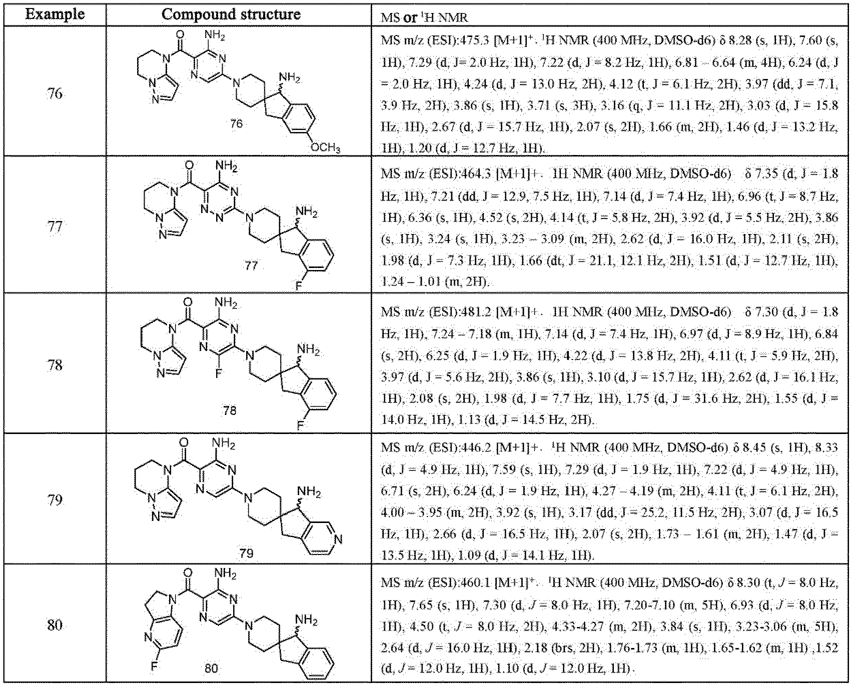

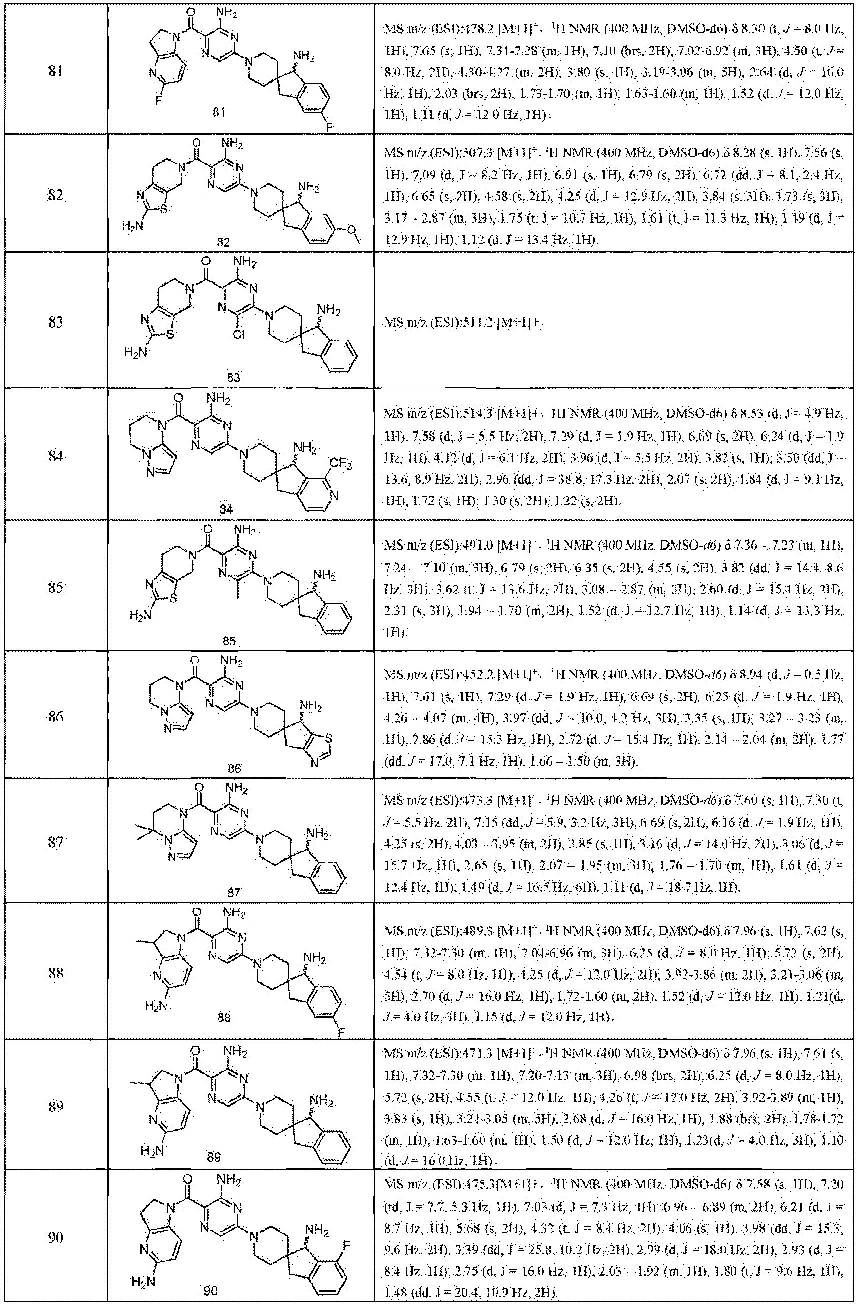

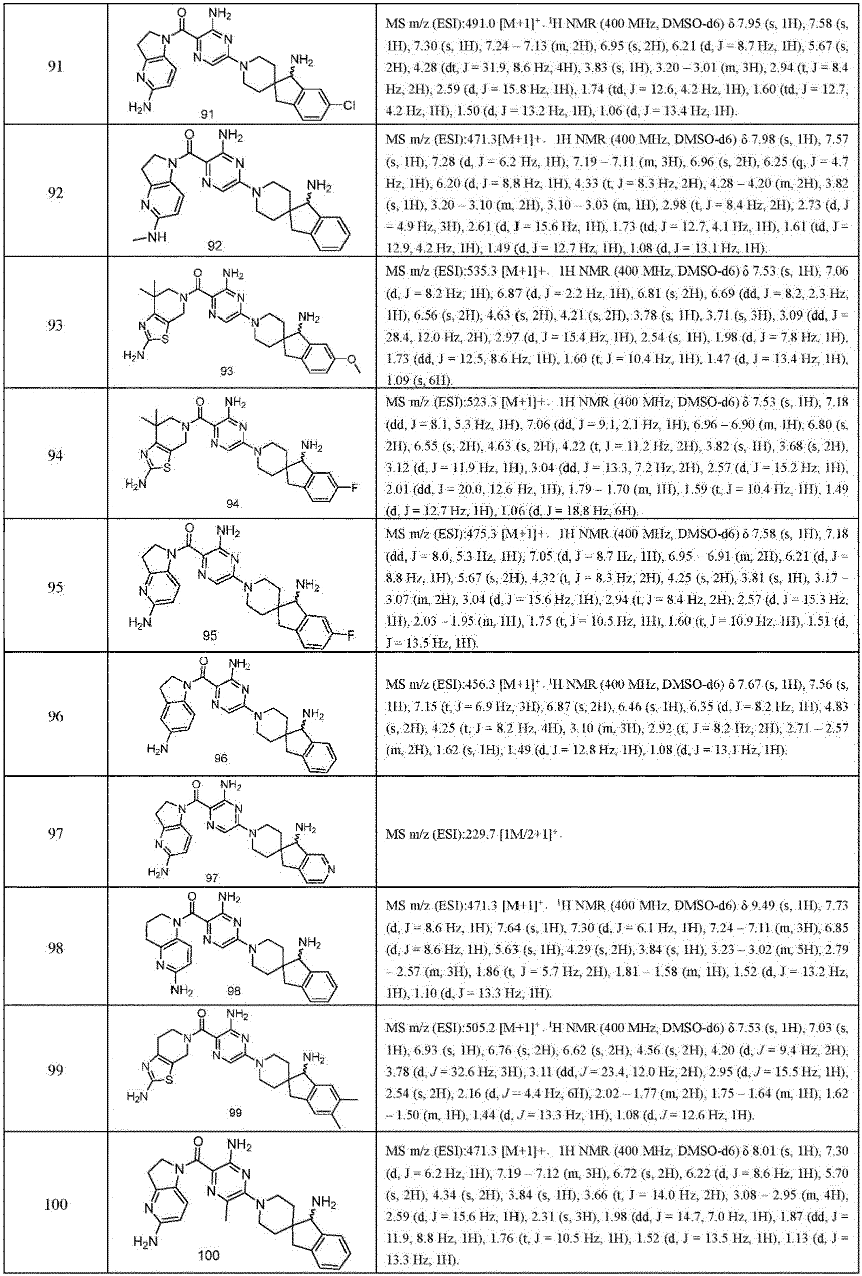

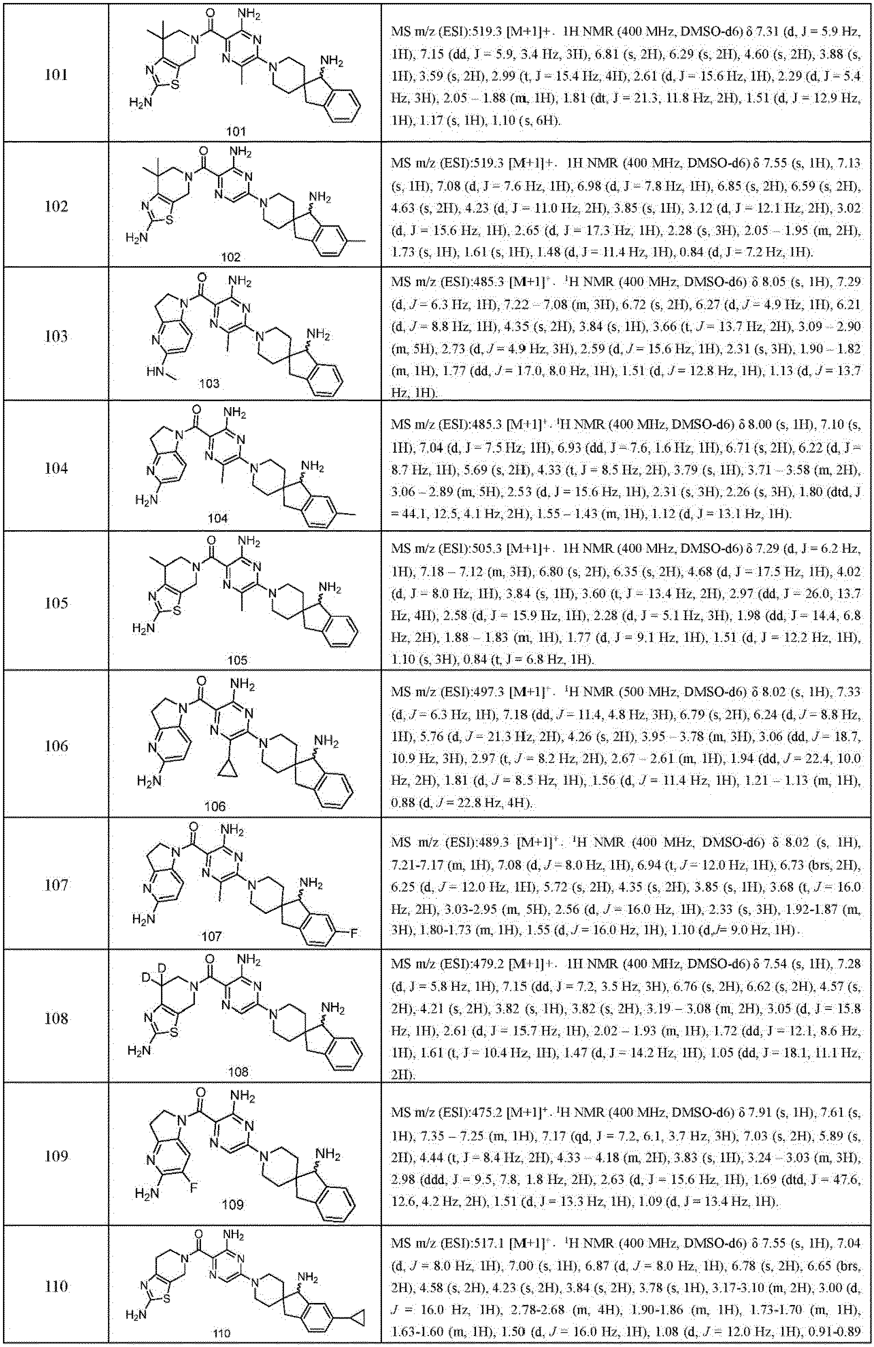

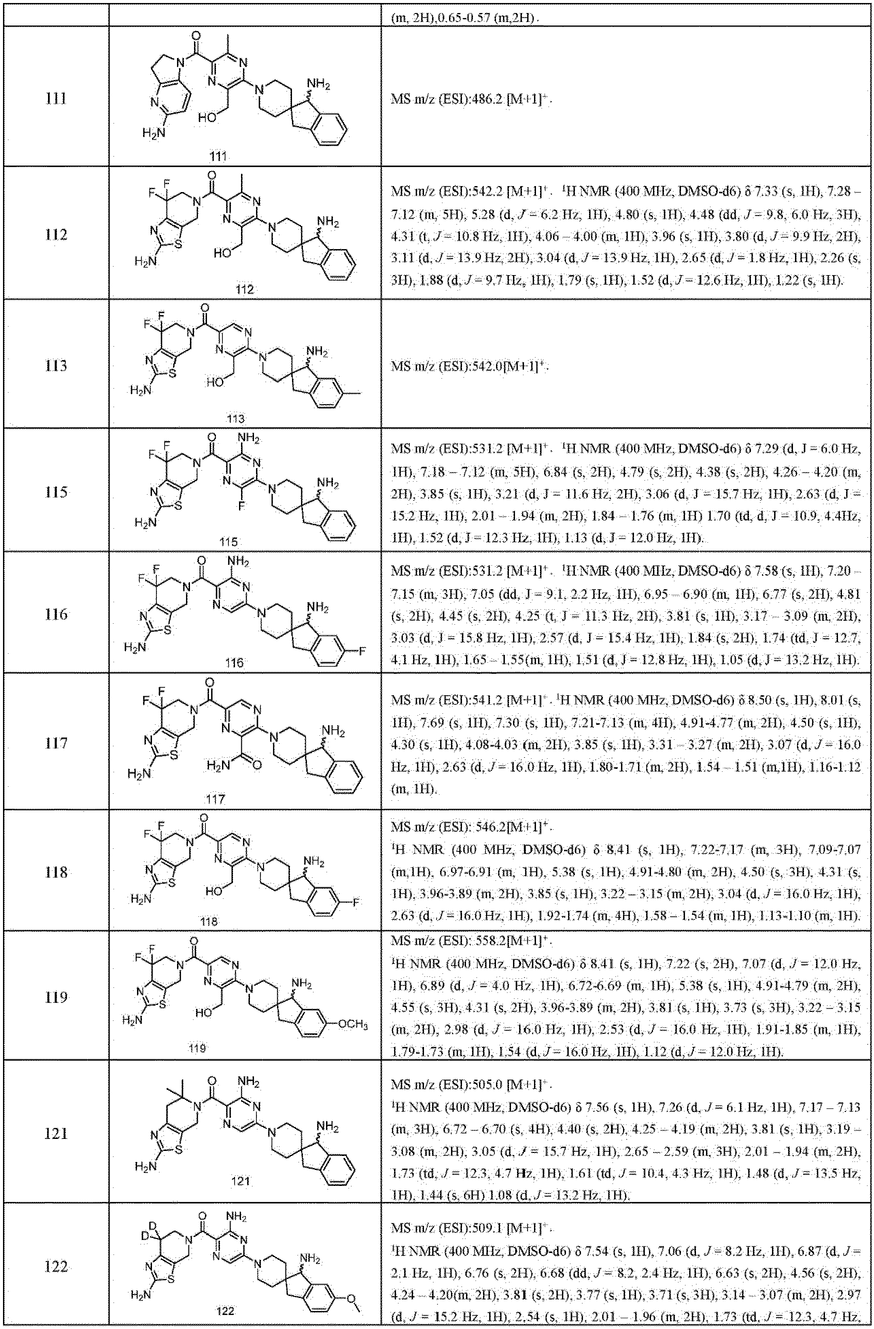

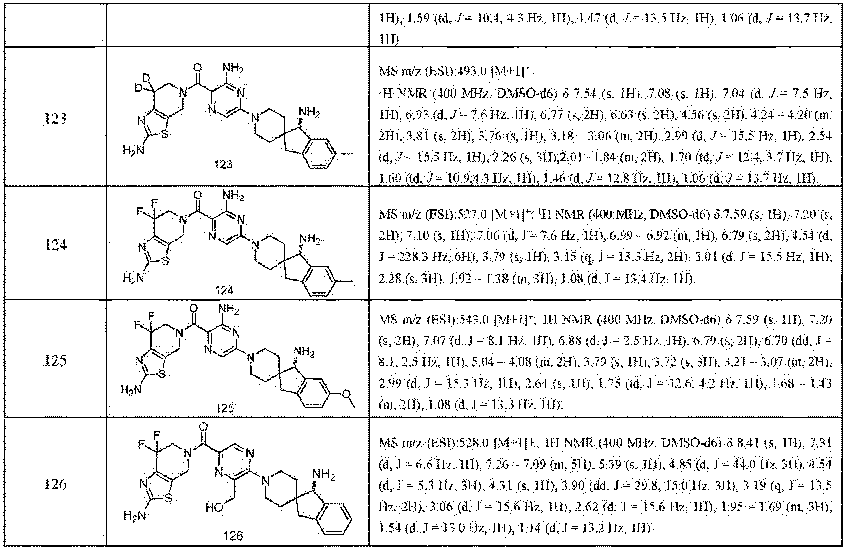

### Example 127 to Example 128: Preparation of Compound 127 and Compound 128

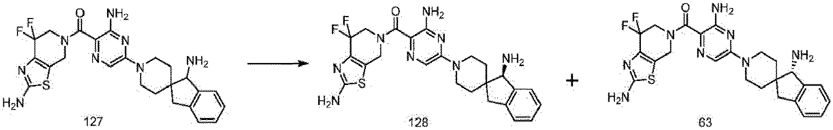

Compound 127 was prepared with reference to Example 63, with the difference that Compound 3e was replaced by Compound 3o. ¹H NMR (400 MHz, dmso) δ 7.58 (s, 1H), 7.29 (d, *J* = 5.7 Hz, 1H), 7.24 - 7.08 (m, 3H), 6.76 (s, 2H), 4.81 (s, 2H), 4.45 (s, 2H), 4.24 (s, 2H), 3.84 (s, 1H), 3.45 (s, 1H), 3.26 - 3.11 (m, 1H), 3.11 - 2.98 (m, 1H), 2.62 (d, *J =* 15.6 Hz, 1H), 1.80 - 1.67 (m, 1H), 1.68 - 1.54 (m, 1H), 1.49 (d, *J* = 12.9 Hz, 1H), 1.17 - 1.03 (m, 1H).

Compound 127 was separated with a chiral HPLC column (system: Waters SFC-200; column: AD-3 4.6*100mm 3 µm, solvent: MeOH [0.2% NH₃ (7M in MeOH)]) to obtain Enantiomer P1 Compound 63 (retention time: 3.672 min), and Enantiomer P2 Compound 128 (retention time: 5.235 min), respectively.

Compound 63 synthesized in Example 63 was compared with Enantiomer P1 and Enantiomer P2 in the above reaction through a chiral HPLC column (system: Waters SFC-200; column: AD-3 4.6*100mm 3µm, solvent: MeOH [0.2% NH₃ (7M in MeOH)]). The retention time of Compound 63 and Enantiomer P1 was the same. Therefore, it was presumed that the absolute configuration of Enantiomer P1 is (S). Therefore, the absolute configuration of Enantiomer P2 (Compound 128) is (R).

### Example 129 to Example 130: Preparation of Compound 129 and Compound 130

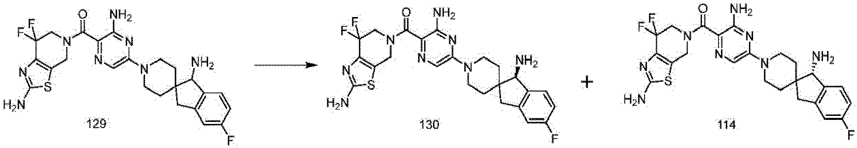

Compound 129 was prepared with reference to Example 63, with the difference that Compound 3e was replaced by Compound 3p. MS m/z (ESI):531.2 [M+1]⁺. ¹H NMR (400 MHz, DMSO-d6)δ 7.58 (s, 1H), 7.29 - 7.24(m, 1H), 7.18 (s, 2H), 7.00 -6.93 (m, 2H), 6.77 (s, 2H), 4.81 (s, 2H), 4.42 (s, 2H), 4.27 - 4.22 (m, 2H), 3.78 (s, 1H), 3.21 - 3.12 (m, 2H), 3.05 (d, J = 16.0 Hz, 1H), 2.62 (d, J = 15.9 Hz, 1H), 1.80 (s, 2H), 1.75 - 1.59 (m, 2H), 1.48 (d, J = 12.7 Hz, 1H), 1.09 (d, J = 12.7 Hz, 1H).

The obtained Compound 129 was separated with a chiral HPLC column (system: Waters SFC-200; column: AD-3 4.6 * 100mm 3µm solvent: MeOH [0.2% NH3 (7M in MeOH)]) to obtain Enantiomer P1 (retention time: 3.274min) and Enantiomer P2 (retention time: 4.272min), respectively.

The Compound 114 synthesized in Example 114 was compared with the Enantiomer P1 and Enantiomer P2 in the above reaction through a chiral HPLC column (system: Waters SFC-200; column: AD-3 4.6 * 100mm 3µm solvent: MeOH [0.2% NH3 (7M in MeOH)]). The retention time of Compound 114 and Enantiomer P1 was the same. Therefore, it was presumed that the absolute configuration of Enantiomer P1 is (S). Therefore, the absolute configuration of Enantiomer P2 (Compound 130) is (R).

### Test Example 1: Phosphatase activity assay

IC₅₀ values were assayed using Homogeneous Full Length SHP-2 Assay Kit (BPS Bioscience# 79330), which uses 6,8-difluoro-4-methylumbelliferone phosphate (DiFMUP) as the reaction substrate. SHP2 enzyme solution (diluted to 0.04 ng/µL with 1*buffer) was mixed with 0.5 µM SHP-2 activating Peptide in a 1*buffer solution containing 2.5 mM DTT to activate PTP. At the same time, DMSO (0.1% (V/V)) or the compounds (concentration: 0.5 nM to 1 µM) were added, mixed and co-incubated for 1 hour. DiFMUP (50 µM, total reaction system volume of 20 µL) was added, and the reaction was started. After 30 min of incubation, the fluorescence intensity of the reaction system (excitation wavelength: 350 nm, emission wavelength: 460 nm) was measured by Tecan Infinite M1000. The test results are shown in Table 1.

**Table 1: IC₅₀ values for inhibition of SHP-2 enzyme activity by the compounds**

| **Compound number** | SHP-2 IC₅₀ (nM) | **Compound number** | SHP-2 IC₅₀ (nM) |
|---|---|---|---|
| 2 | 4 | 71 | 5 |
| 3 | 13 | 72 | 9 |
| 4 | 1 | 73 | 10 |
| 5 | 1 | 74 | 6 |
| 6 | 83 | 75 | 5 |
| 7 | 6 | 76 | 10 |
| 8 | 5 | 77 | 11 |
| 9 | 59 | 78 | 10 |
| 10 | 4 | 79 | 14 |
| 11 | 24 | 80 | 4 |
| 12 | 49 | 81 | 4 |
| 13 | 11 | 82 | 4 |
| 14 | 22 | 83 | 4 |
| 15 | 13 | 85 | 4 |
| 16 | 5 | 86 | 8 |
| 17 | 4 | 87 | 9 |
| 19 | 5 | 88 | 5 |
| 20 | 17 | 89 | 4 |
| 21 | 3 | 90 | 24 |
| 22 | 6 | 91 | 3 |
| 23 | 22 | 92 | 4 |
| 25 | 15 | 93 | 8 |
| 27 | 5 | 94 | 7 |
| 28 | 4 | 95 | 3 |
| 29 | 43 | 96 | 4 |
| 31 | 47 | 97 | 10 |
| 32 | 28 | 98 | 26 |
| 33 | 29 | 99 | 10 |
| 34 | 6 | 100 | 7 |
| 35 | 5 | 101 | 18 |
| 36 | 18 | 102 | 11 |
| 37 | 5 | 103 | 13 |
| 39 | 36 | 104 | 10 |
| 40 | 59 | 105 | 13 |
| 43 | 60 | 106 | 15 |
| 44 | 25 | 107 | 9 |
| 45 | 7 | 108 | 3 |
| 46 | 29 | 109 | 3 |
| 48 | 4 | 110 | 9 |
| 49 | 8 | 111 | is |
| 50 | 14 | 112 | 14 |
| 51 | 35 | 113 | 6 |
| 52 | 4 | 114 | 7 |
| 53 | 4 | 115 | 4 |
| 54 | 8 | 116 | 5 |
| 55 | 50 | 117 | 11 |
| 56 | 21 | 118 | 6 |
| 57 | 6 | 119 | 7 |
| 59 | 6 | 121 | 9 |
| 60 | 4 | 122 | 8 |
| 61 | 7 | 123 | 5 |
| 62 | 5 | 124 | 9 |
| 63 | 3 | 125 | 10 |
| 64 | 5 | 126 | 6 |
| 65 | 4 | 127 | 7 |
| 66 | 9 | 129 | 5 |
| 67 | 7 | D1 | 37 |
| 68 | 7 | | |
| 69 | 9 | | |
| 70 | 8 | | |

The structure of Compound D1 is as follows, with CAS number of 2377352-41-3.

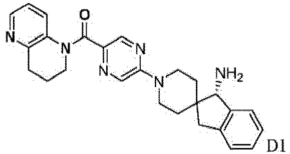

### Test Example 2: Cell proliferation assay

MV 411 cells (20000 cells/well) were inoculated and cultured in 96 (Corning#3916) well plates, and the medium was 80 µL/well (IMDM with 10% fetal bovine serum (FBS), Gibco#10099-141C). After 24 hours of incubation, 20 µL of the compounds of the present invention configured to different concentrations (DMSO final concentration of 0.1% (V/V)) were added. On day 4, 60 µL of Cell Titer-Glo (Promega # G7558) was added to each well and mixed at 80 rpm, 25°C, 15 min, and the fluorescence values were detected by Victor X5 (PE). The test results are shown in Table 2.

**Table 2: IC50 values for inhibitory activity on MV-4-11 cell proliferation by the compounds**

| **Compound number** | MV-4-11 IC₅₀ (nM) | **Compound number** | MV-4-11 IC₅₀ (nM) |
|---|---|---|---|
| 2 | 24 | 78 | 218 |
| 3 | 54 | 79 | 257 |
| 4 | 78 | 80 | 127 |
| 5 | 220 | 81 | 128 |
| 7 | 95 | 82 | 25 |
| 8 | 111 | 83 | 17 |
| 10 | 44 | 85 | 12 |
| 13 | 82 | 86 | 286 |
| 15 | 170 | 87 | 159 |
| 16 | 40 | 88 | 235 |
| 17 | 17 | 89 | 127 |
| 19 | 142 | 91 | 61 |
| 21 | 107 | 92 | 119 |
| 22 | 78 | 93 | 114 |
| 27 | 98 | 94 | 110 |
| 28 | 44 | 95 | 176 |
| 33 | 285 | 96 | 157 |
| 34 | 56 | 99 | 46 |
| 35 | 101 | 100 | 51 |
| 37 | 172 | 101 | 85 |
| 40 | 59 | 102 | 57 |
| 45 | 176 | 103 | 65 |
| 46 | 166 | 104 | 58 |
| 49 | 241 | 105 | 25 |
| 50 | 241 | 106 | 164 |
| 52 | 196 | 107 | 119 |
| 53 | 192 | 108 | 8 |
| 54 | 206 | 109 | 58 |
| 59 | 253 | 110 | 21 |
| 60 | 46 | 112 | 18 |
| 61 | 71 | 113 | <5 |
| 62 | 156 | 114 | 7 |
| 63 | <5 | 115 | 4 |
| 64 | 70 | 116 | 6 |
| 65 | 5 | 117 | 39 |
| 66 | 196 | 118 | 5 |
| 67 | 79 | 119 | 5 |
| 68 | 125 | 121 | 49 |
| 69 | 49 | 122 | 25 |
| 70 | 25 | 123 | 16 |
| 71 | 16 | 124 | 12 |
| 72 | 12 | 125 | 19 |
| 73 | 19 | 126 | 6 |
| 74 | 6 | 127 | 13 |
| 75 | 9 | 129 | 9 |
| 76 | 191 | D1 | 860 |
| 77 | 150 | | |

### Test Example 3: Inhibition effect on hERG potassium ion channel

The electrophysiological manual patch clamp method was used to test the effect of the compounds on the current of hERG potassium channel (human *Ether-a-go-go* Related Gene potassium channel).

### 1. Cell culture and treatment

CHO cells stably expressing hERG were cultured in a cell Petri dish having a diameter of 35 mm, placed in an incubator at 37°C and with 5% CO₂, and passaged every 48 hours at a ratio of 1:5. The culture medium formula was: 90% F12 (Invitrogen), 10% fetal bovine serum (Gibco), 100 µg/mL G418 (Invitrogen), and 100 µg/mL Hygromycin B (Invitrogen). On the day of the test, the cell culture medium was sucked away, and the cells were washed with extracellular fluid once. Then 0.25% Trypsin-EDTA (Invitrogen) solution was added, and the cells was digested at room temperature for 3-5 min. The digestive fluid was sucked away, and the cells were resuspended with extracellular fluid, and transfered to the experimental dish for electrophysiological recording.

### 2. Compound preparation

On the day of test, the compounds were prepared using DMSO into mother solution with concentration of 20 mM, and then serial dilution with DMSO was performed three times, i.e., 10 µL of the solution was added to 20 µL of DMSO, then 10 µL of the serial diluted solution of the compounds in DMSO was added to 4990 µL of extracellular fluid, and 500 times of dilution was performed to obtain the final concentration to be tested.

### 3. Electrophysiological recording process

The hERG potassium channel currents of CHO (Chinese hamster ovary) cells stably expressing hERG potassium channels were recorded using whole-cell patch clamp technique at room temperature. The glass microelectrodes were made from glass electrode blanks (BF150-86-10, Sutter) by pulling through a puller, and the tip resistance was about 2-5 MΩ after filling the electrode with the inner liquid. The glass microelectrodes could be connected to a patch clamp amplifier by inserting them into the amplifier probe. Clamp voltage and data recording were controlled and recorded by a computer using software pClamp 10 with a sampling frequency of 10 kHz and a filtering frequency of 2 kHz. After obtaining a whole-cell recording, the cell was clamped at -80 mV, and the step voltage inducing hERG potassium current (*I* hERG) was given a depolarizing voltage from -80 mV to +20 mV for 2 s, and repolarized to -50 mV for 1 s, then returning to -80 mV. This voltage stimulus was given every 10 s. After it is determined that the potassium hERG current was stable (for 1 min), the dosing process was initiated. The compounds were administered for at least 1 min at each tested concentration, and at least 2 cells (n≥2) were tested at each concentration.

### 4. Data processing

Data analysis and processing were conducted using softwares pClamp 10, GraphPad Prism 5, and Excel. The degree of inhibitory activity of different compound concentrations on hERG potassium current (peak value of hERG tail current induced at -50 mV) was calculated using the following formula: Inhibition%=[1- (*I*/*I*o)] × 100%. Among them, Inhibition% represents the percentage inhibition of the compound on hERG potassium current, and *I* and *I*o represent the amplitude of hERG potassium current after and before dosing, respectively.

IC50 of the compounds was calculated using software GraphPad Prism 5 by fitting the following equation: Y=Bottom+(Top-Bottom)/(1+10^(LogIC50-X)*HillSlope). Among them, X is the Log value of the detection concentration of test material, Y is the inhibition percentage at the corresponding concentration, and Bottom and Top are the minimum and maximum inhibition percentages, respectively.

**Table 3: IC50 values for inhibition of hERG potassium channel currents by certain compounds of the present invention**

| **Compound number** | hERG IC₅₀ (µM) |
|---|---|
| 59 | >30 |
| 63 | >30 |
| 70 | >30 |
| 82 | >30 |
| 108 | >30 |
| 114 | >30 |
| 125 | >30 |
| 126 | >30 |

As can be seen from the data in the above table, the compounds of the present invention have a weak current inhibitory effect on hERG and are therefore of good safety.

### Test Example 4: In vivo pharmacodynamic assay

### 1. KYSE-520 subcutaneous transplantation tumor model

The human esophageal squamous carcinoma cell line KYSE-520 was cultured in RPMI 1640 containing 10% fetal bovine serum placed in a 37°C, 5% CO₂ sterile incubator. The cells were passaged 2-3 times per week, digested with 0.25% trypsin-EDTA. After terminating the digestion with the above complete medium, the system was centrifugated at 1000 rpm for 5 min. The supernatant was discarded, and the cells were resuspended and passaged at a ratio of 1:3-1:5. Cells in logarithmic phase were collected, resuspended in serum-free RPMI 1640, counted and adjusted to the appropriate density.

KYSE-520 cells (3×10⁶ cells/mouse) were inoculated into the right ventral dorsum of approximately 6- to 8-week-old female Balb/C nude mice. Then the tumors grew to an average volume of about 200 mm³, the tumors were randomly grouped according to the tumor volume and body weight (n=8-10). Administration was started on the day of grouping and was performed once daily, defining the day of grouping as day 0. Tumor diameters were measured twice a week with vernier calipers, and tumor volume was calculated by the formula TV (mm³) = 0.5 × a × b², with a and b representing the long and short diameters (in mm) of the tumors, respectively. When the average tumor volume of the control group was about 800 mm³, the experiment was terminated. The tumor tissues were isolated and weighed to calculate the tumor weight proliferation rate with T/C_{weight}% = TW_{T}/TW_{C}%, and TW_{T} and TW_{C} represent the tumor weight of the administered group and the vehicle control group, respectively.

**Table 4: Tumor weight proliferation rate of certain compounds of the present invention**

| **Compound number** | **dose** | **T/C**_{weíght} (%) |
|---|---|---|
| 60 | 10 mg/kg, PO, QD | 44.62 |
| 63 | 10 mg/kg, PO, QD | 28.94 |
| 70 | 10 mg/kg, PO, QD | 35.04 |
| 114 | 10 mg/kg, PO, QD | 39.69 |

### 2. MC38 subcutaneous tumor model

The mouse colon cancer cell line MC38 was cultured in RPMI 1640 containing 10% fetal bovine serum placed in a 37°C, 5% CO₂ sterile incubator. The cells were passaged 2-3 times per week, digested with 0.25% trypsin-EDTA, and after terminating the digestion with the above complete medium, the system was centrifugated at 1000 rpm for 5 min. The supernatant was discarded, and the cells were resuspended and passaged at a ratio of 1:4-1:8. Cells in logarithmic phase were collected, resuspended in serum-free RPMI 1640, counted and adjusted to the appropriate density.

MC38 cells (5×10⁶ cells/mouse) were inoculated into the right ventral dorsum of approximately 6- to 8-week-old female C57BL/6 mice. When the tumors grew to an average volume of about 50-100 mm³, the tumors were randomly grouped according to the tumor volume and body weight (n=8-10). Oral administration was started on the day of grouping at 10 mg/kg and was performed once daily, defining the day of grouping as day 0. Tumor diameters were measured twice a week with vernier calipers, and tumor volume was calculated by the formula TV (mm³) = 0.5 × a × b², with a and b representing the long and short diameters (in mm) of the tumor, respectively. When the average tumor volume of the control group was about 1800 mm³, the experiment was terminated, and the tumor tissues were isolated and weighed to calculate the tumor weight proliferation rate with T/C_{weight}% = TW_{T}/TW_{C}%, wherein TW_{T} and TW_{C} represent the tumor weight of the administered group and the vehicle control group, respectively.

In the MC38 subcutaneous tumor model, the tumor weight proliferation rates T/C_{weight}% of Compound 60, Compound 63 and Compound 114 were less than 30%, which showed significant tumor growth inhibition and good antitumor efficacy.

All documents mentioned in the present invention are cited as references in the present application as if each document were cited individually as a reference. It is further to be understood that after reading the foregoing teachings of the present invention, those skilled in the art may make various alterations or modifications to the present invention, and these equivalent forms will likewise fall within the scope of the claims appended to this application.

## Claims

1. A compound represented by formula (I), or a pharmaceutically acceptable salt thereof, or a stereoisomer thereof: 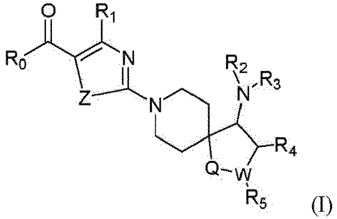 wherein in formula (I),
R₀ is of the structure represented by formula (a): 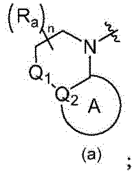
wherein Q₁ is a bond or is CR_{q1}R_{q2}, O or NR_{q3}; Q₂ represents a ring atom that is C or N; and when Q₁ contains N, Q₂ is not N;
R_{q1} and R_{q2} are each independently hydrogen, C₁₋₈ alkyl (preferably C₁₋₆ alkyl, more preferably C₁₋₃ alkyl), C₃₋₈ cycloalkyl (preferably C₃₋₆ cycloalkyl), C₁₋₈ alkoxy (preferably C₁₋₆ alkoxy, more preferably C₁₋₃ alkoxy), cyano, hydroxyl, carboxyl, halogen (preferably fluorine or chlorine), -C(O)NRₐ₀R_{b0}, -C(O)C₁₋₈ alkyl (preferably -C(O)C₁₋₆ alkyl, more preferably -C(O)C₁₋₃ alkyl) or -C(O)OC₁₋₈ alkyl (preferably -C(O)OC₁₋₆ alkyl, more preferably -C(O)OC₁₋₃ alkyl); or R_{q1}, R_{q2} and the linked carbon atom together form a 3- to 7-membered saturated or partially unsaturated heteromonocycle or a 3- to 7-membered saturated or partially unsaturated monocycle; wherein C₁₋₈ alkyl, C₁₋₈ alkoxy, C₃₋₈ cycloalkyl, 3- to 7-membered saturated or partially unsaturated heteromonocycle, and 3- to 7-membered saturated or partially unsaturated monocycle are unsubstituted or substituted by 1, 2, or 3 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from a substituent group S;
R_{q3} is hydrogen, C₁₋₈ alkyl (preferably C₁₋₆ alkyl, more preferably C₁₋₃ alkyl), C₃₋₈ cycloalkyl (preferably C₃₋₆ cycloalkyl), -C(O)NRₐ₀R_{b0}, -C(O)C₁₋₈ alkyl (preferably -C(O)C₁₋₆ alkyl, more preferably -C(O)C₁₋₃ alkyl) or -SO₂C₁₋₈ alkyl (preferably -So₂C₁₋₆ alkyl, more preferably -SO₂C₁₋₃ alkyl); wherein C₁₋₈ alkyl and C₃₋₈ cycloalkyl are unsubstituted or substituted by 1, 2 or 3 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl, and 5- to 6-membered heteroaryl; wherein 3- to 6-membered heterocycloalkyl, phenyl, and 5- to 6-membered heteroaryl among the substituents are each optionally substituted by 1, 2 or 3 substituents each independently selected from the substituent group S;
(Rₐ)ₙ represents n Rₐ that substitute hydrogens on ring atoms of nitrogen-containing 6-membered heterocycle, and n is 0, 1 or 2; each Rₐ is identical or different, and each Rₐ is independently deuterium, cyano, hydroxyl, carboxyl, halogen (preferably fluorine or chlorine), C₁₋₈ alkyl (preferably C₁₋₆ alkyl, more preferably C₁₋₃ alkyl), C₁₋₈ alkoxy (preferably C₁₋₆ alkoxy, more preferably C₁₋₃ alkoxy), -C(O)C₁₋₈ alkyl (preferably -C(O)C₁₋₆ alkyl, more preferably -C(O)C₁₋₃ alkyl), -C(O)OC₁₋₈ alkyl (preferably -C(O)OC₁₋₆ alkyl, more preferably -C(O)OC₁₋₃ alkyl), -OC(O)C₁₋₈ alkyl (preferably -OC(O)C₁₋₆ alkyl, more preferably -OC(O)C₁₋₃ alkyl) or -C(O)NRₐ₀R_{b0}; or any two Rₐ linked to the same ring atom or to adjacent ring atoms connect and form a 3- to 7-membered saturated or partially unsaturated heteromonocycle or a 3- to 7-membered saturated or partially unsaturated monocycle; wherein C₁₋₈ alkyl, C₁₋₈ alkoxy, 3- to 7-membered saturated or partially unsaturated heteromonocycle and 3- to 7-membered saturated or partially unsaturated monocycle are unsubstituted or substituted by 1, 2 or 3 substituents each independently selected from the group consisting of: deuterium, halogen, cyano, hydroxy, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl, and 5- to 6-membered heteroaryl among the substituents are each optionally substituted by 1, 2 or 3 substituents each independently selected from the substituent group S;
ring A is benzene ring or 5- to 6-membered heteroaryl ring; benzene ring and 5- to 6-membered heteroaryl ring are unsubstituted or substituted by 1, 2, 3 or 4 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)C₁₋₃ alkyl, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from the substituent group S;
or R₀ is of the structure represented by formula (b): 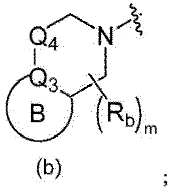
wherein Q₃ represents a ring atom that is C or N; Q₄ is a bond or is CR_{q4}R_{q5};
R_{q4} and R_{q5} are each independently hydrogen, deuterium, C₁₋₈ alkyl (preferably C₁₋₆ alkyl, more preferably C₁₋₃ alkyl), C₃₋₈ cycloalkyl (preferably C₃₋₆ cycloalkyl), C₁₋₈ alkoxy (preferably C₁₋₆ alkoxy, more preferably C₁₋₃ alkoxy), cyano, hydroxyl, carboxyl, halogen (preferably fluorine or chlorine), -C(O)NRₐ₀R_{b0}, -C(O)C₁₋₈ alkyl (preferably -C(O)C₁₋₆ alkyl, more preferably -C(O)C₁₋₃ alkyl) or -C(O)OC₁₋₈ alkyl (preferably -C(O)OC₁₋₆ alkyl, more preferably -C(O)OC₁₋₃ alkyl); or R_{q4}, R_{q5} and the linked carbon atom together form a 3- to 7-membered saturated or partially unsaturated heteromonocycle or a 3- to 7-membered saturated or partially unsaturated monocycle; wherein C₁₋₈ alkyl, C₁₋₈ alkoxy, C₃₋₈ cycloalkyl, 3- to 7-membered saturated or partially unsaturated heteromonocycle and 3- to 7-membered saturated or partially unsaturated monocycle are unsubstituted or substituted by 1, 2, or 3 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5-to 6-membered heteroaryl among the substituents are each optionally substituted by 1, 2 or 3 substituents each independently selected from the substituent group S;
(R_{b})ₘ represents m R_{b} that substitute hydrogens on ring atoms of nitrogen-containing heterocycle, and m is 0, 1 or 2; each R_{b} is identical or different, and each R_{b} is independently deuterium, cyano, hydroxyl, carboxyl, halogen (preferably fluorine or chlorine), C₁₋₈ alkyl (preferably C₁₋₆ alkyl, more preferably C₁₋₃ alkyl), C₁₋₈ alkoxy (preferably C₁₋₆ alkoxy, more preferably C₁₋₃ alkoxy ), -C(O)C₁₋₈ alkyl (preferably -C(O)C₁₋₆ alkyl, more preferably -C(O)C₁₋₃ alkyl), -C(O)OC₁₋₈ alkyl (preferably -C(O)OC₁₋₆ alkyl, more preferably -C(O)OC₁₋₃ alkyl), -OC(O)C₁₋₈ alkyl (preferably -OC(O)C₁₋₆ alkyl, more preferably -OC(O)C₁₋₃ alkyl) or -C(O)NRₐ₀R_{b0};
or any two R_{b} linked to the same ring atom or different ring atoms connect and form a 3- to 7-membered saturated or partially unsaturated heteromonocycle;
or one of R_{q4} and R_{q5} together with R_{b} on the adjacent carbon atom connect and form a 3- to 7-membered saturated or partially unsaturated heteromonocycle or a 3- to 7-membered saturated or partially unsaturated monocycle;
wherein C₁₋₈ alkyl, C₁₋₈ alkoxy, 3- to 7-membered saturated or partially unsaturated heteromonocycle and 3- to 7-membered saturated or partially unsaturated monocycle are unsubstituted or substituted by 1, 2, or 3 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5-to 6-membered heteroaryl among the substituents are each optionally substituted by 1, 2 or 3 substituents each independently selected from the substituent group S;
ring B is benzene ring or 5- to 6-membered heteroaryl ring; benzene ring and 5- to 6-membered heteroaryl ring are unsubstituted or substituted by 1, 2, 3 or 4 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)C₁₋₃ alkyl, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from the substituent group S;
R₁ is hydrogen, C₁₋₈ alkyl (preferably C₁₋₆ alkyl, more preferably C₁₋₃ alkyl), NRₐ₀R_{b0}, C₃₋₈ cycloalkyl (preferably C₃₋₆ cycloalkyl), C₁₋₈ alkoxy (preferably C₁₋₆ alkoxy, more preferably C₁₋₃ alkoxy), cyano, hydroxyl, carboxyl, halogen (preferably fluorine or chlorine), -C(O)NRₐ₀R_{b0}, -C(O)C₁₋₈ alkyl (preferably -C(O)C₁₋₆ alkyl, more preferably -C(O)C₁₋₃ alkyl) or -C(O)OC₁₋₈ alkyl (preferably -C(O)OC₁₋₆ alkyl, more preferably -C(O)OC₁₋₃ alkyl); wherein C₁₋₈ alkyl, C₁₋₈ alkoxy and C₃₋₈ cycloalkyl are unsubstituted or substituted by 1, 2 or 3 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from the substituent group S;
R₂ and R₃ are each independently hydrogen, C₁₋₈ alkyl (preferably C₁₋₆ alkyl, more preferably C₁₋₃ alkyl), C₃₋₈ cycloalkyl (preferably C₃₋₆ cycloalkyl), -C(O)NRₐ₀R_{b0} or -C(O)C₁₋₈ alkyl (preferably -C(O)C₁₋₆ alkyl, more preferably -C(O)C₁₋₃ alkyl); wherein C₁₋₈ alkyl and C₃₋₈ cycloalkyl are unsubstituted or substituted by 1, 2 or 3 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from the substituent group S;
Z is -N=CR_{Z1}-, -N=N-, -C(O)-NR_{Z2}- or -NR_{Z3}-CHR_{Z4}-;
or Z is of the structure represented by formula (c): 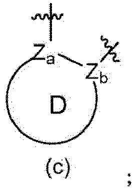
wherein Zₐ and Z_{b} represent ring atoms, and are each independently C or N;
ring D is 5- to 6-membered heteroaryl ring; 5- to 6-membered heteroaryl ring is unsubstituted or substituted by 1, 2, or 3 substituents each independently selected from the group consisting of: deuterium, halogen, cyano, hydroxyl, carboxyl, C₁₋₃ alkyl, hydroxyl-substituted C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)C₁₋₃ alkyl, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from the substituent group S;
R_{Z1} is hydrogen, C₁₋₈ alkyl (preferably C₁₋₆ alkyl, more preferably C₁₋₃ alkyl), C₃₋₈ cycloalkyl (preferably C₃₋₆ cycloalkyl), C₁₋₈ alkoxy (preferably C₁₋₆ alkoxy, more preferably C₁₋₃ alkoxy), cyano, hydroxyl, carboxyl, halogen (preferably fluorine or chlorine), -C(O)NRₐ₀R_{b0}, -C(O)C₁₋₈ alkyl (preferably -C(O)C₁₋₆ alkyl, more preferably -C(O)C₁₋₃ alkyl) or -C(O)OC₁₋₈ alkyl (preferably -C(O)OC₁₋₆ alkyl, more preferably -C(O)OC₁₋₃ alkyl); wherein C₁₋₈ alkyl, C₁₋₈ alkoxy and C₃₋₈ cycloalkyl are unsubstituted or substituted by 1, 2 or 3 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from the substituent group S;
R_{Z2} is hydrogen, C₁₋₈ alkyl (preferably C₁₋₆ alkyl, more preferably C₁₋₃ alkyl), C₃₋₈ cycloalkyl (preferably C₃₋₆ cycloalkyl), -C(O)NRₐ₀R_{b0}, -C(O)C₁₋₈ alkyl (preferably -C(O)C₁₋₆ alkyl, more preferably -C(O)C₁₋₃ alkyl) or -SO₂C₁₋₈ alkyl (preferably -SO₂C₁₋₆ alkyl, more preferably -SO₂C₁₋₃ alkyl); wherein C₁₋₈ alkyl and C₃₋₈ cycloalkyl are unsubstituted or substituted by 1, 2 or 3 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from the substituent group S;
R_{Z3} and R_{Z4} connect and form a 3- to 7-membered saturated or partially unsaturated heteromonocycle; 3- to 7-membered saturated or partially unsaturated heteromonocycle is unsubstituted or substituted by 1, 2, or 3 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from the substituent group S;
Q, W, R₄ and R₅ are selected from the group consisting of:
(i) Q is CR_{q6}R_{q7}, O or NR_{q8}; W is C or N; and Q and W are not heteroatoms at the same time; R₄, R₅ and the linked ring atoms together form benzene ring or 5- to 6-membered heteroaryl ring, and benzene ring or 5- to 6-membered heteroaryl ring is fused to a 5-membered ring; benzene ring and 5-to 6-membered heteroaryl ring are unsubstituted or substituted by 1, 2, 3 or 4 substituents each independently selected from the group consisting of: deuterium, halogen, cyano, hydroxyl, carboxyl, C₁₋₃ alkyl, hydroxyl-substituted C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)C₁₋₃ alkyl, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5-to 6-membered heteroaryl among the substituents are each optionally substituted by 1, 2 or 3 substituents each independently selected from the substituent group S;
and (ii) Q is CR_{q6}R_{q7}; W is O; R₅ is absent; R₄ is hydrogen, C₁₋₈ alkyl (preferably C₁₋₆ alkyl, more preferably C₁₋₃ alkyl), C₃₋₈ cycloalkyl (preferably C₃₋₆ cycloalkyl), C₁₋₈ alkoxy (preferably C₁₋₆ alkoxy, more preferably C₁₋₃ alkoxy), cyano, hydroxy, carboxyl or halogen (preferably fluorine or chlorine);
wherein R_{q6} and R_{q7} are each independently hydrogen, C₁₋₈ alkyl (preferably C₁₋₆ alkyl, more preferably C₁₋₃ alkyl), C₃₋₈ cycloalkyl (preferably C₃₋₆ cycloalkyl), C₁₋₈ alkoxy (preferably C₁₋₆ alkoxy, more preferably C₁₋₃ alkoxy), cyano, hydroxyl, carboxyl, halogen (preferably fluorine or chlorine), -C(O)NRₐ₀R_{b0}, -C(O)C₁₋₈ alkyl (preferably -C(O)C₁₋₆ alkyl, more preferably -C(O)C₁₋₃ alkyl) or -C(O)OC₁₋₈ alkyl (preferably -C(O)OC₁₋₆ alkyl, more preferably -C(O)OC₁₋₃ alkyl); or R_{q6}, R_{q7} and the linked carbon atom together form a 3- to 7-membered saturated or partially unsaturated heteromonocycle or a 3- to 7-membered saturated or partially unsaturated monocycle; wherein C₁₋₈ alkyl, C₁₋₈ alkoxy, C₃₋₈ cycloalkyl, 3- to 7-membered saturated or partially unsaturated heteromonocycle and 3- to 7-membered saturated or partially unsaturated monocycle are unsubstituted or substituted by 1, 2 or 3 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5-to 6-membered heteroaryl among the substituents are each optionally substituted by 1, 2 or 3 substituents each independently selected from the substituent group S;
R_{q8} is hydrogen, C₁₋₈ alkyl (preferably C₁₋₆ alkyl, more preferably C₁₋₃ alkyl), C₃₋₈ cycloalkyl (preferably C₃₋₆ cycloalkyl), -C(O)NRₐ₀R_{b0}, -C(O)C₁₋₈ alkyl (preferably -C(O)C₁₋₆ alkyl, more preferably -C(O)C₁₋₃ alkyl) or -SO₂C₁₋₈ alkyl (preferably -SO₂C₁₋₆ alkyl, more preferably -SO₂C₁₋₃ alkyl); wherein C₁₋₈ alkyl and C₃₋₈ cycloalkyl are unsubstituted or substituted by 1, 2 or 3 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from the substituent group S;
the substituent group S is selected from the group consisting of: halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl;
Rₐ₀ and R_{b0} are each independently hydrogen, C₁₋₃ alkyl or acetyl; or Rₐ₀, R_{b0} and the linked nitrogen atom together form a 4- to 6-membered saturated heteromonocycle; 4- to 6-membered saturated heteromonocycle is optionally substituted by 1, 2 or 3 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, -SO₂C₁₋₈ alkyl, -S(O)C₁₋₃ alkyl, -C(O)NH₂, -C(O)NH(C₁₋₃ alkyl), -C(O)N(C₁₋₃ alkyl)₂, -C(O)OC₁₋₃ alkyl, -OC(O)C₁₋₃ alkyl, C₃₋₆ cycloalkyl, C₃₋₆ cycloalkoxy and 3- to 6-membered heterocycloalkyl; and
Rₐ₁ and R_{b1} are each independently hydrogen, C₁₋₃ alkyl or acetyl; or Rₐ₁, R_{b1} and the linked nitrogen atom together form a 4- to 6-membered saturated heteromonocycle; 4- to 6-membered saturated heteromonocycle is optionally substituted by 1, 2 or 3 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, -SO₂C₁₋₈ alkyl, -S(O)C₁₋₃ alkyl, -C(O)NH₂, -C(O)NH(C₁₋₃ alkyl), -C(O)N(C₁₋₃ alkyl)₂, -C(O)OC₁₋₃ alkyl, -OC(O)C₁₋₃ alkyl, C₃₋₆ cycloalkyl, C₃₋₆ cycloalkoxy and 3- to 6-membered heterocycloalkyl.

2. The compound according to claim 1, or the pharmaceutically acceptable salt thereof, or the stereoisomer thereof, wherein the compound represented by formula (I) is of the structure represented by formula (IA): 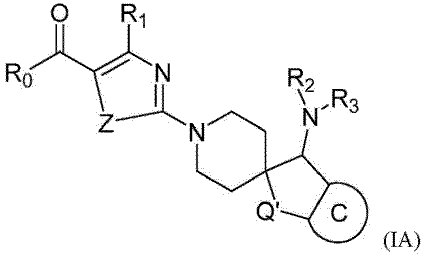
wherein, Q' is CR_{q6}R_{q7}, O or NR_{q8};
ring C is benzene ring or 5- to 6-membered heteroaryl ring; benzene ring and 5- to 6-membered heteroaryl ring are unsubstituted or substituted by 1, 2, 3, or 4 substituents each independently selected from the group consisting of: deuterium, halogen, cyano, hydroxyl, carboxyl, C₁₋₃ alkyl, hydroxyl-substituted C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)C₁₋₃ alkyl, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from the substituent group S; and wherein R₀, R₁, R₂, R₃, Z, R_{q6}, R_{q7}, R_{q8}, Rₐ₁ and R_{b1} are as defined in claim 1.

3. The compound according to claim 2, or the pharmaceutically acceptable salt thereof, or the stereoisomer thereof, wherein the compound represented by formula (IA) is of the structure represented by formula (IA-a) or formula (IA-b):

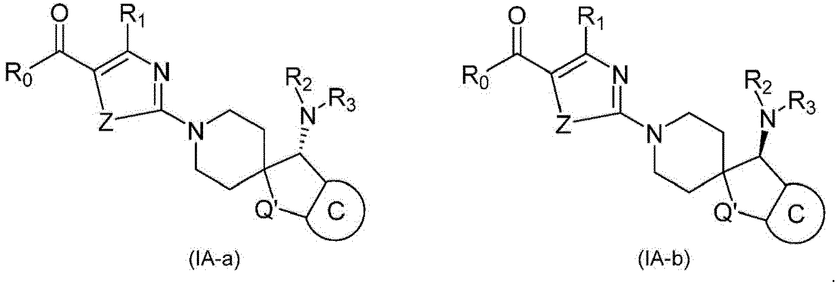

4. The compound according to claim 2, or the pharmaceutically acceptable salt thereof, or the stereoisomer thereof, wherein the compound represented by formula (IA) is of the structure represented by formula (IA-a):

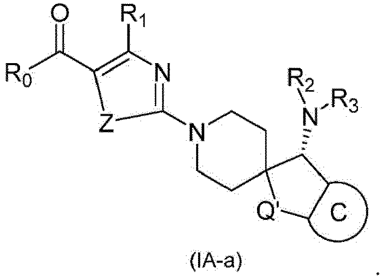

5. The compound according to claim 1, or the pharmaceutically acceptable salt thereof, or the stereoisomer thereof, wherein R₁ is hydrogen or NH₂.

6. The compound according to claim 1, or the pharmaceutically acceptable salt thereof, or the stereoisomer thereof, wherein Z is -N=CR_{Z1}-; R_{Z1} is hydrogen, C₁₋₃ alkyl (preferably methyl), C₃₋₆ cycloalkyl (preferably cyclopropyl), C₁₋₃ alkoxy (preferably methoxy), cyano, hydroxyl, carboxyl, halogen (preferably fluorine or chlorine), -C(O)NH₂ or -C(O)C₁₋₃ alkyl (preferably -C(O)CH₃) or -C(O)OC₁₋₃ alkyl (preferably -C(O)OCH₃); wherein C₁₋₃ alkyl, C₁₋₃ alkoxy and C₃₋₆ cycloalkyl are unsubstituted or substituted with 1, 2 or 3 substituents each independently selected from the group consisting of: deuterium, halogen, cyano, hydroxyl, carboxyl, C₁₋₃ alkyl, C₁₋₃ alkoxy, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)OC₁₋₃ alkyl, -OC(O)C₁₋₃ alkyl, C₃₋₆ cycloalkyl, C₃₋₆ cycloalkyloxy and 3- to 6-membered heterocycloalkyl.

7. The compound according to claim 1, or the pharmaceutically acceptable salt thereof, or the stereoisomer thereof, wherein Z is -N=CH-.

8. The compound according to claim 1, or the pharmaceutically acceptable salt thereof, or the stereoisomer thereof, wherein when ring B is 5- to 6-membered heteroaryl ring, ring B is selected from the group consisting of imidazole ring, pyrazole ring, 1,2,4-triazole ring, thiazole ring, oxazole ring and pyridine ring.

9. The compound according to claim 1, or the pharmaceutically acceptable salt thereof, or the stereoisomer thereof, wherein when ring B is 5- to 6-membered heteroaryl ring, ring B is a ring selected from the following rings: 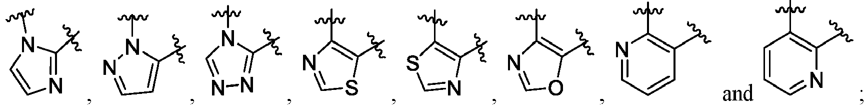 wherein " - " represents that the two linked ring atoms share a pair of adjacent atoms with the other ring to which they are fused; the above rings are each optionally substituted by 1, 2, 3 or 4 substituents each independently selected from the group consisting of: deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)C₁₋₃ alkyl, -C(O)OC₁₋₃ alkyl, - OC(O)C₁₋₃ alkyl, C₃₋₆ cycloalkyl, C₃₋₆ cycloalkyloxy, 3- to 6-membered heterocycloalkyl, phenyl and 5- to 6-membered heteroaryl; wherein 3- to 6-membered heterocycloalkyl, phenyl and 5- to 6-membered heteroaryl among the substituents are each optionally substituted by 1, 2 or 3 substituents each independently selected from the substituent group S; wherein Rₐ₁, R_{b1} and the substituent group S are as defined in claim 1.

10. The compound according to claim 1, or the pharmaceutically acceptable salt thereof, or the stereoisomer thereof, wherein the structure represented by formula (b) is selected from the group consisting of: 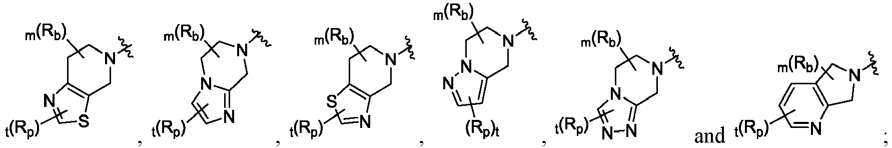 wherein (R_{b})ₘ is as defined in claim 1; (Rₚ)ₜ represents t Rₚ that substitute hydrogens on heteroaryl ring, and t is 0, 1, 2, 3 or 4; each Rₚ is identical or different, and each Rₚ is independently deuterium, halogen, cyano, hydroxyl, carboxyl, C₁₋₃ alkyl, C₁₋₃ alkoxy, C₂₋₄ alkenyl, C₂₋₄ alkynyl, halo-C₁₋₃ alkyl, halo-C₁₋₃ alkoxy, NRₐ₁R_{b1}, -SO₂C₁₋₃ alkyl, -S(O)C₁₋₃ alkyl, -C(O)NRₐ₁R_{b1}, -C(O)C₁₋₃ alkyl, -C(O)OC₁₋₃ alkyl, -OC(O)C₁₋₃ alkyl, C₃₋₆ cycloalkyl, C₃₋₆ cycloalkyloxy, 3- to 6-membered heterocycloalkyl, phenyl or 5- to 6-membered heteroaryl; wherein 3- to 6-membered heterocycloalkyl, phenyl and 5- to 6-membered heteroaryl are each optionally substituted by 1, 2 or 3 substituents each independently selected from the substituent group S; wherein Rₐ₁, R_{b1}, and the substituent group S are as defined in claim 1.

11. The compound according to claim 2, or the pharmaceutically acceptable salt thereof, or the stereoisomer thereof, wherein R₂ and R₃ are each independently hydrogen.

12. The compound according to claim 2, or the pharmaceutically acceptable salt thereof, or the stereoisomer thereof, wherein ring C is benzene ring; benzene ring is unsubstituted or substituted by 1, 2, or 3 substituents each independently selected from the group consisting of: deuterium, halogen (preferably fluorine or chlorine), cyano, hydroxyl, carboxyl, amino, C₁₋₃ alkyl (preferably methyl), hydroxy-substituted C₁₋₃ alkyl (preferably hydroxymethyl), C₁₋₃ alkoxy (preferably methoxy), halo-C₁₋₃ alkyl (preferably trifluoromethyl), halo-C₁₋₃ alkoxy, NH(C₁₋₃ alkyl), N(C₁₋₃ alkyl)₂, -SO₂C₁₋₈ alkyl, -C(O)NH₂, -C(O)NH(C₁₋₃ alkyl), -C(O)N(C₁₋₃ alkyl)₂, -C(O)C₁₋₃ alkyl, -C(O)OC₁₋₃ alkyl, -OC(O)C₁₋₃ alkyl, C₃₋₆ cycloalkyl (preferably cyclopropyl), C₃₋₆ cycloalkyloxy and 3- to 6-membered heterocycloalkyl.

13. The compound according to claim 2, or the pharmaceutically acceptable salt thereof, or the stereoisomer thereof, wherein Q' is CH₂.

14. The compound according to claim 1, or the pharmaceutically acceptable salt thereof, or the stereoisomer thereof, wherein the compound of formula (I) is at least one compound selected from Table A and/or Table D or a stereoisomer thereof.

15. The compound according to claim 1, or the pharmaceutically acceptable salt thereof, or the stereoisomer thereof, wherein the compound of formula (I) is at least one compound selected from Table B and/or Table E.

16. The compound according to claim 1, or the pharmaceutically acceptable salt thereof, or the stereoisomer thereof, wherein the compound of formula (I) is at least one compound selected from Table C and/or Table F.

17. A pharmaceutical composition comprising the compound according to any one of claims 1-16, or the pharmaceutically acceptable salt thereof, or the stereoisomer thereof; and a pharmaceutically acceptable carrier.

18. Use of the compound according to any one of claims 1-16, or the pharmaceutically acceptable salt thereof, or the stereoisomer thereof, or the pharmaceutical composition according to claim 17, in the preparation of a medicament for treatment and/or prevention of a disease or condition mediated by SHP2 or associated with aberrant SHP2 activity.

19. The use according to claim 18, wherein the disease or condition associated with aberrant SHP2 activity is selected from the group consisting of: solid tumor and hematologic tumor.

20. The use according to claim 18, wherein the SHP2-mediated disease or condition is a cancer selected from the group consisting of: juvenile myelomonocytic leukemia, acute myeloid leukemia, B-cell acute lymphoblastic leukemia, neuroblastoma, esophageal cancer, breast cancer, lung cancer, colon cancer, gastric cancer, and head and neck cancer.
